# EUROPEAN PATENT APPLICATION

(11) **EP 4 620 950 A1**
(43) Date of publication of application: **24.09.2025**
(21) Application number: 23892042.5
(22) Date of filing: 16.11.2023
(51) Int. Cl.: C07D 211/56, A61K 31/445, A61P 35/00, A61P 35/02, C07D 211/60, C07D 211/96, C07D 401/12

(54) **PIPERIDINE DERIVATIVE, SALT OF SAME, AND PHARMACEUTICAL COMPOSITION CONTAINING SAME AS ACTIVE INGREDIENT**

(30) Priority: 17.11.2022 KR 20220154909; 17.11.2022 KR 20220154910; 17.11.2022 KR 20220154911; 17.11.2022 KR 20220154912; 17.11.2022 KR 20220154913
(71) Applicant: Pharmcadd Co., Ltd., Busan 48548 (KR); Korea Research Institute of Chemical Technology, Daejeon 34114 (KR)
(72) Inventor: WU, Sangwook, Busan 48594 (KR); JEONG, Kwan Young, Daejeon 34114 (KR); LEE, Kyu Myung, Daejeon 34114 (KR); SHIN, Eun Gyeong, Daejeon 34114 (KR); LEE, Sang Keun, Daejeon 34114 (KR)
(74) Representative: Vossius & Partner Patentanwälte Rechtsanwälte mbB
(86) International application number: PCT/KR2023/018471
(87) International publication number: WO 2024/106979

(57) **Abstract**

The present invention relates to a piperidine derivative compound and a use thereof. The piperidine derivative compound of the present invention exhibits excellent inhibitory activity against FLT3, and thus can be usefully employed in the treatment or prevention of FLT3-related diseases.

## Description

### [Technical Field]

The present invention relates to a piperidine derivative compound and medicinal uses thereof. Specifically, the present invention relates to a piperidine derivative compound having FLT3 inhibitory activity.

### [Background Art]

Fms-like tyrosine kinase-3 (FLT3) is a type of tyrosine kinase receptor that is mainly expressed in hematopoietic stem cells and is involved in the normal development and survival of hematopoietic stem cells and progenitor cells. However, overexpression and mutations in the gene of FLT3 are reported to be the most common factors affecting the development of acute myeloid leukemia (AML) by disrupting the intracellular signaling network, which contributes to poor prognosis and drug resistance. Mutant FLT3 may be classified based on the location and type of mutation, with internal tandem duplications (ITD) and point mutation in tyrosine kinase domain (TKD) being representative examples. FLT3 mutations are common enough to be identified in approximately one-third of newly diagnosed adult patients. Among the mutations, FLT3-ITD mutations are observed in

approximately 20-30% of AML patients and are associated with a poor prognosis resulting in a high relapse rate and low survival rate. Another mutant, FLT3-TKD, is found in 5% of AML patients, but its prognosis remains unknown.

Although a variety of FLT3 inhibitors are available to treat AML, the expression of mutant FLT3 in addition to wild-type FLT3 appears to reduce the effectiveness of conventional drugs (Oncogene, 2010, 29(37), 5120-5134). In addition, there is a problem of frequent relapse with conventional standard chemotherapy for acute myeloid leukemia (AML) (J. Natl. Cancer Inst. 2014, 106(2), djt440).

Therefore, there is an urgent need for the development of effective new drugs that target wild-type FLT3 and mutant FLT3 in patients who fail to respond to treatment or experience relapse after treatment.

### [Disclosure]

### [Technical Problem]

An object of the present invention is to provide a piperidine derivative having a novel structure, a stereoisomer thereof, or a pharmaceutically acceptable salt thereof.

Another object of the present invention is to provide a method for preparing the piperidine derivative compound.

Still another object of the present invention is to provide a pharmaceutical use of the piperidine derivative

compound, and specifically, to a pharmaceutical composition for the treatment or prevention of FLT3-related diseases comprising the piperidine derivative compound as an active ingredient, use of the compound for the treatment or prevention of FLT3-related diseases, or a method for treating or preventing FLT3-related diseases comprising administering the compound.

### [Technical Solution]

In order to achieve the above object, an embodiment provides a piperidine derivative compound having a specific structure that inhibits FLT3 activity.

### Piperidine derivative compound

The present invention provides a compound represented by the following Chemical Formula 1, a stereoisomer thereof, or a pharmaceutically acceptable salt thereof: 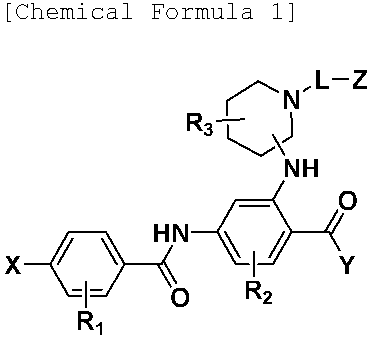 in Chemical Formula 1 above,
X is -C₁₋₆ alkyl or -C₁₋₆ haloalkyl;
Y is -C₁₋₆ alkyl, -OH, -O-C₁₋₆ alkyl, -NH₂, -NH-C₁₋₆ alkyl, or -N(C₁₋₆ alkyl) (C₁₋₆ alkyl);
L is -C(=O)-, -C(=O)NH-, -S(=O)₂-, or null;
Z is -H, -C₁₋₁₀ alkyl, -OH, -O-C₁₋₆ alkyl, -NH₂, -NH-C₁₋₆ alkyl, -N(C₁₋₆ alkyl) (C₁₋₆ alkyl), -(CH₂)n-cycloalkyl, - (CH₂)n-heterocycloalkyl, -(CH₂)n-aryl, or -(CH₂)n-heteroaryl, wherein at least one H of the - (CH₂)n-cycloalkyl, - (CH₂)n-heterocycloalkyl, -(CH₂)n-aryl, or -(CH₂)n-heteroaryl ring may be substituted with -C₁₋₆ alkyl, -C₁₋₆ aminoalkyl, -C₁₋₆ hydroxyalkyl, -C₁₋₆ haloalkyl, -CN, -C(=O)-C₁₋₆ alkyl, -NH₂, - NH-C₁₋₆ alkyl, -N(C₁₋₆ alkyl) (C₁₋₆ alkyl), -NO₂, -OH, -O-C₁₋₆ alkyl, -halo, aryl, or heteroaryl;
n is 0, 1, 2, 3, or 4; and
R₁ to R₃ are each independently -H, -C₁₋₆ alkyl, -C₁₋₆ aminoalkyl, -C₁₋₆ hydroxyalkyl, -C₁₋₆ haloalkyl, -CN, -NH₂, - NH-C₁₋₆ alkyl, -N(C₁₋₆ alkyl)(C₁₋₆ alkyl), -NO₂, -OH, -O-C₁₋₆ alkyl, or -halo.

According to an embodiment of the present invention, the compound represented by Chemical Formula 1 above, a stereoisomer thereof, or a pharmaceutically acceptable salt thereof may be in the following ranges:
X is -C₁₋₆ alkyl;
Y is -OH, -O-C₁₋₆ alkyl, -NH₂, -NH-C₁₋₆ alkyl, or -N(C₁₋₆ alkyl) (C₁₋₆ alkyl);
L is -C(=O)-, -C(=O)NH-, -S(=O)₂-, or null;
Z is -H, -C₁₋₁₀ alkyl, -NH₂, -NH-C₁₋₆ alkyl, -N(C₁₋₆ alkyl) (C₁₋₆ alkyl), -(CH₂)n-cycloalkyl, -(CH₂)n-aryl, or - (CH₂)n-heteroaryl, wherein at least one H of the -(CH₂)n-cycloalkyl, -(CH₂)n-aryl, or -(CH₂)n-heteroaryl ring may be substituted with -C₁₋₆ alkyl, -C₁₋₆ haloalkyl, -C(=O)-C₁₋₆ alkyl, -NO₂, -O-C₁₋₆ alkyl, -halo, or aryl;
n is 0, 1, or 2; and
R₁ to R₃ are each independently -H.

Further, the present invention provides a compound represented by the following Chemical Formula 2, a stereoisomer thereof, or a pharmaceutically acceptable salt thereof: 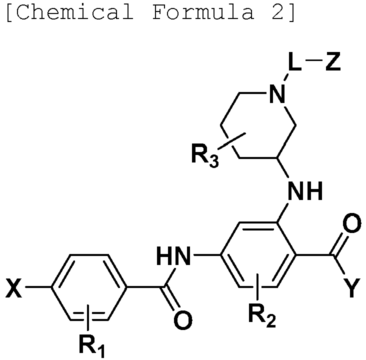 in Chemical Formula 2 above,
X is -C₁₋₆ alkyl or -C₁₋₆ haloalkyl;
Y is -C₁₋₆ alkyl, -OH, -O-C₁₋₆ alkyl, -NH₂, -NH-C₁₋₆ alkyl, or -N(C₁₋₆ alkyl) (C₁₋₆ alkyl);
L is -C(=O)-, -C(=O)NH-, -S(=O)₂-, or null;
Z is -H, -C₁₋₁₀ alkyl, -OH, -O-C₁₋₆ alkyl, -NH₂, -NH-C₁₋₆ alkyl, -N(C₁₋₆ alkyl) (C₁₋₆ alkyl), -(CH₂)n-cycloalkyl, - (CH₂)n-heterocycloalkyl, -(CH₂)n-aryl, or -(CH₂)n-heteroaryl, wherein at least one H of the -(CH₂)n-cycloalkyl, -(CH₂)n-heterocycloalkyl, -(CH₂)n-aryl, or -(CH₂)n-heteroaryl ring may be substituted with -C₁₋₆ alkyl, -C₁₋₆ aminoalkyl, -C₁₋₆ hydroxyalkyl, -C₁₋₆ haloalkyl, -CN, -C(=O)-C₁₋₆ alkyl, -NH₂, - NH-C₁₋₆ alkyl, -N(C₁₋₆ alkyl) (C₁₋₆ alkyl), -NO₂, -OH, -O-C₁₋₆ alkyl, -halo, aryl, or heteroaryl;
n is 0, 1, 2, 3, or 4; and
R₁ to R₃ are each independently -H, -C₁₋₆ alkyl, -C₁₋₆ aminoalkyl, -C₁₋₆ hydroxyalkyl, -C₁₋₆ haloalkyl, -CN, -NH₂, - NH-C₁₋₆ alkyl, -N(C₁₋₆ alkyl) (C₁₋₆ alkyl), -NO₂, -OH, -O-C₁₋₆ alkyl, or -halo.

Further, the present invention provides a compound represented by the following Chemical Formula 3, a stereoisomer thereof, or a pharmaceutically acceptable salt thereof: 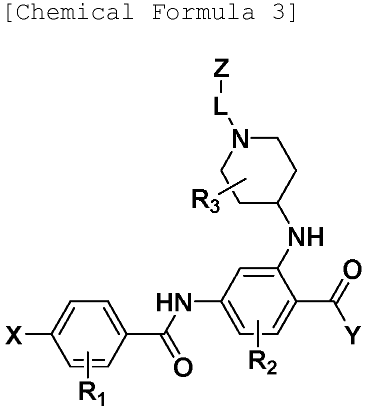 in Chemical Formula 3 above,
X is -C₁₋₆ alkyl or -C₁₋₆ haloalkyl;
Y is -C₁₋₆ alkyl, -OH, -O-C₁₋₆ alkyl, -NH₂, -NH-C₁₋₆ alkyl, or -N(C₁₋₆ alkyl) (C₁₋₆ alkyl);
L is -C(=O)-, -C(=O)NH-, -S(=O)₂-, or null;
Z is -H, -C₁₋₁₀ alkyl, -OH, -O-C₁₋₆ alkyl, -NH₂, -NH-C₁₋₆ alkyl, -N(C₁₋₆ alkyl) (C₁₋₆ alkyl), -(CH₂)n-cycloalkyl, - (CH₂)n-heterocycloalkyl, -(CH₂)n-aryl, or -(CH₂)n-heteroaryl, wherein at least one H of the -(CH₂)n-cycloalkyl, -(CH₂)n-heterocycloalkyl, -(CH₂)n-aryl, or -(CH₂)n-heteroaryl ring may be substituted with -C₁₋₆ alkyl, -C₁₋₆ aminoalkyl, -C₁₋₆ hydroxyalkyl, -C₁₋₆ haloalkyl, -CN, -C(=O)-C₁₋₆ alkyl, -NH₂, - NH-C₁₋₆ alkyl, -N(C₁₋₆ alkyl) (C₁₋₆ alkyl), -NO₂, -OH, -O-C₁₋₆ alkyl, -halo, aryl, or heteroaryl;
n is 0, 1, 2, 3, or 4; and
R₁ to R₃ are each independently -H, -C₁₋₆ alkyl, -C₁₋₆ aminoalkyl, -C₁₋₆ hydroxyalkyl, -C₁₋₆ haloalkyl, -CN, -NH₂, - NH-C₁₋₆ alkyl, -N(C₁₋₆ alkyl) (C₁₋₆ alkyl), -NO₂, -OH, -O-C₁₋₆ alkyl, or -halo.

According to an embodiment of the present invention, the compounds represented by Chemical Formulas 1 to 3 above, stereoisomers thereof, or pharmaceutically acceptable salts thereof may be selected from the group consisting of compounds according to Examples as described in [Table 1] below, stereoisomers thereof, or pharmaceutically acceptable salts thereof.

In the present invention, unless otherwise specified, the term "alkyl" may refer to a straight or branched chain acyclic, cyclic, or saturated hydrocarbon to which they are bonded. For example, "C₁₋₆alkyl" may indicate an alkyl containing 1 to 6 carbon atoms. As an example, acyclic alkyl may include, but is not limited to, methyl, ethyl, n-propyl, n-butyl, isopropyl, sec-butyl, isobutyl, tert-butyl, or the like. Cyclic alkyl may be used interchangeably with "cycloalkyl" as used herein, and as an example, may include, but is not limited to, cyclopropyl, cyclobutyl, cyclopentyl, cyclohexyl, cycloheptyl, cyclooctyl, or the like.

In the present invention, "alkoxy" may indicate -(O-alkyl) as an alkyl ether group, wherein alkyl is the same as defined above. For example, "C₁₋₆alkoxy" may mean alkoxy containing C₁₋₆alkyl, that is, -(O-C₁₋₆alkyl), and as an example, may include, but is not limited to, methoxy, ethoxy, n-propoxy, isopropoxy, n-butoxy, isobutoxy, sec-butoxy, tert-butoxy, or the like.

In the present invention, "halo" may be F, Cl, Br, or I.

In the present invention, "haloalkyl" may mean a straight or branched chain alkyl (hydrocarbon) having one or more halo-substituted carbon atoms as defined herein. Examples of the haloalkyl may include, but are not limited to, methyl, ethyl, propyl, isopropyl, isobutyl or n-butyl independently substituted with one or more halogens, such as F, Cl, Br, or I.

In the present specification, "hydroxyalkyl" may indicate a straight or branched chain alkyl (hydrocarbon) having a carbon atom substituted with -hydroxy (-OH). Examples of the hydroxyalkyl may include, but are not limited to, methyl, ethyl, propyl, isopropyl, isobutyl or n-butyl independently substituted with one or more, for example -OH.

In the present specification, "aminoalkyl" may mean a straight or branched chain alkyl (hydrocarbon) having a carbon atom substituted with amino (NR'R"). Here, R' and R" may be each independently selected from the group consisting of hydrogen and C₁₋₆alkyl, and the selected R' and R" may be each independently substituted or unsubstituted.

In the present specification, "cyanoalkyl" may refer to a straight or branched chain alkyl (hydrocarbon) having carbon atoms substituted with cyano(CN).

In the present invention, "cycloalkyl" may refer to a hydrocarbon ring that does not contain a hetero atom (N, O, P, P(=O), S, or the like) in the ring, and may be saturated or partially unsaturated. Here, when unsaturated, the cycloalkyl may be referred to as a cycloalkenyl. Unless otherwise stated, the cycloalkyl may be a single ring or multiple rings such as a spiro ring, a bridged ring or a fused ring.

In the present invention, "heterocycloalkyl" may mean a ring containing at least one selected from N, O, P, P(=O), and S in the ring and may be saturated or partially unsaturated. Here, when unsaturated, it may be referred to as a heterocycloalkene. Unless otherwise stated, heterocycloalkyl may be a single ring. In addition, "3- to 12-membered heterocycloalkyl" may indicate a heterocycloalkyl containing 3 to 12 atoms forming a ring. As an example, the heterocycloalkyl may include, but is not limited to, pyrrolidine, piperidine, imidazolidine, pyrazolidine, butyrolactam, valerolactam, imidazolidinone, hydantoin, dioxolane, phthalimide, pyrimidin-2,4(1H,3H)-dione, 1,4-dioxane, morpholine, thiomorpholine, thiomorpholine-S-oxide, thiomorpholine-S,S-oxide, piperazine, pyran, pyridone, 3-pyrroline, thiopyran, pyrone, tetrahydrofuran, tetrahydrothiophene, and the like.

In the present invention, "heterobicycloalkyl" may mean multiple rings such as a spiro ring, a bridged ring or a fused ring containing at least one selected from N, O, P, P(=O), and S in the rings, and may be saturated or partially unsaturated. Herein, when unsaturated, the heterobicycloalkyl may be referred to as a heterobicycloalkene. Examples of the heterobicycloalkyl may include, but are not limited to, quinuclidine, trophane, 2-azaspiro[3.3]heptane, (1r,5s)-3-azabicyclo[3.2.1]octane, (1s,4s)-2-azabicyclo[2.2.2]octane, or (1r,4r)-2-oxa-5-azabicyclo[2.2.2]octane, and the like.

In the present invention, "arene" may mean an aromatic hydrocarbon ring. The arene may be a single ring or multiple rings. The number of ring carbon atoms in the arene may be 5 or more and 30 or less, 5 or more and 20 or less, or 5 or more and 15 or less. Examples of arene may include, but are not limited to, benzene, naphthalene, fluorene, anthracene, phenanthrene, bibenzene, terbenzene, quaterbenzene, quinquebenzene, sexibenzene, triphenylene, pyrene, benzofluoranthene, chrysene, and the like. In the present specification, a moiety obtained by removing one hydrogen atom from the above "arene" is referred to as "aryl".

In the present invention, "heteroarene" may be a ring containing at least one or more of O, N, P, Si, and S as a heterogeneous element. The number of ring-forming carbons in the heteroarene may be 2 or more and 30 or less, or 2 or more and 20 or less. The heteroarene may be a monocyclic heteroarene or a polycyclic heteroarene. The polycyclic heteroarene may have, for example, a bicyclic or tricyclic structure. Examples of the heteroarene may include thiophene, purine, pyrrole, pyrazole, imidazole, thiazole, oxazole, isothiazole, oxadiazole, triazole, pyridine, pyridin-2-one, pyridin-3-one, pyridin-4-one, bipyridyl, triazine, acridyl, pyridazine, pyrazine, quinoline, quinazoline, quinoxaline, phenoxazine, phthalazine, pyrimidine, pyridopyrimidine, pyridopyrazine, pyrazinopyrazine, isoquinoline, indole, carbazole, imidazopyridazine, imidazopyridine, imidazopyrimidine, pyrazolopyrimidine, imidazopyrazine, or pyrazolopyridine, *N-*arylcarbazole, *N*-heteroarylcarbazole, *N*-alkylcarbazole, benzoxazole, benzoimidazole, benzothiazole, benzocarbazole, benzothiophene, dibenzothiophene, thienothiophene, benzofuran, phenanthroline, isoxazole, oxadiazole, thiadiazole, benzothiazole, tetrazole, phenothiazine, dibenzosilole, dibenzofuran, and the like, but are not limited thereto. In an embodiment of the present invention, heteroarene may also include bicyclic heterocyclo-arene containing heteroarene fused to an arene ring or a cycloalkyl ring fused to heterocycloalkyl rings. In the present specification, the residue obtained by removing one hydrogen atom from the "heteroarene" is referred to as "heteroaryl".

In the present invention, "hydroarene" or "hydroaryl" may be a ring saturated one or more double bonds in an aromatic hydrocarbon ring.

In the present invention, "heterohydroarene" or "heterohydroaryl" may be a ring saturated one or more double bonds in a "heteroarene" or "heteroaryl" ring.

In the present invention, "ring" may be a single ring or multiple rings, and the multiple rings may be in the form of a spiro ring, a bridged ring, or a fused ring.

In the present invention, the term "stereoisomer" means a compound of the present invention, having the same chemical formula or molecular formula but different in spatial arrangement. In the present specification, the stereoisomer includes an optical isomer, an enantiomer, a diastereomer, cis/trans isomer, rotamer, and atropisomer. Each of these isomer, racemate and mixture thereof are also included within the scope of the present invention. For example, Chemical Formulas 1 to 3 of the present invention may include stereoisomers of Chemical Formulas 1 to 3 because the stereochemistry is not specified. Unless otherwise specified, a solid bond ( 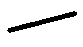 ) connected to an asymmetric carbon atom may include a wedged solid bond ( 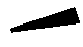 ) or wedge dashed bond ( 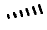 ) representing the absolute arrangement of stereocenters.

The compound represented by Chemical Formulas 1 to 3 of the present invention may be present in the form of a "pharmaceutically acceptable salt". Thus, pharmaceutically acceptable salts of the compound represented by Chemical Formulas 1 to 3 above are included in the scope of the compound of the present invention. The term "pharmaceutically acceptable salt" as used herein refers to a concentration having a relatively non-toxic and harmless effective effect on patients, which includes any organic acid or inorganic acid addition salt of the compound represented by Chemical Formulas 1 to 3 in which side effects caused by these salts do not reduce the beneficial efficacy of the compound.

In particular, the pharmaceutically acceptable salt may be an acid addition salt formed from a free acid. Here, the acid addition salts may be obtained from inorganic acids such as hydrochloric acid, nitric acid, phosphoric acid, sulfuric acid, hydrobromic acid, hydroiodic acid, nitrous acid, phosphorous acid, and the like, non-toxic organic acids such as aliphatic mono- and dicarboxylates, phenyl-substituted alkanoates, hydroxy alkanoates and alkanedioates, aromatic acids, aliphatic and aromatic sulfonic acids, and organic acids such as trifluoroacetic acid, acetate, benzoic acid, citric acid, lactic acid, maleic acid, gluconic acid, methanesulfonic acid, 4-toluenesulfonic acid, tartaric acid, fumaric acid, and the like.

Examples of the pharmaceutically acceptable salt may include sulfate, sulfite, nitrate, phosphate, pyrophosphate, chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, benzoate, phthalate, benzenesulfonate, toluenesulfonate, phenylacetate, phenylpropionate, phenylbutyrate, citrate, lactate, glycolate, malate, tartrate, mandelate, and the like.

The acid addition salt may be prepared by a conventional method, for example, may be prepared by dissolving the compound represented by Chemical Formulas 1 to 3 in an organic solvent such as methanol, ethanol, acetone, methylene chloride, acetonitrile, or the like, and adding an organic acid or an inorganic acid to form a precipitate, followed by filtering and drying the resulting precipitate, or may be prepared by distilling a solvent and excess acid under reduced pressure, followed by drying and crystallizing the product in an organic solvent.

Further, the pharmaceutically acceptable salt may be a salt obtained using a base or a metal salt. As an example of the metal salt, an alkali metal salt or an alkaline earth metal salt may be obtained by dissolving the compound in an excess alkali metal hydroxide or alkaline earth metal hydroxide solution, and filtering an insoluble compound salt, followed by evaporating and drying the filtrate. Sodium, potassium or calcium salts may be pharmaceutically suitable as alkali metal salts. In addition, the corresponding silver salt may be obtained by reacting an alkali metal or alkaline earth metal with a suitable silver salt (for example, silver nitrate) or may be prepared by salt production methods known in the art.

### Uses of piperidine derivative compounds

The present invention provides uses of the following compounds represented by Chemical Formulas 1 to 3, stereoisomers thereof, or pharmaceutically acceptable salts thereof:

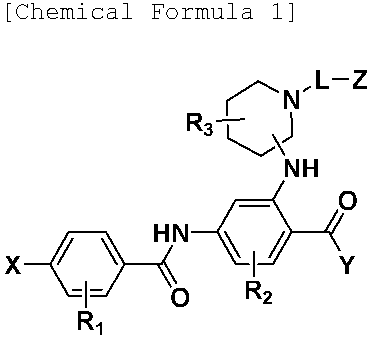

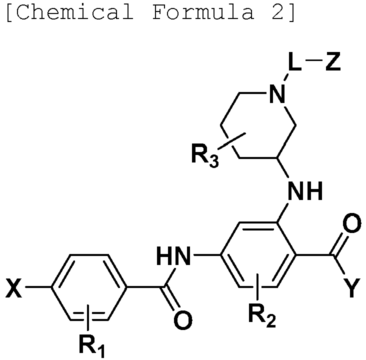

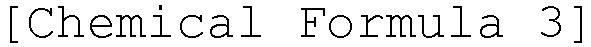

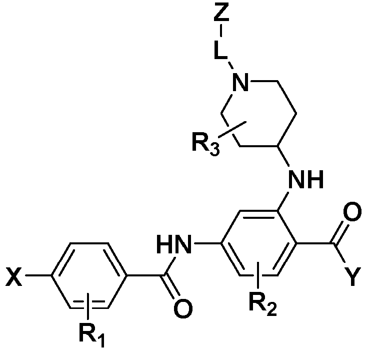

The Chemical Formulas 1 to 3 above are as defined above.

The compound represented by Chemical Formulas 1 to 3 of the present invention, the stereoisomer thereof, or the pharmaceutically acceptable salt thereof exhibits inhibitory activity against FLT3.

According to an embodiment of the present invention, the piperidine derivative represented by Chemical Formulas 1 to 3 exhibit excellent inhibitory activity against FLT3, thereby being usefully employed for treatment or prevention of FLT3-related diseases, in particular, cancer.

In the present invention, the cancer includes all cancers capable of exhibiting therapeutic or preventive efficacy by inhibition of FLT3 activity, and may be solid cancer or blood cancer (hematologic malignancy). For example, the cancer may be at least one selected from the group consisting of liver cancer, thyroid cancer, colon cancer, myelodysplastic syndrome, glioblastoma, acute myeloid leukemia, acute lymphoblastic leukemia, multiple myeloma, colorectal cancer, chronic myeloid leukemia, chronic lymphocytic leukemia, adrenal cancer, non-small cell lung cancer, small cell lung cancer, pediatric lymphoma, pediatric leukemia, glioma, kidney cancer, malignant bone cancer, malignant lymphoma, malignant melanoma, stomach cancer, breast cancer, prostate cancer, rectal cancer, pancreatic cancer, lung cancer, and squamous cell carcinoma of the lung, but is not limited thereto. Further, the cancer includes not only primary cancer but also metastatic cancer.

According to an embodiment of the present invention, the present invention provides a pharmaceutical composition for treatment or prevention of FLT3-related diseases containing the compound represented by Chemical Formulas 1 to 3, the stereoisomer thereof, or the pharmaceutically acceptable salt thereof as an active ingredient. Specifically, the FLT3-related disease may be cancer. The types of cancer are the same as described above.

The pharmaceutical composition of the present invention may further include one or more active ingredients exhibiting the same or similar drug efficacy in addition to the compound represented by Chemical Formulas 1 to 3 above, the stereoisomer thereof, or the pharmaceutically acceptable salt thereof.

The pharmaceutical composition of the present invention can be used for clinical administration, and may be prepared to be administrated in various oral and parenteral dosage forms.

In addition, according to an embodiment of the present invention, the present invention provides use of the compound represented by Chemical Formulas 1 to 3 above, the stereoisomer thereof, or the pharmaceutically acceptable salt thereof for the manufacture of a medicament for the treatment or prevention of FLT3-related diseases. Specifically, the FLT3-related diseases may be a cancer. The types of cancer are the same as described above.

In addition, according to an embodiment of the present invention, the present invention provides use of the compound represented by Chemical Formulas 1 to 3 above, the stereoisomer thereof, or the pharmaceutically acceptable salt thereof for use in preparation of a medicament to treat or prevent diseases of cancer. The types of cancer are the same as described above.

In addition, according to an embodiment of the present invention, the present invention provides a method for treating or preventing FLT3-related diseases, comprising: administering a therapeutically effective amount of the compound represented by Chemical Formulas 1 to 3 above, the stereoisomer thereof, or the pharmaceutically acceptable salt thereof to a subject in need thereof. The subject may be a mammal including a human. Specifically, the FLT3-related diseases may be cancer. The types of cancer are the same as described above.

In addition, according to an embodiment of the present invention, the present invention provides a method for treating or preventing cancer, comprising: administering a therapeutically effective amount of the compound represented by Chemical Formulas 1 to 3, the stereoisomer thereof, or the pharmaceutically acceptable salt thereof to a subject in need thereof. The types of cancer are the same as described above.

In addition, according to an embodiment of the present invention, the present invention provides a method for inhibiting FLT3, comprising: administering a therapeutically effective amount of the compound represented by Chemical Formulas 1 to 3, the stereoisomer thereof, or the pharmaceutically acceptable salt thereof to a subject in need thereof.

The term "therapeutically effective amount" as used herein refers to an amount of the compound represented by Chemical Formulas 1 to 3 that is effective for the treatment or prevention of FLT3-related diseases. Specifically, "therapeutically effective amount" indicates an amount sufficient to treat a disease with a reasonable benefit/risk ratio applicable to medical treatment, and the effective dose level may be determined depending on factors including the subject type and severity, age, sex, type of disease, drug activity, drug sensitivity, administration time, administration route and excretion rate, treatment period, drugs used at the same time, and other factors well-known in medical fields. The pharmaceutical composition of the present invention may be administered as an individual therapeutic agent or may be administered in combination with other therapeutic agents, and may be administered sequentially or simultaneously with commercially available therapeutic agents. In addition, the pharmaceutical composition of the present invention may be administered in a single dose or multiple doses. It is important to administer the minimum amount capable of obtaining the maximum effect without side effects in consideration of all of the above factors, and the amount may be readily determined by those skilled in the art. The dosage of the pharmaceutical composition of the present invention may be determined by a medical specialist according to various factors such as the patient's condition, age, sex, complications, and the like. Since the active ingredient of the pharmaceutical composition of the present invention has excellent safety, it may be used at a dose higher than the determined dosage.

As used herein, term "prevention" refers to any action that suppresses or delays the occurrence, spread, and recurrence of the disease by administering the compound, and term "treatment" refers to any action in which the symptoms of the disease are improved or beneficially changed by administration of the compound.

Further, according to an embodiment of the present invention, the pharmaceutical composition may further comprise a pharmaceutically acceptable carrier, diluent, or excipient. In an embodiment, the present invention provides a pharmaceutical composition comprising the compound represented by Chemical Formulas 1 to 3, or the pharmaceutically acceptable salt or the stereoisomer thereof, and a pharmaceutically acceptable additive.

Examples of additives used in the pharmaceutical composition may include sweeteners, binders, solvents, solubilizers, wetting agents, emulsifiers, isotonic agents, absorbents, disintegrants, antioxidants, preservatives, lubricants, fillers, flavoring agents, and the like. For example, the additives may include lactose, dextrose, sucrose, mannitol, sorbitol, cellulose, glycine, silica, talc, stearic acid, stearin, magnesium stearate, magnesium aluminosilicate, starch, gelatin, gum tragacanth, alginic acid, sodium alginate, methylcellulose, sodium carboxymethylcellulose, agar, water, ethanol, polyethylene glycol, polyvinylpyrrolidone, sodium chloride, calcium chloride, orange essence, strawberry essence, vanilla flavor, and the like.

The pharmaceutical composition may be combined in various formulation forms for oral administration (for example, tablets, pills, powders, capsules, syrups or emulsions) or parenteral administration (for example, intramuscular, intravenous or subcutaneous injection).

For example, the pharmaceutical composition may be combined into preparations for oral administration, and additives used at this time may include cellulose, calcium silicate, corn starch, lactose, sucrose, dextrose, calcium phosphate, stearic acid, magnesium stearate, calcium stearate, gelatin, talc, surfactants, suspending agents, emulsifiers, diluents, and the like. Specifically, solid preparations for oral administration may include tablets, pills, powders, granules, capsules, and the like, and these solid preparations may be formulated by mixing at least one excipient in the composition, such as starch, calcium carbonate, sucrose, lactose, gelatin, etc. In addition, lubricants such as magnesium stearate and talc may be used in addition to simple excipients. Further, examples of liquid preparations for oral administration may include suspensions, emulsions, syrups, and the like. Various excipients such as wetting agents, sweeteners, aromatics, preservatives, and the like, may be included in addition to water and liquid paraffin that are commonly used simple diluents.

Further, preparations for parenteral administration may include sterilized aqueous solutions, non-aqueous solutions, suspensions, emulsions, freeze-dried preparations, and suppositories. As the non-aqueous solvents and the suspensions, propylene glycol, polyethylene glycol, vegetable oils such as olive oil, and injectable esters such as ethyl oleate, and the like, may be used. As the base of the suppository, witepsol, macrogol, Tween 61, cacao butter, laurin butter, glycerogelatin and the like, may be used. Meanwhile, conventional additives such as solubilizers, isotonic agents, suspending agents, emulsifiers, stabilizers, preservatives, and the like, may be contained in the injection.

Further, the pharmaceutical composition may have a synergistic effect of active ingredients by being prepared as a complex formulation with other active agents.

Matters mentioned in the uses, compositions and treatment methods of the present invention are applied equally except to the extent that they are inconsistent with each other.

The embodiments of the present invention may be modified in various different forms, and the scope of the present invention is not limited to the embodiments described below. In addition, the embodiments of the invention are provided to more fully explain the present invention to those skilled in the art. Furthermore, the use of the term "comprising" with respect to any component throughout the specification should not be interpreted as excluding the presence of additional components, unless expressly stated otherwise, but rather means that other components may also be included.

### [Advantageous Effects]

The piperidine derivative compound of the present invention exhibits excellent inhibitory activity against FLT3, and thus may be usefully employed for the treatment or prevention of FLT3-related diseases.

### [Best Mode]

Hereinafter, the present invention will be described in more detail through Examples and Experimental Examples. However, the following Examples and Experimental Examples are only provided to illustrate the present invention, and the content of the present disclosure is not limited to these Examples.

### <Analysis and Purification Conditions>

The synthesized compounds were purified by recrystallization in ether solvent or by medium pressure liquid chromatography (MPLC) using a combination of hexane and ethyl acetate as an eluent. Then, structures thereof were confirmed using analytical techniques such as ¹H NMR, LC/MS, and IR.

The commercially available reagents used were used without further purification. In the present invention, room temperature refers to a temperature of about 20°C to 25°C, and ambient temperature refers to a temperature of 15°C to 25°C. Concentration under reduced pressure or solvent removal by distillation was performed using a rotary evaporator.

### <General Preparation Method>

The compounds of the present invention may be prepared using the Reaction Schemes A to E shown below.

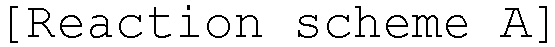

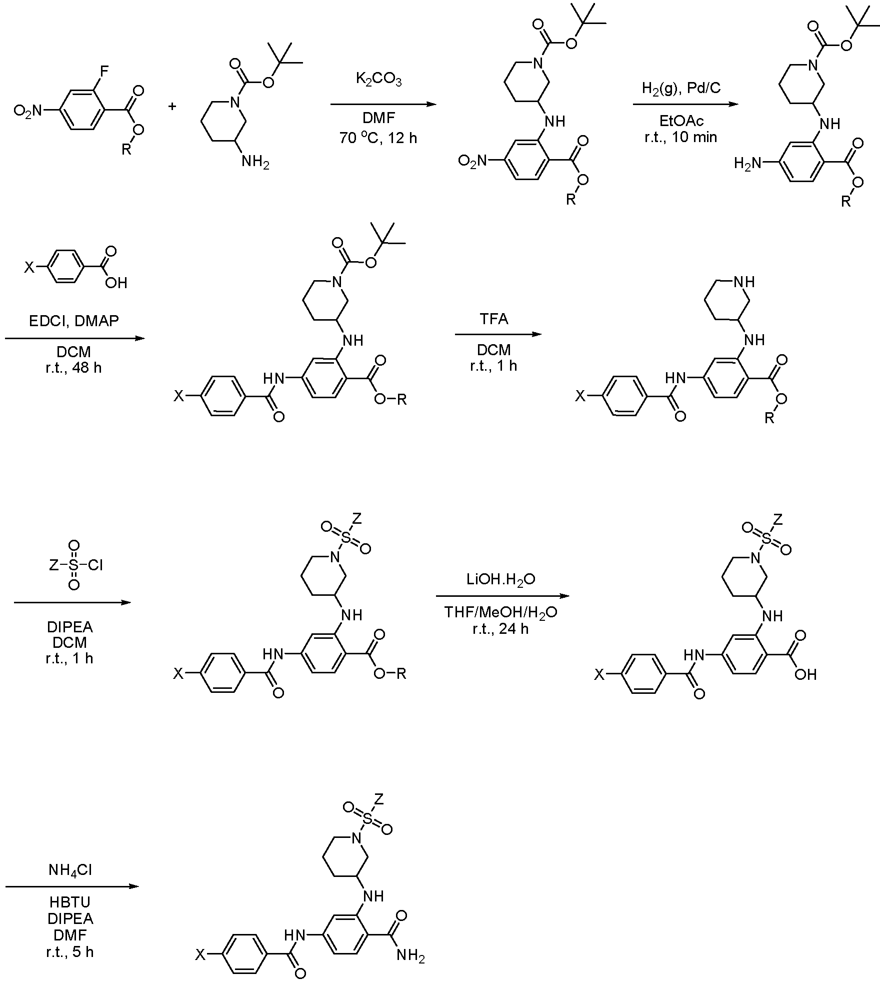

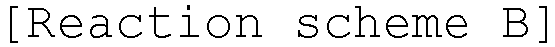

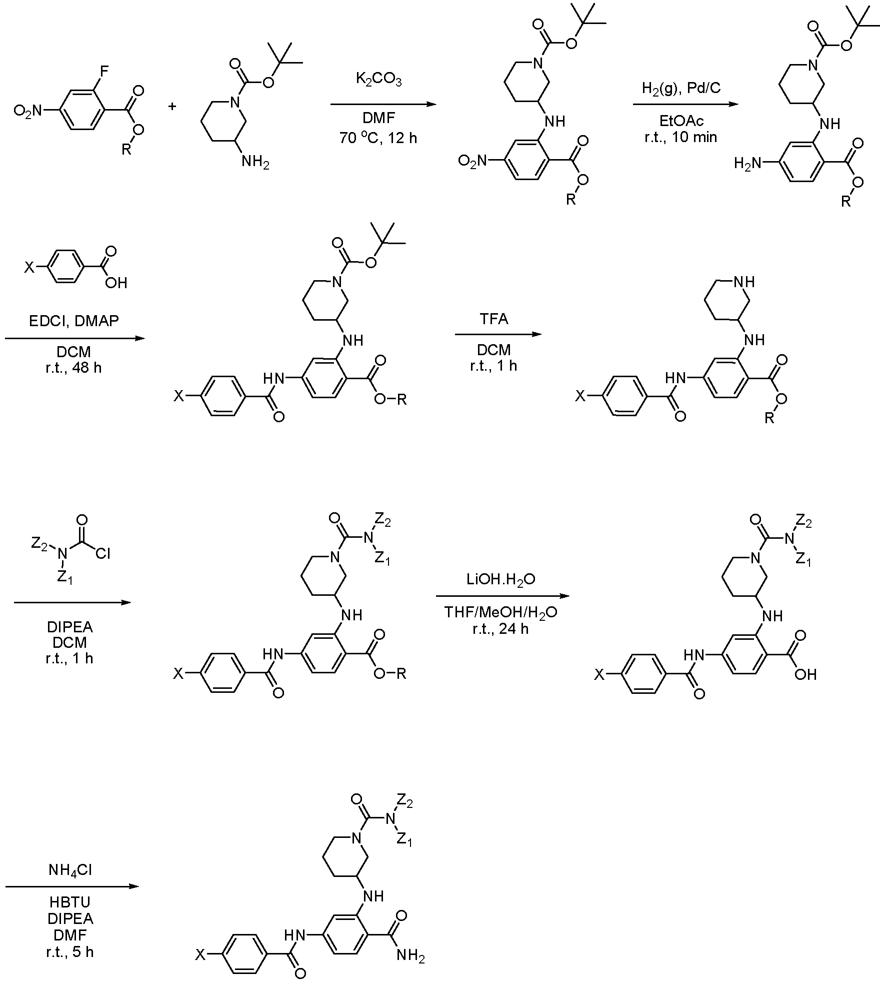

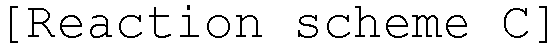

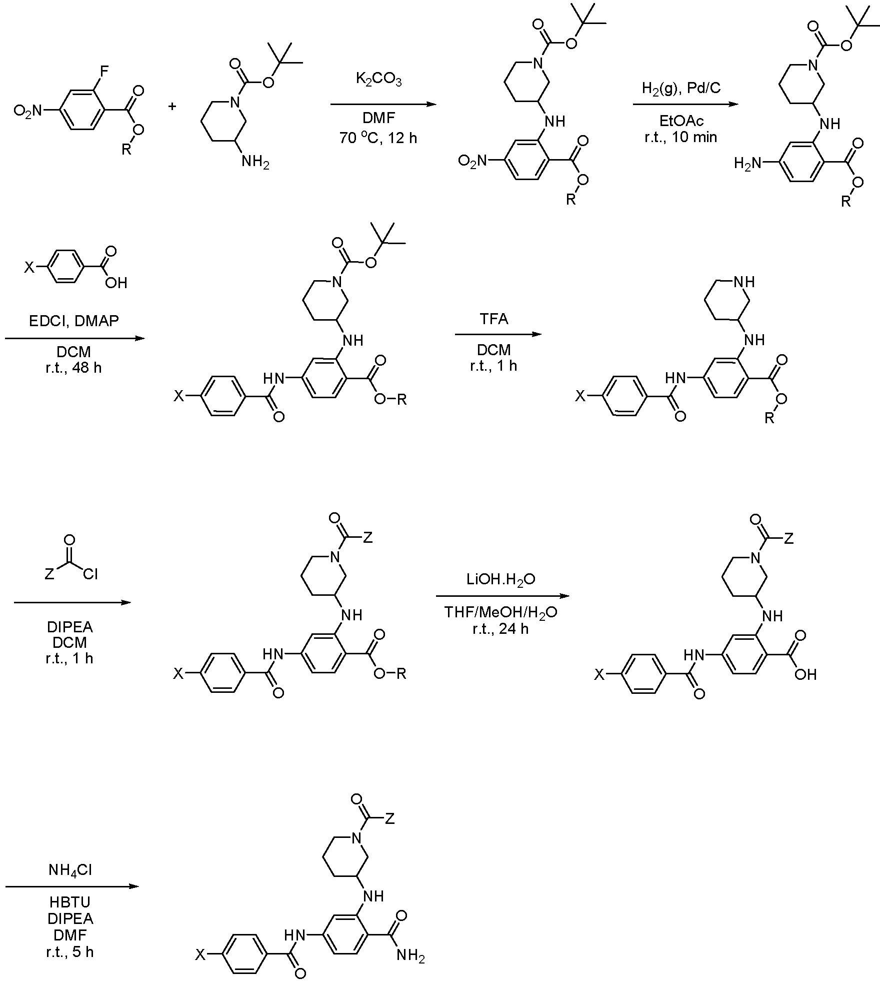

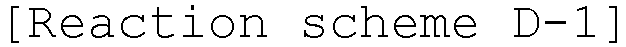

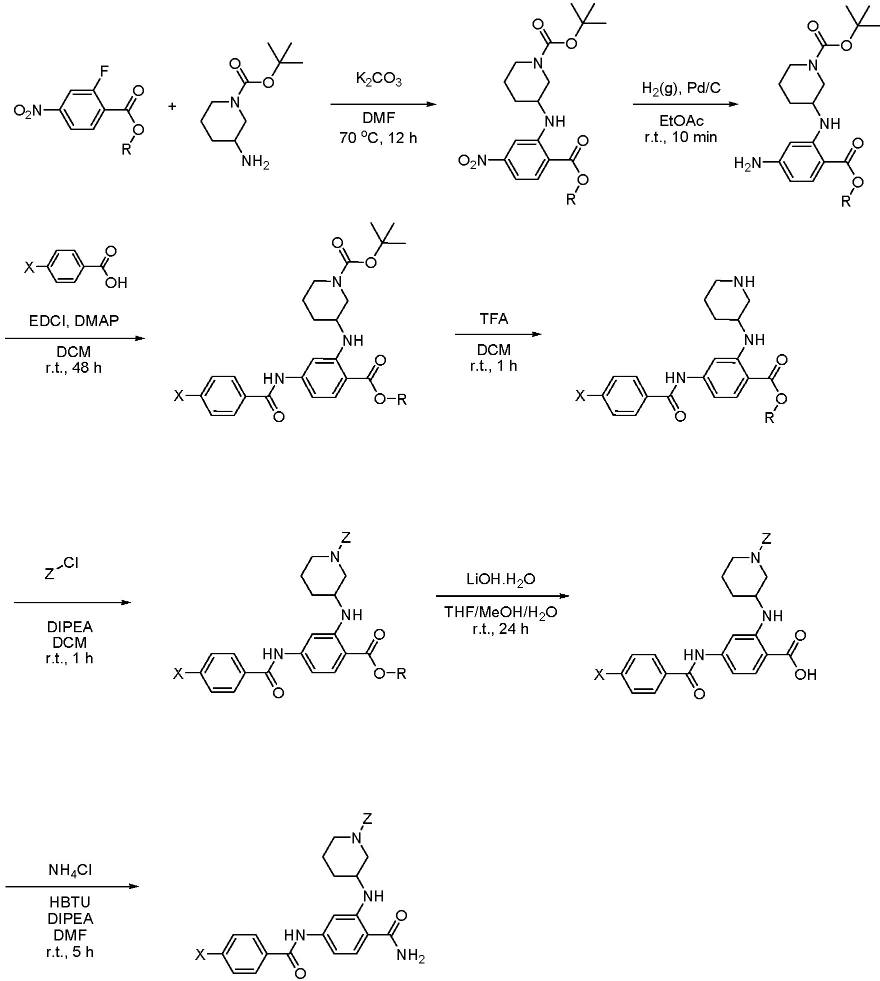

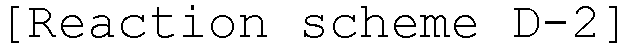

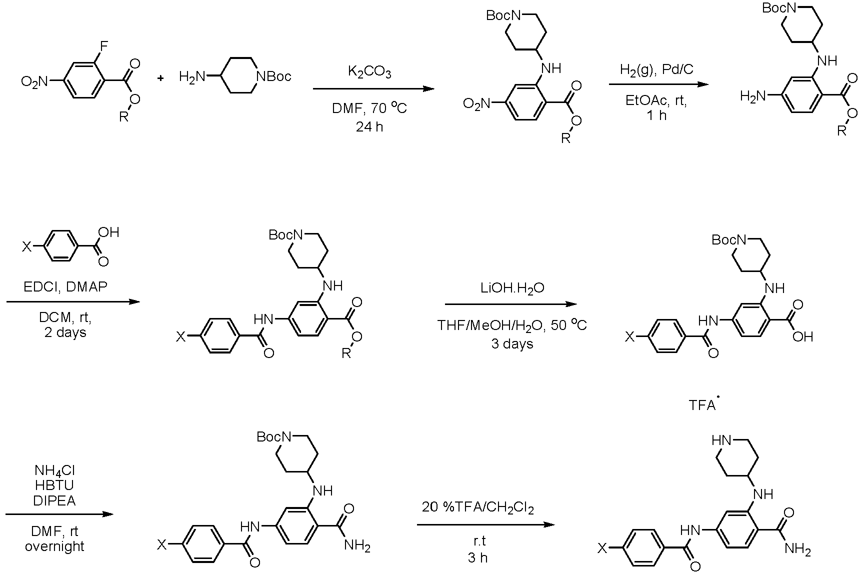

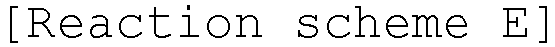

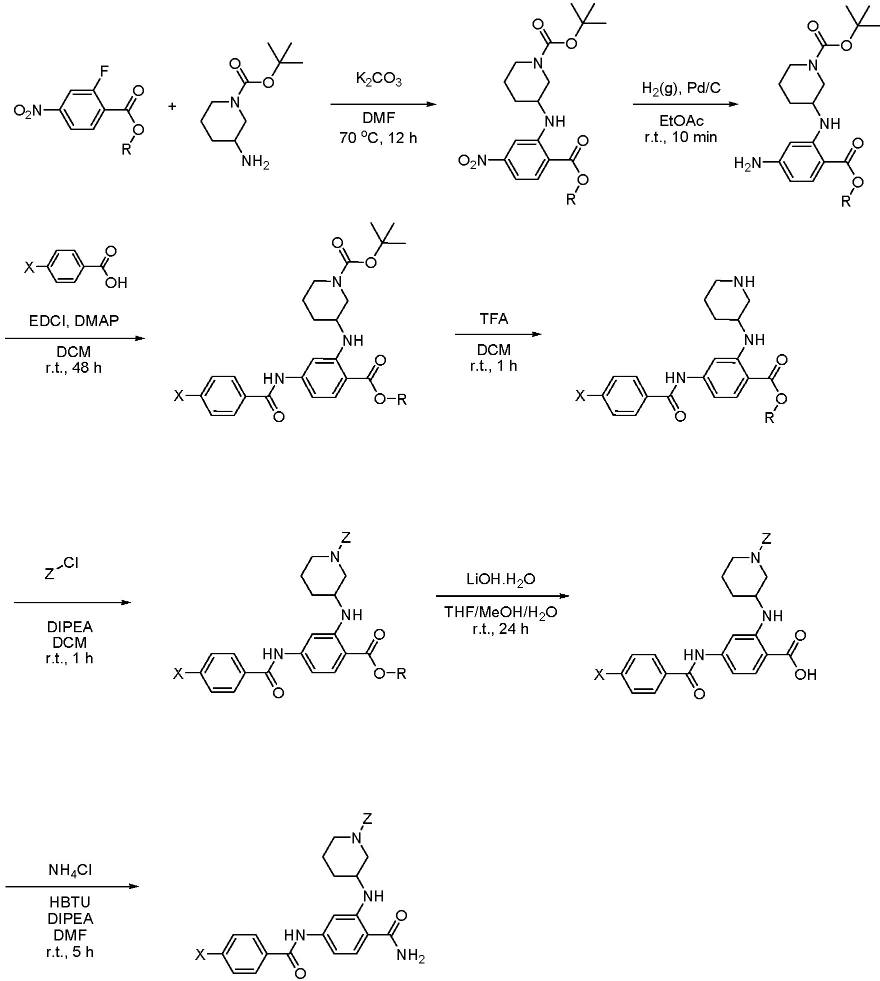

### Preparation Example 1: Synthesis of tert-butyl 3-((2-(methoxycarbonyl)-5-nitrophenyl)amino)piperidine-1-carboxylate

Methyl 2-fluoro-4-nitrobenzoate (5.0 g, 25.1 mmol) and tert-butyl 3-aminopiperidine-1-carboxylate (5.5 g, 27.6 mmol) were dissolved in anhydrous DMF (100 mL), and K₂CO₃ (6.9 g, 50.2 mmol) was added and stirred at 70°C for 12 hours. The reaction product was confirmed by TLC, and the reaction solution was diluted with EtOAc and washed with an aqueous NaHCO₃ solution. The organic layer was dried over anhydrous Na₂SO₄, concentrated under reduced pressure, and separated by column chromatography to obtain the target compound (6.01 g).

¹H-NMR (400 MHz, DMSO-d₆) δ 8.06 (t, *J* = 9.0 Hz, 2H), 7.58-7.49 (m, 1H), 7.36 (dd, *J* = 8.7, 2.2 Hz, 1H), 3.85 (s, 3H), 3.76 (m, *J* = 7.2, 3.8 Hz, 1H), 3.50 (s, 4H), 1.94 (m, *J* = 12.8, 8.3, 3.9 Hz, 1H), 1.81-1.49 (m, 4H), 1.31 (d, *J* = 70.9 Hz, 9H); LC-MS (ESI, m/z) = 380.0 (M+H⁺).

### Preparation Example 2: Synthesis of tert-butyl 3-((5-amino-2-(methoxycarbonyl)phenyl)amino)piperidine-1-carboxylate

The compound prepared in Preparation Example 1 (2.0 g, 5.30 mmol) was dissolved in anhydrous EtOAc (120 mL), Pd/C (56 mg, 0.50 mmol) was added, and the reaction product was reacted for 10 minutes in the presence of H₂ gas. The reaction product was confirmed by TLC, then the reaction solution was passed through a Celite filter to remove residual Pd/C, and the filtrate was concentrated to obtain the target compound (1.68 g).

¹H-NMR (400 MHz, DMSO-d₆) δ 8.15 (d, *J* = 7.5 Hz, 1H), 8.07 (d, *J* = 8.7 Hz, 1H), 7.57 (d, *J* = 2.2 Hz, 1H), 7.38 (d, *J* = 8.8, 2.2 Hz, 1H), 3.91 (s, 3H), 3.87-3.82 (m, 1H), 3.62 (s, 2H), 3.39-3.16 (m, 2H), 2.08 (d, *J* = 12.7 Hz, 1H), 1.90-1.61 (m, 3H), 1.44 (s, 9H); LC-MS (ESI, m/z) = 350.1 (M+H⁺).

### Preparation Example 3: Synthesis of tert-butyl 3-((5-(4-(tert-butyl)benzamido)-2-(methoxycarbonyl)phenyl)amino)piperidine-1-carboxylate

The compound prepared in Preparation Example 2 (800 mg, 2.29 mmol) and 4-(tert-butyl)benzoic acid (408 mg, 2.29 mmol) were dissolved in anhydrous DCM (23 mL), and then EDCI (426 mg, 2.75 mmol) and DMAP (28 mg, 0.23 mmol) were added and stirred at ambient temperature for 48 hours. The reaction product was confirmed by TLC, then diluted with EtOAc and washed with an aqueous NaHCO₃ solution. The organic layer was dried over anhydrous Na₂SO₄, concentrated under reduced pressure, and separated by column chromatography to obtain the target compound (1.06 g).

¹H-NMR (300 MHz, DMSO-d₆) δ 10.23 (s, 1H), 7.88 (d, *J* = 8.4 Hz, 3H), 7.78 (d, *J* = 8.8 Hz, 1H), 7.56 (d, *J* = 8.4 Hz, 2H), 7.05 (s, 1H), 3.76 (s, 3H), 1.95 (s, 1H), 1.68 (s, 2H), 1.54 (s, 2H), 1.33 (s, 9H), 1.24 (s, 9H); LC-MS (ESI, m/z) = 510.1 (M+H⁺).

### Preparation Example 4: Synthesis of methyl 4-(4-(tert-butyl)benzamido)-2-(piperidin-3-yl)amino)benzoate

The compound prepared in Preparation Example 3 (500 mg, 1.96 mmol) was dissolved in anhydrous DCM (20 mL), TFA (4 mL) was added, and the mixture was stirred at ambient temperature for 1 hour. The reaction product was confirmed by TLC and concentrated under reduced pressure to remove the reaction solvent. Then, cold diethyl ether was added, and the resulting solid was filtered under reduced pressure to obtain the target compound (310 mg).

¹H-NMR (300 MHz, DMSO-d₆) δ 10.22 (s, 1H), 7.84 (d, *J* = 8.4 Hz, 3H), 7.72 (d, *J* = 8.8 Hz, 1H), 7.60 (d, *J* = 8.4 Hz, 2H), 7.04 (s, 1H), 3.35 (s, 3H), 1.96 (s, 1H), 1.64 (s, 2H), 1.50 (s, 2H), 1.42 (s, 9H); MS (ESI, m/z) = 410.3 (M+H⁺).

### Example A-1: Synthesis of methyl 4-(4-(tert-butyl)benzamido)-2-((1-(N,N-dimethylsulfamoyl)piperidin-3-yl)amino)benzoate

The compound prepared in Preparation Example 4 (300 mg, 0.59 mmol) was dissolved in DCM (6 mL), and then DIPEA (179 mg, 1.77 mmol) and dimethylsulfamoyl chloride (169 mg, 1.18 mmol) were sequentially added, followed by stirring at ambient temperature for 1 hour. The reaction product was confirmed by TLC, then diluted with EtOAc and washed with an aqueous NaHCO₃ solution. The organic layer was dried over anhydrous Na₂SO₄, concentrated under reduced pressure, and separated by column chromatography to obtain the target compound (151 mg).

¹H NMR (300 MHz, DMSO-d₆) δ 10.26 (s, 1H), 7.93 (d, *J* = 8.1 Hz, 1H), 7.91-7.85 (m, 2H), 7.79 (d, *J* = 8.8 Hz, 1H), 7.60-7.52 (m, 2H), 7.46 (d, *J* = 1.9 Hz, 1H), 7.07 (dd, *J* = 8.8, 1.8 Hz, 1H), 3.78 (s, 3H), 3.67 (s, 1H), 3.43 (m, *J* = 11.8, 3.2 Hz, 1H), 3.20 (d, *J* = 5.2 Hz, 2H), 3.11 (m, *J* = 11.9, 6.5 Hz, 1H), 1.95-1.51 (m, 4H), 1.33 (s, 9H); LC-MS (ESI, m/z) = 517.4 (M+H⁺).

### Example A-2: Synthesis of 4-(4-(tert-butyl)benzamido)-2-((1-(N,N-dimethylsulfamoyl)piperidin-3-yl)amino)benzoic acid

The compound obtained in Example A-1 (120 mg, 0.23 mmol) was dissolved in 5 mL of a THF/MeOH/H₂O mixture (mixing ratio = 3 : 1 : 1), and LiOH•H₂O (30 mg, 0.70 mmol) was added and stirred at ambient temperature for 24 hours. The reaction product was confirmed by TLC, and 1M HCl aqueous solution was added dropwise so that the pH of the reaction solution was 2 to 3. The resulting white solid was filtered to obtain the target compound (51 mg).

¹H NMR (300 MHz, DMSO-d₆) δ 12.44 (s, 1H), 10.24 (s, 1H), 8.14 (d, *J* = 8.0 Hz, 1H), 7.96-7.83 (m, 2H), 7.77 (d, *J* = 8.7 Hz, 1H), 7.63-7.49 (m, 2H), 7.43 (d, *J* = 1.9 Hz, 1H), 7.04 (dd, *J* = 8.8, 1.8 Hz, 1H), 3.66 (s, 1H), 3.43 (d, *J* = 3.1 Hz, 1H), 3.17 (s, 2H), 3.10 (dd, *J* = 11.7, 6.3 Hz, 1H), 2.77 (s, 6H), 1.94-1.54 (m, 4H), 1.33 (s, 9H); LC-MS (ESI, m/z) = 503.4 (M+H⁺).

### Example A-3: Synthesis of 4-(tert-butyl)-N-(4-carbamoyl-3-((1-(N,N-dimethylsulfamoyl)piperidin-3-yl)amino)phenyl)benzamide

The compound obtained in Example A-2 (40 mg, 0.08 mmol) and NH₄Cl (12 mg, 0.24 mmol) were dissolved in DMF (4 mL), then DIPEA (30 mg, 0.24 mmol) and HBTU (45 mg, 0.12 mmol) were added and stirred at ambient temperature for 5 hours. The reaction product was confirmed by TLC, then diluted with EtOAc and washed with an aqueous NaHCO₃ solution. The organic layer was dried over anhydrous Na₂SO₄, concentrated under reduced pressure, and separated by column chromatography to obtain the target compound (38 mg).

¹H NMR (300 MHz, DMSO-d₆) δ 10.12 (s, 1H), 8.44 (d, *J* = 7.8 Hz, 1H), 7.88 (d, *J* = 8.4 Hz, 2H), 7.58 (dd, *J* = 15.7, 8.5 Hz, 3H), 7.25 (d, *J* = 2.0 Hz, 1H), 7.03 (dd, *J* = 8.7, 1.8 Hz, 2H), 3.91 (d, *J* = 12.7 Hz, 1H), 3.70 (d, *J* = 13.0 Hz, 1H), 3.14-2.96 (m, 2H), 2.87 (t, *J* = 10.9 Hz, 1H), 2.75 (dd, *J* = 12.8, 8.8 Hz, 1H), 2.02 (d, *J* = 11.2 Hz, 1H), 1.65 (s, 1H), 1.47-1.39 (m, 1H), 1.33 (s, 9H), 0.99 (t, *J* = 7.1 Hz, 3H); LC-MS (ESI, m/z) = 502.4 (M+H⁺).

### Example A-4: Synthesis of methyl 4-(4-(tert-butyl)benzamide)-2-((1-(cyclopropylsulfonyl)piperidin-3-yl)amino)benzoate

The compound prepared in Preparation Example 4 (200 mg, 0.40 mmol) and cyclopropanesulfonyl chloride (72 mg, 0.51 mmol) were reacted in the same manner as in Example A-1 to obtain the target compound (70 mg).

¹H NMR (300 MHz, DMSO-d₆) δ 10.27 (s, 1H), 7.97-7.84 (m, 3H), 7.80 (d, *J* = 8.8 Hz, 1H), 7.60-7.51 (m, 2H), 7.42 (d, *J* = 1.9 Hz, 1H), 7.13 (dd, *J* = 8.9, 1.8 Hz, 1H), 3.79 (s, 3H), 3.69 (s, 1H), 3.53 (dd, *J* = 11.6, 3.2 Hz, 1H), 3.31-3.01 (m, 3H), 2.68-2.54 (m, 1H), 1.96-1.70 (m, 2H), 1.69-1.49 (m, 2H), 1.33 (s, 9H), 1.08-0.84 (m, 4H); LC-MS (ESI, m/z) = 514.4 (M+H⁺).

### Example A-5: Synthesis of 4-(4-(tert-butyl)benzamido)-2-((1-(cyclopropylsulfonyl)piperidin-3-yl)amino)benzoic acid

The compound obtained in Example A-4 (140 mg, 0.27 mmol) was reacted in the same manner as in Example A-2 to obtain the target compound (115 mg).

¹H NMR (300 MHz, DMSO-d₆) δ 12.43 (s, 1H), 10.24 (s, 1H), 8.10 (d, *J* = 8.1 Hz, 1H), 7.92-7.83 (m, 2H), 7.77 (d, *J* = 8.7 Hz, 1H), 7.60-7.52 (m, 2H), 7.38 (d, *J* = 1.9 Hz, 1H), 7.09 (dd, *J* = 8.8, 1.8 Hz, 1H), 3.73-3.50 (m, 2H), 3.16 (d, *J* = 8.0 Hz, 1H), 3.03 (dd, *J* = 11.5, 7.2 Hz, 1H), 2.62 (m, *J* = 12.4, 7.7, 5.1 Hz, 1H), 1.92 (d, *J* = 5.5 Hz, 1H), 1.78 (s, 1H), 1.71-1.50 (m, 2H), 1.33 (s, 9H), 1.06-0.82 (m, 5H); LC-MS (ESI, m/z) = 500.4 (M+H⁺).

### Example A-6: Synthesis of 4-(tert-butyl)-N-(4-carbamoyl-3-((1-(cyclopropylsulfonyl)piperidin-3-yl)amino)phenyl)benzamide

The compound obtained in Example A-5 (40 mg, 0.08 mmol) was reacted in the same manner as in Example A-3 to obtain the target compound (12 mg).

¹H NMR (400 MHz, DMSO-d₆) δ 10.15 (s, 1H), 8.57 (d, *J* = 8.0 Hz, 1H), 7.95-7.81 (m, 2H), 7.74 (s, 1H), 7.61 (d, *J* = 8.7 Hz, 1H), 7.58-7.51 (m, 2H), 7.28 (d, *J* = 2.0 Hz, 1H), 7.06 (m, *J* = 9.4, 4.7 Hz, 2H), 3.57 (d, *J* = 9.8 Hz, 3H), 3.08 (t, *J* = 8.2 Hz, 1H), 2.92 (dd, *J* = 11.9, 8.0 Hz, 1H), 2.69 (s, 2H), 2.65-2.57 (m, 2H), 1.95 (d, *J* = 13.6 Hz, 1H), 1.79 (d, *J* = 3.2 Hz, 1H), 1.62 (dd, *J* = 9.1, 3.9 Hz, 1H), 1.54-1.43 (m, 1H), 1.33 (s, 9H), 1.01-0.86 (m, 4H); LC-MS (ESI, m/z) = 499.3 (M+H⁺).

### Example A-7: Synthesis of methyl 4-(4-(tert-butyl)benzamido)-2-((1-(phenylsulfonyl)piperidin-3-yl)amino)benzoate

The compound prepared in Preparation Example 4 (100 mg, 0.20 mmol) and benzenesulfonyl chloride (41 mg, 0.24 mmol) were reacted in the same manner as in Example A-1 to obtain the target compound (97 mg).

¹H NMR (300 MHz, DMSO-d₆) δ 10.24 (s, 1H), 7.95-7.83 (m, 2H), 7.77 (dd, *J* = 8.5, 1.5 Hz, 3H), 7.73-7.70 (m, 1H), 7.69-7.62 (m, 2H), 7.61-7.52 (m, 2H), 7.37 (d, *J* = 1.9 Hz, 1H), 7.10 (dd, *J* = 8.7, 1.8 Hz, 1H), 3.64 (s, 1H), 3.08 (s, 2H), 2.93-2.53 (m, 3H), 1.77 (s, 2H), 1.66-1.43 (m, 2H), 1.33 (s, 9H); LC-MS (ESI, m/z) = 550.5 (M+H⁺).

### Example A-8: Synthesis of 4-(4-(tert-butyl)benzamido)-2-((1-(phenylsulfonyl)piperidin-3-yl)amino)benzoic acid

The compound obtained in Example A-7 (97 mg, 0.18 mmol) was reacted in the same manner as in Example A-2 to obtain the target compound (50 mg).

¹H NMR (300 MHz, DMSO-d₆) δ 10.24 (s, 1H), 7.95-7.83 (m, 2H), 7.77 (dd, *J* = 8.5, 1.5 Hz, 3H), 7.73-7.70 (m, 1H), 7.69-7.62 (m, 2H), 7.61-7.52 (m, 2H), 7.37 (d, *J* = 1.9 Hz, 1H), 7.10 (dd, *J* = 8.7, 1.8 Hz, 1H), 3.64 (s, 1H), 3.08 (s, 2H), 2.93-2.53 (m, 3H), 1.77 (s, 2H), 1.66-1.43 (m, 2H), 1.33 (s, 9H); LC-MS (ESI, m/z) = 536.4 (M+H⁺).

### Example A-9: Synthesis of 4-(tert-butyl)-N-(4-carbamoyl-3-((1-(phenylsulfonyl)piperidin-3-yl)amino)phenyl)benzamide

The compound obtained in Example A-8 (40 mg, 0.08 mmol) was reacted in the same manner as in Example A-3 to obtain the target compound (38 mg).

¹H NMR (300 MHz, DMSO-d₆) δ 7.96 (s, 3H), 7.92-7.86 (m, 1H), 7.80-7.53 (m, 4H), 7.32-7.19 (m, 1H), 7.14-7.03 (m, 1H), 2.90 (s, 9H), 2.74 (d, *J* = 0.6 Hz, 8H), 2.69 (s, 1H), 1.33 (s, 4H), 1.28-1.20 (m, 3H); LC-MS (ESI, m/z) = 535.4 (M+H⁺).

### Example A-10: Synthesis of methyl 4-(4-(tert-butyl)benzamido)-2-((1-(ethylsulfonyl)piperidin-3-yl)amino)benzoate

The compound prepared in Preparation Example 4 (300 mg, 0.59 mmol) and ethanesulfonyl chloride (91 mg, 0.71 mmol) were reacted in the same manner as in Example A-1 to obtain the target compound (154 mg).

¹H NMR (400 MHz, DMSO-d₆) δ 10.27 (s, 1H), 7.88 (dd, *J* = 8.0, 4.0 Hz, 3H), 7.79 (d, *J* = 8.8 Hz, 1H), 7.56 (d, *J* = 8.2 Hz, 2H), 7.44 (d, *J* = 2.0 Hz, 1H), 7.11 (dd, *J* = 8.8, 1.9 Hz, 1H), 3.79 (s, 3H), 3.65 (s, 1H), 3.52 (dd, *J* = 11.7, 3.3 Hz, 1H), 3.31-3.23 (m, 1H), 3.17 (d, *J* = 5.3 Hz, 1H), 3.08 (m, *J* = 7.0, 2.9 Hz, 3H), 1.92 (t, *J* = 10.4 Hz, 1H), 1.76 (s, 1H), 1.62 (t, *J* = 8.4 Hz, 2H), 1.33 (s, 9H), 1.22 (t, *J* = 7.3 Hz, 3H); LC-MS (ESI, m/z) = 502.4 (M+H⁺).

### Example A-11: Synthesis of 4-(4-(tert-butyl)benzamido)-2-((1-(ethylsulfonyl)piperidin-3-yl)amino)benzoic acid

The compound obtained in Example A-10 (150 mg, 0.30 mmol) was reacted in the same manner as in Example A-2 to obtain the target compound (89 mg).

¹H NMR (300 MHz, DMSO-d₆) δ 12.43 (s, 1H), 10.24 (s, 1H), 8.08 (d, J = 7.9 Hz, 1H), 7.97-7.83 (m, 2H), 7.77 (d, *J* = 8.8 Hz, 1H), 7.65-7.47 (m, 2H), 7.40 (d, *J* = 1.9 Hz, 1H), 7.07 (dd, *J* = 8.8, 1.8 Hz, 1H), 3.66-3.49 (m, 2H), 3.17-2.99 (m, 4H), 1.99 (s, 1H), 1.93 (d, *J* = 6.5 Hz, 1H), 1.76 (s, 1H), 1.61 (d, *J* = 7.5 Hz, 2H), 1.33 (s, 9H), 1.27-1.17 (m, 4H); LC-MS (ESI, m/z) = 488.3 (M+H⁺).

### Example A-12: Synthesis of 4-(tert-butyl)-N-(4-carbamoyl-3-((1-(ethylsulfonyl)piperidin-3-yl)amino)phenyl)benzamide

The compound obtained in Example A-11 (40 mg, 0.08 mmol) was reacted in the same manner as in Example A-3 to obtain the target compound (20 mg).

¹H NMR (300 MHz, DMSO-d₆) δ 10.15 (s, 1H), 8.57 (d, *J* = 7.8 Hz, 1H), 7.87 (d, *J* = 8.4 Hz, 2H), 7.74 (s, 1H), 7.62 (d, *J* = 8.7 Hz, 1H), 7.56 (d, *J* = 8.2 Hz, 2H), 7.30 (d, *J* = 2.0 Hz, 1H), 7.11-6.97 (m, 2H), 3.54 (t, *J* = 10.2 Hz, 2H), 3.15-2.98 (m, 3H), 1.93 (d, *J* = 7.7 Hz, 1H), 1.77 (s, 1H), 1.56 (q, *J* = 17.2, 13.0 Hz, 2H), 1.33 (s, 9H), 1.21 (t, *J* = 7.3 Hz, 4H); LC-MS (ESI, m/z) = 487.4 (M+H⁺).

### Example A-13: Synthesis of methyl 2-((1-(benzylsulfonyl)piperidin-3-yl)amino)-4-(4-(tert-butyl)benzamido)benzoate

The compound prepared in Preparation Example 4 (500 mg, 0.94 mmol) and phenylmethanesulfonyl chloride (358 mg, 1.88 mmol) were reacted in the same manner as in Example A-1 to obtain the target compound (303 mg).

¹H NMR (300 MHz, DMSO-d₆) δ 7.88-7.77 (m, 4H), 7.55 (d, *J* = 8.2 Hz, 2H), 7.47-7.33 (m, 6H), 7.10 (d, *J* = 8.8Hz, 1H), 4.43 (s, 2H), 3.78 (s, 3H), 3.64-3.52 (m, 2H), 3.25 (s, 1H), 3.02-2.91 (m, 2H), 1.92 (s, 1H), 1.72 (s, 1H), 1.56 (d, *J* = 8.7 Hz, 2H), 1.32 (s, 9H); LC-MS (ESI, m/z) = 564.3 (M+H⁺).

### Example A-14: Synthesis of 2-((1-(benzylsulfonyl)piperidin-3-yl)amino)-4-(4-(tert-butyl)benzamido)benzoic acid

The compound obtained in Example A-13 (155 mg, 0.275 mmol) was reacted in the same manner as in Example A-2 to obtain the target compound (145 mg).

¹H NMR (500 MHz, DMSO-d₆) δ 12.46 (s, 1H), 10.26 (s, 1H), 8.04 (d, *J* = 7.8 Hz, 1H), 7.87 (d, *J* = 7.8 Hz, 2H), 7.77 (d, *J* = 8.7 Hz, 1H), 7.55 (d, *J* = 7.8 Hz, 2H), 7.45-7.32 (m, 6H), 7.07 (d, *J* = 8.7 Hz, 1H), 4.43 (s, 2H), 3.58 (t, *J* = 13.0 Hz, 2H), 3.28 (s, 1H), 2.99 (t, *J* = 9.7 Hz, 1H), 2.91 (d, *J* = 10.5 Hz, 1H), 1.93 (d, *J* = 13.0 Hz, 1H), 1.72 (s, 1H), 1.53 (t, *J* = 9.7 Hz, 2H), 1.32 (s, 9H); LC-MS (ESI, m/z) = 550.3 (M+H⁺).

### Example A-15: Synthesis of N-(3-((1-(benzylsulfonyl)piperidin-3-yl)amino)-4-carbamoylphenyl)-4-(tert-butyl)benzamide

The compound obtained in Example A-14 (55 mg, 0.1 mmol) was reacted in the same manner as in Example A-3 to obtain the target compound (27 mg).

¹H NMR (500 MHz, DMSO-d₆) δ 10.15 (s, 1H), 8.54 (d, *J* = 8.0 Hz, 1H), 7.87 (d, *J* = 9.0 Hz, 2H), 7.74 (s, 1H), 7.62 (d, *J* = 8.7 Hz, 1H), 7.55 (d, *J* = 8.0 Hz, 2H), 7.46-7.29 (m, 7H), 7.04 (d, *J* = 8.7 Hz, 1H), 4.42 (s, 2H), 3.62 (d, *J* = 12.0 Hz, 1H), 3.47 (s, 1H), 2.93 (d, *J* = 10.5 Hz, 1H), 2.79 (t, *J* = 10.5 Hz, 1H), 1.94 (s, 1H), 1.74 (s, 1H), 1.52 (d, *J* = 12.0 Hz, 1H), 1.43 (d, *J* = 12.0 Hz, 1H), 1.32 (s, 10H); LC-MS (ESI, m/z) = 549.3 (M+H⁺).

### Example A-16: Synthesis of methyl 4-(4-(tert-butyl)benzamido)-2-((1-(isopropylsulfonyl)piperidin-3-yl)amino)benzoate

The compound prepared in Preparation Example 4 (500 mg, 0.94 mmol) and isopropylsulfonyl chloride (268 mg, 1.88 mmol) were reacted in the same manner as in Example A-1 to obtain the target compound (325 mg).

¹H NMR (300 MHz, DMSO-d₆) δ 10.25 (s, 1H), 7.86 (t, *J* = 8.1 Hz, 3H), 7.79 (d, *J* = 8.1 Hz, 1H), 7.55 (d, *J* = 6.7 Hz, 2H), 7.43 (s, 1H), 7.09 (d, *J* = 8.8 Hz, 1H), 3.78 (s, 3H), 3.59 (d, *J* = 10.7 Hz, 2H), 3.38-3.29 (m, 2H), 3.24-3.06 (m, 2H), 2.01-1.90 (m, 1H), 1.75 (s, 1H), 1.62 (d, *J* = 9.1 Hz, 2H), 1.32 (s, 9H), 1.22 (d, *J* = 6.9 Hz, 6H); LC-MS (ESI, m/z) = 516.4 (M+H⁺).

### Example A-17: Synthesis of 4-(4-(tert-butyl)benzamido)-2-((1-(isopropylsulfonyl)piperidin-3-yl)amino)benzoic acid

The compound obtained in Example A-16 (171 mg, 0.332 mmol) was reacted in the same manner as in Example A-2 to obtain the target compound (164 mg).

¹H NMR (500 MHz, DMSO-d₆) δ 12.44 (s, 1H), 10.23 (s, 1H), 8.06 (d, *J* = 7.7 Hz, 1H), 7.87 (d, *J* = 8.5 Hz, 2H), 7.76 (d, *J* = 8.5 Hz, 1H), 7.55 (d, *J* = 8.5 Hz, 2H), 7.40 (s, 1H), 7.04 (d, *J* = 12.0 Hz, 1H), 3.59 (d, J = 12.0 Hz, 2H), 3.35 (s, 2H), 3.17 (t, *J* = 10.0 Hz, 1H), 3.06 (t, *J* = 10.0 Hz, 1H), 1.95-1.90 (m, 1H), 1.74 (s, 1H), 1.59 (s, 2H), 1.32 (s, 9H), 1.21 (d, *J* = 6.9 Hz, 6H); LC-MS (ESI, m/z) = 502.4 (M+H⁺).

### Example A-18: Synthesis of 4-(tert-butyl)-N-(4-carbamoyl-3-((1-(isopropylsulfonyl)piperidin-3-yl)amino)phenyl)benzamide

The compound obtained in Example A-17 (50 mg, 0.1 mmol) was reacted in the same manner as in Example A-3 to obtain the target compound (33 mg).

¹H NMR (500 MHz, CDCl₃) δ 8.23 (s, 1H), 7.82 (d, *J* = 8.0 Hz, 2H), 7.46 (dd, *J* = 16.8, 8.1 Hz, 3H), 7.26 (s, 2H), 7.04 (s, 1H), 5.83 (s, 2H), 3.93 (d, *J* = 13.0 Hz, 1H), 3.65 (d, *J* = 13.0 Hz, 1H), 3.55 (s, 1H), 3.20 (s, 1H), 3.00 (t, *J* = 11.3 Hz, 1H), 2.78 (t, *J* = 11.3 Hz, 1H), 2.10 (d, *J* = 12.6 Hz, 1H), 1.84 (d, *J* = 13.9 Hz, 1H), 1.73 (d, *J* = 12.6 Hz, 1H), 1.57 (t, *J* = 11.7 Hz, 1H), 1.34 (s, 15H); LC-MS (ESI, m/z) = 501.4 (M+H⁺).

### Example A-19: Synthesis of methyl 4-(4-(tert-butyl)benzamido)-2-((1-((2-nitrophenyl)sulfonyl)piperidin-3-yl)amino)benzoate

The compound prepared in Preparation Example 4 (100 mg, 0.188 mmol) and 2-nitrobenzenesulfonyl chloride (44 mg, 0.2 mmol) were reacted in the same manner as in Example A-1 to obtain the target compound (98 mg).

¹H NMR (500 MHz, CDCl₃) δ 8.22 (s, 1H), 7.95 (d, *J* = 7.4 Hz, 1H), 7.86-7.83 (m, 2H), 7.64-7.57 (m, 3H), 7.47 (d, *J* = 7.1 Hz, 2H), 7.13 (d, *J* = 8.8 Hz, 1H), 7.08 (s, 1H), 3.93 (d, *J* = 12.8 Hz, 1H), 3.80 (s, 3H), 3.68-3.55 (m, 2H), 3.10 (t, *J* = 11.0 Hz, 1H), 2.83 (t, *J* = 11.0 Hz, 1H), 2.03 (s, 2H), 1.94-1.86 (m, 1H), 1.73-1.63 (m, 1H), 1.59-1.51 (m, 1H), 1.33 (d, *J* = 3.7 Hz, 9H); LC-MS (ESI, m/z) = 595.3 (M+H⁺).

### Example A-20: Synthesis of 4-(4-(tert-butyl)benzamido)-2-((1-((2-nitrophenyl)sulfonyl)piperidin-3-yl)amino)benzoic acid

The compound obtained in Example A-19 (53 mg, 0.09 mmol) was reacted in the same manner as in Example A-2 to obtain the target compound (36 mg).

¹H NMR (500 MHz, DMSO-d₆) δ 12.42 (s, 1H), 10.22 (s, 1H), 8.08 (d, *J* = 8.2 Hz, 1H), 7.99 (d, *J* = 8.0 Hz, 1H), 7.96 (d, *J* = 8.0 Hz, 1H), 7.88 (d, *J* = 8.2 Hz, 3H), 7.82-7.74 (m, 2H), 7.56 (d, *J* = 8.9 Hz, 2H), 7.36 (s, 1H), 7.09 (d, *J* = 8.9 Hz, 1H), 3.64 (s, 1H), 3.57 (d, *J* = 12.0 Hz, 1H), 3.30 (s, 1H), 3.18-3.06 (m, 2H), 1.91 (s, 1H), 1.81 (s, 1H), 1.64-1.58 (m, 2H), 1.32 (s, 10H); LC-MS (ESI, m/z) = 581.3 (M+H⁺).

### Example A-21: Synthesis of 4-(tert-butyl)-N-(4-carbamoyl-3-((1-((2-nitrophenyl)sulfonyl)piperidin-3-yl)amino)phenyl)benzamide

The compound obtained in Example A-20 (20 mg, 0.03 mmol) was reacted in the same manner as in Example A-3 to obtain the target compound (19 mg).

¹H NMR (500 MHz, DMSO-d₆) δ 10.13 (s, 1H), 8.58 (s, 1H), 8.01 (d, *J* = 8.0 Hz, 1H), 7.96 (d, *J* = 8.0 Hz, 1H), 7.87 (s, 3H), 7.82 (d, *J* = 8.0 Hz, 1H), 7.72 (s, 1H), 7.61 (d, *J* = 8.7 Hz, 1H), 7.56 (d, *J* = 6.6 Hz, 2H), 7.26 (s, 1H), 7.06 (d, *J* = 8.7 Hz, 2H), 3.60 (d, *J* = 12.0 Hz, 1H), 3.55 (s, 1H), 3.35 (s, 1H), 3.04 (t, *J* = 10.4 Hz, 1H), 2.95 (d, *J* = 10.4 Hz, 1H), 1.91 (s, 1H), 1.81 (s, 1H), 1.61 (s, 1H), 1.54-1.47 (m, 1H), 1.32 (s, 10H); LC-MS (ESI, m/z) = 580.3 (M+H⁺).

### Example A-22: Synthesis of methyl 4-(4-(tert-butyl)benzamido)-2-((1-(naphthalen-2-ylsulfonyl)piperidin-3-yl)amino)benzoate

The compound prepared in Preparation Example 4 (300 mg, 0.59 mmol) and 2-naphthalenesulfonyl chloride (160 mg, 0.71 mmol) were reacted in the same manner as in Example A-1 to obtain the target compound (105 mg).

¹H NMR (300 MHz, DMSO-d₆) δ 10.29 (s, 1H), 8.47 (d, *J* = 1.8 Hz, 1H), 8.19 (dd, *J* = 16.6, 8.3 Hz, 2H), 8.08 (d, *J* = 7.6 Hz, 1H), 7.94-7.86 (m, 3H), 7.83-7.76 (m, 2H), 7.75-7.65 (m, 2H), 7.60-7.54 (m, 2H), 7.41 (d, *J* = 1.9 Hz, 1H), 7.15 (dd, *J* = 8.8, 1.8 Hz, 1H), 3.78 (s, 3H), 3.69 (s, 1H), 3.07 (s, 1H), 3.03-2.94 (m, 1H), 2.89 (dd, *J* = 11.5, 6.8 Hz, 1H), 1.75 (d, *J* = 9.5 Hz, 2H), 1.56 (dd, *J* = 18.4, 10.3 Hz, 2H), 1.33 (s, 9H), 1.30-1.22 (m, 1H); LC-MS (ESI, m/z) = 600.2 (M+H⁺).

### Example A-23: Synthesis of 4-(4-(tert-butyl)benzamido)-2-((1-(naphthalen-2-ylsulfonyl)piperidin-3-yl)amino)benzoic acid

The compound obtained in Example A-22 (90 mg, 0.15 mmol) was reacted in the same manner as in Example A-2 to obtain the target compound (85 mg).

¹H NMR (300 MHz, DMSO-d₆) δ 12.42 (s, 1H), 10.26 (s, 1H), 8.52-8.44 (m, 1H), 8.26-8.19 (m, 1H), 8.16 (d, *J* = 8.7 Hz, 1H), 8.07 (dd, *J* = 9.6, 3.9 Hz, 2H), 7.93-7.87 (m, 2H), 7.82-7.64 (m, 4H), 7.60-7.54 (m, 2H), 7.39 (d, *J* = 1.9 Hz, 1H), 7.12 (dd, *J* = 8.8, 1.8 Hz, 1H), 3.16 (s, 1H), 2.81 (dt, *J* = 34.1, 8.8 Hz, 2H), 1.77 (d, *J* = 8.6 Hz, 2H), 1.62 (d, *J* = 8.3 Hz, 1H), 1.49 (p, *J* = 7.8, 7.2 Hz, 2H), 1.34 (s, 9H), 1.30-1.20 (m, 2H); LC-MS (ESI, m/z) = 586.2 (M+H⁺).

### Example A-24: Synthesis of 4-(tert-butyl)-N-(4-carbamoyl-3-((1-(naphthalen-2-ylsulfonyl)piperidin-3-yl)amino)phenyl)benzamide

The compound obtained in Example A-23 (70 mg, 0.12 mmol) was reacted in the same manner as in Example A-3 to obtain the target compound (38 mg).

¹H NMR (300 MHz, DMSO-d₆) δ 10.17 (s, 1H), 8.55 (d, *J* = 8.1 Hz, 1H), 8.51-8.43 (m, 1H), 8.23 (d, *J* = 8.1 Hz, 1H), 8.16 (d, *J* = 8.7 Hz, 1H), 8.07 (d, *J* = 7.9 Hz, 1H), 7.94-7.86 (m, 2H), 7.81-7.53 (m, 7H), 7.29 (d, *J* = 2.0 Hz, 1H), 7.09 (dd, *J* = 8.7, 1.8 Hz, 1H), 3.60-3.44 (m, 2H), 3.24 (d, *J* = 11.5 Hz, 1H), 2.82-2.70 (m, 1H), 2.65-2.55 (m, 1H), 2.51 (p, *J* = 1.8 Hz, 5H), 1.78 (d, *J* = 10.7 Hz, 2H), 1.67-1.50 (m, 1H), 1.42 (d, *J* = 8.7 Hz, 1H), 1.34 (s, 9H); LC-MS (ESI, m/z) = 585.2 (M+H⁺).

### Example A-25: Synthesis of methyl 4-(4-(tert-butyl)benzamido)-2-((1-((1-methyl-1H-imidazol-4-yl)sulfonyl)piperidin-3-yl)amino)benzoate

The compound prepared in Preparation Example 4 (300 mg, 0.59 mmol) and methyl-1H-imidazole-4-sulfonyl chloride (128 mg, 0.71 mmol) were reacted in the same manner as in Example A-1 to obtain the target compound (268 mg).

¹H NMR (300 MHz, DMSO-d₆) δ10.27 (s, 1H), 7.56 (d, *J* = 8.3 Hz, 2H), 7.40 (d, *J* = 2.0 Hz, 1H), 7.14 (dd, *J* = 8.8, 1.8 Hz, 1H), 3.79 (s, 3H), 3.71 (s, 4H), 3.11 (d, *J* = 8.9 Hz, 1H), 2.95 (t, *J* = 8.9 Hz, 1H), 2.84 (dd, *J* = 11.6, 7.0 Hz, 1H), 1.79 (s, 2H), 1.68-1.43 (m, 2H), 1.33 (s, 9H); LC-MS (ESI, m/z) = 554.3 (M+H⁺).

### Example A-26: Synthesis of 4-(4-(tert-butyl)benzamido)-2-((1-((1-methyl-1H-imidazol-4-yl)sulfonyl)piperidin-3-yl)amino)benzoic acid

The compound obtained in Example A-25 (230 mg, 2.08 mmol) was reacted in the same manner as in Example A-2 to obtain the target compound (130 mg).

¹H NMR (300 MHz, DMSO-d₆) δ 12.35 (s, 1H), 10.25 (s, 1H), 8.03 (d, *J* = 8.2 Hz, 1H), 7.89 (d, *J* = 8.3 Hz, 2H), 7.83 (d, *J* = 3.8 Hz, 2H), 7.77 (d, *J* = 8.8 Hz, 1H), 7.56 (d, *J* = 8.2 Hz, 2H), 7.37 (d, *J* = 1.8 Hz, 1H), 7.11 (d, *J* = 8.7 Hz, 1H), 3.71 (s, 3H), 3.63 (s, 1H), 3.25-3.13 (m, 1H), 2.93-2.80 (m, 1H), 2.70 (dd, *J* = 11.4, 7.7 Hz, 1H), 1.91 (s, 1H), 1.89-1.71 (m, 2H), 1.63 (d, *J* = 9.4 Hz, 1H), 1.45 (d, *J* = 8.8 Hz, 1H), 1.33 (s, 9H), 1.24 (s, 1H), 0.85 (d, *J* = 7.1 Hz, 1H); LC-MS (ESI, m/z) = 540.3 (M+H⁺).

### Example A-27: Synthesis of 4-(tert-butyl)-N-(4-carbamoyl-3-((1-((1-methyl-1H-imidazol-4-yl)sulfonyl)piperidin-3-yl)amino)phenyl)benzamide

The compound obtained in Example A-26 (100 mg, 0.19 mmol) was reacted in the same manner as in Example A-3 to obtain the target compound (83 mg).

¹H NMR (300 MHz, DMSO-d₆) δ10.16 (s, 1H), 8.50 (d, *J* = 8.1 Hz, 1H), 7.89 (d, *J* = 8.2 Hz, 2H), 7.82 (d, *J* = 4.5 Hz, 2H), 7.59 (dd, *J* = 16.4, 8.5 Hz, 3H), 7.27 (d, *J* = 1.9 Hz, 1H), 7.13-7.03 (m, 1H), 4.25 (d, *J* = 5.6 Hz, 1H), 3.70 (s, 3H), 3.52 (d, *J* = 5.5 Hz, 3H), 3.27 (d, *J* = 11.1 Hz, 1H), 2.75 (t, *J* = 10.2 Hz, 1H), 1.76 (s, 2H), 1.71-1.50 (m, 1H), 1.33 (s, 10H), 1.24 (s, 1H), 0.96-0.80 (m, 2H); LC-MS (ESI, m/z) = 539.4 (M+H⁺).

### Example A-28: Synthesis of methyl 2-((1-((4-acetylphenyl)sulfonyl)piperidin-3-yl)amino)-4-(4-(tert-butyl)benzamido)benzoate

The compound prepared in Preparation Example 4 (300 mg, 0.59 mmol) and 4-acetylbenzenesulfonyl chloride (155 mg, 0.71 mmol) and trimethylamine (170 µL, 1.18 mmol) were reacted in the same manner as in Example A-1 to obtain the target compound (313 mg).

¹H NMR (300 MHz, DMSO-d₆) δ10.29 (s, 1H), 8.16 (d, *J* = 8.2 Hz, 2H), 7.90 (dd, *J* = 8.4, 2.3 Hz, 5H), 7.79 (d, *J* = 8.8 Hz, 1H), 7.57 (d, *J* = 8.2 Hz, 2H), 7.41 (d, *J* = 1.9 Hz, 1H), 7.14 (dd, *J* = 8.8, 1.7 Hz, 1H), 3.79 (s, 3H), 3.68 (s, 1H), 3.05 (s, 1H), 2.97 (d, *J* = 7.5 Hz, 1H), 2.87 (dd, *J* = 11.4, 6.7 Hz, 1H), 2.65 (s, 3H), 1.78 (d, *J* = 9.5 Hz, 2H), 1.69-1.47 (m, 2H), 1.33 (s, 9H); LC-MS (ESI, m/z) = 592.2 (M+H⁺).

### Example A-29: Synthesis of 2-((1-((4-acetylphenyl)sulfonyl)piperidin-3-yl)amino)-4-(4-(tert-butyl)benzamido)benzoic acid

The compound obtained in Example A-28 (200 mg, 0.34 mmol) was reacted in the same manner as in Example A-2 to obtain the target compound (190 mg).

¹H NMR (300 MHz, DMSO-d₆) δ 12.44 (s, 1H), 10.25 (s, 1H), 8.16 (d, *J* = 8.2 Hz, 2H), 8.05 (d, *J* = 8.1 Hz, 1H), 7.90 (dd, *J* = 8.4, 2.9 Hz, 4H), 7.77 (d, *J* = 8.8 Hz, 1H), 7.57 (d, *J* = 8.2 Hz, 2H), 7.38 (d, *J* = 1.9 Hz, 1H), 7.10 (dd, *J* = 8.7, 1.8 Hz, 1H), 2.86 (t, *J* = 9.1 Hz, 1H), 2.76 (d, *J* = 10.9 Hz, 1H), 2.65 (s, 3H), 1.78 (s, 2H), 1.54 (dd, *J* = 26.1, 16.0 Hz, 2H), 1.33 (s, 9H), 1.24 (s, 1H); LC-MS (ESI, m/z) = 578.3 (M+H⁺).

### Example A-30: Synthesis of N-(3-((1-((4-acetylphenyl)sulfonyl)piperidin-3-yl)amino)-4-carbamoylphenyl)-4-(tert-butyl)benzamide

The compound obtained in Example A-29 (100 mg, 0.17 mmol) was reacted in the same manner as in Example A-3 to obtain the target compound (95 mg).

¹H NMR (300 MHz, DMSO-d₆) δ10.17 (s, 1H), 8.55 (d, *J* = 8.2 Hz, 1H), 8.20-8.12 (m, 2H), 8.09 (s, 2H), 7.90 (dd, *J* = 8.4, 3.5 Hz, 4H), 7.70 (d, *J* = 8.5 Hz, 2H), 7.59 (dd, *J* = 16.3, 8.6 Hz, 3H), 7.29 (d, *J* = 2.0 Hz, 1H), 7.08 (dd, *J* = 8.7, 1.8 Hz, 1H), 4.24 (dd, *J* = 5.6, 1.5 Hz, 2H), 4.03 (q, *J* = 7.1 Hz, 1H), 3.51-3.41 (m, 1H), 3.21 (d, *J* = 11.3 Hz, 1H), 2.64 (s, 3H), 2.62-2.53 (m, 1H), 1.99 (s, 1H), 1.79 (s, 2H), 1.60 (d, *J* = 7.4 Hz, 1H), 1.42 (t, *J* = 7.2 Hz, 1H), 1.33 (s, 9H), 1.27-1.09 (m, 2H), 0.97-0.84 (m, 4H), 0.85 (s, 4H); LC-MS (ESI, m/z) = 577.3 (M+H⁺).

### Example A-31: Synthesis of methyl 2-((1-([1,1'-biphenyl]-4-ylsulfonyl)piperidin-3-yl)amino)-4-(4-(tert-butyl)benzamido)benzoate

The compound prepared in Preparation Example 4 (300 mg, 0.59 mmol) and [1,1'-biphenyl]-4-sulfonyl chloride (179 mg, 0.71 mmol) were reacted in the same manner as in Example A-1 to obtain the target compound (250 mg).

¹H NMR (300 MHz, DMSO-d₆) δ 10.30 (d, *J* = 2.0 Hz, 1H), 7.91 (m, *J* = 8.5, 6.3, 2.1 Hz, 5H), 7.86-7.81 (m, 2H), 7.81-7.70 (m, 3H), 7. 64-7.37 (m, 6H), 7.16 (m, *J* = 8.8, 2.0 Hz, 1H), 3.79 (d, *J* = 2.0 Hz, 3H), 3.71 (s, 1H), 3.05 (s, 1H), 2.98 (s, 1H), 2.86 (d, *J* = 10.4 Hz, 1H), 1.78 (d, *J* = 10.4 Hz, 2H), 1.59 (dd, *J* = 16.7, 8.9 Hz, 2H), 1.33 (d, *J* = 2.0 Hz, 9H); LC-MS (ESI, m/z) = 626.4 (M+H⁺).

### Example A-32: Synthesis of 2-((1-([1,1'-biphenyl]-4-ylsulfonyl)piperidin-3-yl)amino)-4-(4-(tert-butyl)benzamido)benzoic acid

The compound obtained in Example A-31 (250 mg, 0.40 mmol) was reacted in the same manner as in Example A-2 to obtain the target compound (237 mg).

¹H NMR (300 MHz, DMSO-d₆) δ 10.12 (s, 1H), 8.97 (s, 1H), 8.01-7.86 (m, 4H), 7.82-7.70 (m, 3H), 7.50 (dt, *J* = 21.0, 7.7 Hz, 4H), 7.22 (s, 1H), 7.02 (d, *J* = 8.6 Hz, 1H), 2.41 (s, 1H), 1.82 (s, 2H), 1.61 (s, 1H), 1.33 (s, 9H); LC-MS (ESI, m/z) = 612.4 (M+H⁺).

### Example A-33: Synthesis of N-(3-((1-([1,1'-biphenyl]-4-ylsulfonyl)piperidin-3-yl)amino)-4-carbamoylphenyl)-4-(tert-butyl)benzamide

The compound obtained in Example A-32 (100 mg, 0.16 mmol) was reacted in the same manner as in Example A-3 to obtain the target compound (55 mg).

¹H NMR (300 MHz, DMSO-d₆) δ 10.18 (s, 1H), 8.56 (d, *J* = 8.2 Hz, 1H), 7.99-7.86 (m, 5H), 7.83 (d, *J* = 8.5 Hz, 2H), 7.79-7.70 (m, 3H), 7.63 (d, *J* = 8.7 Hz, 1H), 7.60-7.40 (m, 5H), 7.30 (d, *J* = 1.9 Hz, 1H), 7.10 (dd, *J* = 8.6, 1.8 Hz, 2H), 3.58 (s, 1H), 3.47 (d, *J* = 11.2 Hz, 1H), 3.23 (d, *J* = 11.1 Hz, 1H), 2.81-2.68 (m, 1H), 2.63-2.54 (m, 1H), 1.80 (s, 2H), 1.70-1.51 (m, 1H), 1.42 (d, *J* = 8.9 Hz, 1H), 1.33 (s, 9H); LC-MS (ESI, m/z) = 611.4 (M+H⁺).

### Example B-1: Synthesis of methyl 4-(4-(tert-butyl)benzamido)-2-((1-(ethylcarbamoyl)piperidin-3-yl)amino)benzoate

The compound prepared in Preparation Example 4 (300 mg, 0.59 mmol) was dissolved in DCM (6 mL), and then DIPEA (179 mg, 1.77 mmol) and ethyl isocyanate (84 mg, 1.18 mmol) were sequentially added, followed by stirring at ambient temperature for 1 hour. The reaction product was confirmed by TLC, then diluted with EtOAc and washed with an aqueous NaHCO₃ solution. The organic layer was dried over anhydrous Na₂SO₄, concentrated under reduced pressure, and separated by column chromatography to obtain the target compound (282 mg).

¹H NMR (300 MHz, DMSO-d₆) δ 10.25 (s, 1H), 7.94-7.83 (m, 2H), 7.78 (dd, *J* = 8.4, 2.9 Hz, 2H), 7.64-7.49 (m, 2H), 7.39 (d, *J* = 2.0 Hz, 1H), 7.11 (dd, *J* = 8.9, 1.8 Hz, 1H), 6.47 (t, *J* = 5.4 Hz, 1H), 4.11 (q, *J =* 5.2 Hz, 1H), 3.64-3.50 (m, 1H), 3.44 (d, *J* = 7.9 Hz, 1H), 3.18 (d, *J* = 5.0 Hz, 3H), 3.03 (m, *J* = 15.3, 8.2, 7.7, 4.0 Hz, 4H), 1.99 (s, 1H), 1.81-1.59 (m, 1H), 1.51 (p, J = 11.2, 10.7 Hz, 2H), 1.33 (s, 9H), 1.25 (m, *J* = 7.4, 5.3 Hz, 2H), 1.00 (t, *J* = 7.2 Hz, 3H); LC-MS (ESI, m/z)=481.4 (M+H⁺).

### Example B-2: Synthesis of 4-(4-(tert-butyl)benzamido)-2-((1-(ethylcarbamoyl)piperidin-3-yl)amino)benzoic acid

The compound obtained in Example B-1 (280 mg, 0.58 mmol) was dissolved in 5 mL of a THF/MeOH/H₂O mixture (mixing ratio = 3 : 1 : 1), and LiOH•H₂O (74 mg, 1.76 mmol) was added and stirred at ambient temperature for 24 hours. The reaction product was confirmed by TLC, and 1M HCl aqueous solution was added dropwise so that the pH of the reaction solution was 2 to 3. The resulting white solid was filtered to obtain the target compound (161 mg).

¹H NMR (300 MHz, DMSO-d₆) δ 12.37 (s, 1H), 10.21 (s, 1H), 7.94 (d, *J* = 7.9 Hz, 1H), 7.91-7.84 (m, 2H), 7.76 (d, *J* = 8.7 Hz, 1H), 7.60-7.51 (m, 2H), 7.35 (d, *J* = 1.9 Hz, 1H), 7.06 (dd, *J* = 8.7, 1.8 Hz, 1H), 3.85 (d, *J* = 13.3 Hz, 1H), 3.62 (d, *J* = 13.1 Hz, 1H), 3.15-2.84 (m, 4H), 2.00 (d, *J* = 4.3 Hz, 1H), 1.66 (s, 1H), 1.48 (d, *J* = 9.5 Hz, 2H), 1.33 (s, 9H), 0.99 (t, *J* = 7.1 Hz, 3H); LC-MS (ESI, m/z) = 467.4 (M+H⁺).

### Example B-3: Synthesis of 3-((5-(4-(tert-butyl)benzamido)-2-carbamoylphenyl)amino)-N-ethylpiperidine-1-carboxamide

The compound of Example B-2 (60 mg, 0.13 mmol) and NH₄Cl (20 mg, 0.39 mmol) were dissolved in DMF (4 mL), and DIPEA (50 mg, 0.39 mmol) and HBTU (76 mg, 0.20 mmol) were added and stirred at ambient temperature for 5 hours. The reaction product was confirmed by TLC, then diluted with EtOAc and washed with an aqueous NaHCO₃ solution. The organic layer was dried over anhydrous Na₂SO₄, concentrated under reduced pressure, and separated by column chromatography to obtain the target compound (47 mg).

¹H NMR (300 MHz, DMSO-d₆) δ 10.13 (s, 1H), 8.62 (d, *J* = 8.2 Hz, 1H), 7.93-7.82 (m, 2H), 7.62 (d, *J* = 8.7 Hz, 1H), 7.56 (d, *J =* 8.4 Hz, 2H), 7.32 (d, *J =* 2.0 Hz, 1H), 7.01 (dd, *J =* 8.7, 1.8 Hz, 2H), 3.69-3.53 (m, 2H), 3.48-3.36 (m, 1H), 3.22-3.06 (m, 3H), 2.99 (dd, *J* = 11.7, 7.1 Hz, 1H), 2.76 (s, 6H), 1.85 (dd, *J* = 34.5, 23.1 Hz, 2H), 1.56 (t, *J* = 8.1 Hz, 2H), 1.33 (s, 9H); LC-MS (ESI, m/z) = 466.4 (M+H⁺).

### Example B-4: Synthesis of methyl 4-(4-(tert-butyl)benzamido)-2-((1-(butylcarbamoyl)piperidin-3-yl)amino)benzoate

The compound prepared in Preparation Example 4 (400 mg, 0.79 mmol) and butyl isocyanate (93 mg, 0.95 mmol) were reacted in the same manner as in Example B-1 to obtain the target compound (322 mg).

¹H NMR (300 MHz, DMSO-d₆) δ 10.24 (s, 1H), 7.88 (d, *J* = 8.4 Hz, 2H), 7.78 (d, *J* = 9.0 Hz, 2H), 7.56 (d, *J* = 8.4 Hz, 2H), 7.39 (s, 1H), 7.10 (d, *J* = 8.8 Hz, 1H), 6.43 (t, *J* = 5.5 Hz, 1H), 3.56 (d, *J* = 13.4 Hz, 1H), 3.44 (s, 1H), 3.10-2.91 (m, 4H), 2.06-1.93 (m, 1H), 1.66 (d, *J* = 6.4 Hz, 1H), 1.56-1.43 (m, 2H), 1.38 (d, *J* = 7.9 Hz, 1H), 1.33 (s, 9H), 1.29-1.14 (m, 3H), 0.85 (m, *J* = 11.3, 7.1 Hz, 4H); LC-MS (ESI, m/z) = 509.4 (M+H⁺).

### Example B-5: Synthesis of 4-(4-(tert-butyl)benzamido)-2-((1-(butylcarbamoyl)piperidin-3-yl)amino)benzoic acid

The compound obtained in Example B-4 (300 mg, 0.59 mmol) was reacted in the same manner as in Example B-2 to obtain the target compound (288 mg).

¹H NMR (300 MHz, DMSO-d₆) δ 12.35 (s, 1H), 10.21 (s, 1H), 7.94 (d, *J* = 7.6 Hz, 1H), 7.88 (d, *J* = 8.2 Hz, 2H), 7.76 (d, *J* = 8.7 Hz, 1H), 7.59-7.51 (m, 2H), 7.35 (d, *J =* 1.9 Hz, 1H), 7.06 (dd, *J* = 8.8, 1.8 Hz, 1H), 6.42 (t, *J =* 5.5 Hz, 1H), 3.87 (dd, *J* = 12.2, 3.0 Hz, 1H), 3.65 (d, *J =* 12.9 Hz, 1H), 3.07-2.93 (m, 3H), 2.88 (m, J = 12.8, 8.5 Hz, 1H), 2.01 (d, *J* = 8.8 Hz, 1H), 1.66 (s, 1H), 1.48 (d, *J* = 9.4 Hz, 2H), 1.37 (d, *J* = 7.6 Hz, 1H), 1.33 (d, *J* = 1.0 Hz, 9H), 1.23 (td, *J* = 9.3, 4.8 Hz, 3H), 0.87-0.77 (m, 3H); LC-MS (ESI, m/z) = 495.4 (M+H⁺).

### Example B-6: Synthesis of N-butyl-3-((5-(4-(tert-butyl)benzamido)-2-carbamoylphenyl)amino)piperidine-1-carboxamide

The compound obtained in Example B-5 (100 mg, 0.20 mmol) was reacted in the same manner as in Example B-3 to obtain the target compound (36 mg).

¹H NMR (300 MHz, DMSO-d₆) δ 10.11 (s, 1H), 8.44 (d, J = 7.8 Hz, 1H), 7.88 (d, *J* = 8.3 Hz, 2H), 7.71 (s, 1H), 7.61 (d, *J* = 8.6 Hz, 1H), 7.55 (d, *J* = 8.4 Hz, 2H), 7.25 (d, *J* = 2.1 Hz, 1H), 7.02 (dd, *J* = 8.7, 1.9 Hz, 2H), 6.40 (t, *J* = 5.5 Hz, 1H), 3.97-3.83 (m, 1H), 3.72 (d, *J* = 13.1 Hz, 1H), 3.00 (q, *J* = 6.5 Hz, 2H), 2.87 (d, *J* = 11.0 Hz, 1H), 2.73 (dd, *J* = 12.4, 8.6 Hz, 1H), 2.04 (s, 1H), 1.99 (s, 2H), 1.65 (s, 1H), 1.47-1.35 (m, 4H), 1.33 (s, 9H), 1.21 (m, *J =* 16.8, 7.1 Hz, 4H), 0.82 (t, *J* = 7.2 Hz, 3H); LC-MS (ESI, m/z) = 494.4 (M+H⁺).

### Example B-7: Synthesis of methyl 4-(4-(tert-butyl)benzamido)-2-((1-(cyclohexylcarbamoyl)piperidin-3-yl)amino)benzoate

The compound prepared in Preparation Example 4 (300 mg, 0.59 mmol) and cyclohexyl isocyanate (88 mg, 0.71 mmol) were reacted in the same manner as in Example B-1 to obtain the target compound (221 mg).

¹H NMR (300 MHz, DMSO-d₆) δ 10.23 (s, 1H), 7.88 (d, *J* = 8.3 Hz, 2H), 7.77 (t, J = 7.7 Hz, 2H), 7.55 (d, *J* = 8.3 Hz, 2H), 7.37 (d, *J* = 1.9 Hz, 1H), 7.09 (dd, *J* = 8.9, 1.8 Hz, 1H), 6.13 (d, *J* = 7.7 Hz, 1H), 3.85 (s, 1H), 3.81 (d, *J* = 3.6 Hz, 1H), 3.77 (s, 3H), 3.61 (d, *J* = 13.4 Hz, 1H), 3.44 (s, 2H), 3.07-2.87 (m, 2H), 2.00 (s, 1H), 1.80-1.58 (m, 5H), 1.58-1.42 (m, 4H), 1.32 (s, 9H), 1.25-1.06 (m, 5H), 1.01 (dd, *J* = 17.8, 9.0 Hz, 1H); LC-MS (ESI, m/z) = 534.7 (M+H⁺).

### Example B-8: Synthesis of 4-(4-(tert-butyl)benzamido)-2-((1-(cyclohexylcarbamoyl)piperidin-3-yl)amino)benzoic acid

The compound obtained in Example B-7 (200 mg, 0.37 mmol) was reacted in the same manner as in Example B-2 to obtain the target compound (141 mg).

¹H NMR (300 MHz, DMSO-d₆) δ 12.34 (s, 1H), 10.19 (s, 1H), 7.98-7.82 (m, 3H), 7.76 (d, *J* = 8.7 Hz, 1H), 7.55 (d, *J* = 8.4 Hz, 2H), 7.33 (d, J = 2.0 Hz, 1H), 7.05 (dd, *J =* 8.8, 1.8 Hz, 1H), 6.11 (d, *J* = 7.8 Hz, 1H), 3.88 (d, *J* = 12.7 Hz, 1H), 3.67 (d, *J* = 13.0 Hz, 1H), 2.02 (s, 1H), 1.67 (d, *J* = 32.2 Hz, 5H), 1.55-1.41 (m, 3H), 1.33 (s, 9H), 1.26-0.76 (m, 8H); LC-MS (ESI, m/z) = 521.3 (M+H⁺).

### Example B-9: Synthesis of 3-((5-(4-(tert-butyl)benzamido)-2-carbamoylphenyl)amino)-N-cyclohexylpiperidine-1-carboxamide

The compound obtained in Example B-8 (100 mg, 0.19 mmol) was reacted in the same manner as in Example B-3 to obtain the target compound (89 mg).

¹H NMR (300 MHz, DMSO-d₆) δ 10.09 (d, *J* = 5.6 Hz, 1H), 8.41 (d, *J* = 7.0 Hz, 1H), 7.96 (d, *J* = 5.4 Hz, 1H), 7.93-7.80 (m, 2H), 7.71 (s, 1H), 7.66-7.47 (m, 3H), 7.23 (d, *J* = 5.3 Hz, 1H), 7.02 (d, *J* = 7.4 Hz, 2H), 6.10 (t, *J =* 6.9 Hz, 1H), 3.95 (d, *J* = 12.8 Hz, 1H), 3.85-3.64 (m, 1H), 3.01-2.60 (m, 6H), 2.02 (d, *J* = 11.5 Hz, 1H), 1.85-1.39 (m, 9H), 1.36-1.27 (m, 9H), 1.26-0.74 (m, 9H); LC-MS (ESI, m/z) = 520.4 (M+H⁺). .

### Example B-10: Synthesis of methyl 4-(4-(tert-butyl)benzamido)-2-((1-(phenylcarbamoyl)piperidin-3-yl)amino)benzoate

The compound prepared in Preparation Example 4 (300 mg, 0.59 mmol) and phenyl isocyanate (84 mg, 0.71 mmol) were reacted in the same manner as in Example B-1 to obtain the target compound (181 mg).

¹H NMR (400 MHz, DMSO-d₆) δ 10.23 (s, 1H), 8.53 (s, 1H), 7.91-7.80 (m, 3H), 7.77 (d, *J* = 8.8 Hz, 1H), 7.54 (d, *J* = 8.4 Hz, 2H), 7.49-7.42 (m, 2H), 7.41 (d, *J* = 2.0 Hz, 1H), 7.19 (t, *J* = 7.8 Hz, 2H), 7.13 (dd, *J* = 8.9, 1.8 Hz, 1H), 6.91 (t, *J =* 7.4 Hz, 1H), 3.91 (dd, *J =* 13.1, 3.5 Hz, 1H), 3.76 (s, 3H), 3.70 (d, *J* = 13.7 Hz, 1H), 3.62-3.49 (m, 1H), 3.25 (td, *J* = 12.6, 5.8 Hz, 2H), 2.01 (d, *J* = 17.7 Hz, 1H), 1.75 (d, *J =* 6.9 Hz, 1H), 1.68-1.50 (m, 2H), 1.32 (s, 9H); LC-MS (ESI, m/z) = 529.4 (M+H⁺).

### Example B-11: Synthesis of 4-(4-(tert-butyl)benzamido)-2-((1-(phenylcarbamoyl)piperidin-3-yl)amino)benzoic acid

The compound obtained in Example B-10 (181 mg, 0.34 mmol) was reacted in the same manner as in Example B-2 to obtain the target compound (118 mg).

¹H NMR (300 MHz, DMSO-d₆) δ 12.37 (s, 1H), 10.20 (s, 1H), 8.52 (s, 1H), 8.02 (d, *J* = 7.9 Hz, 1H), 7.91-7.81 (m, 2H), 7.76 (d, *J* = 8.8 Hz, 1H), 7.58-7.49 (m, 2H), 7.48-7.40 (m, 2H), 7.37 (d, *J* = 1.9 Hz, 1H), 7.18 (t, *J* = 7.8 Hz, 2H), 7.10 (dd, *J =* 8.8, 1.8 Hz, 1H), 6.91 (t, *J* = 7.2 Hz, 1H), 3.98 (d, *J =* 12.4 Hz, 1H), 3.76 (d, *J =* 13.2 Hz, 1H), 3.17 (td, *J* = 12.9, 5.9 Hz, 2H), 2.02 (d, *J =* 17.2 Hz, 1H), 1.75 (s, 1H), 1.56 (s, 2H), 1.32 (s, 10H); LC-MS (ESI, m/z) = 515.4 (M+H⁺).

### Example B-12: Synthesis of 3-((5-(4-(tert-butyl)benzamido)-2-carbamoylphenyl)amino)-N-phenylpiperidine-1-carboxamide

The compound obtained in Example B-11 (50 mg, 0.09 mmol) was reacted in the same manner as in Example B-3 to obtain the target compound (18 mg).

¹H NMR (400 MHz, DMSO-d₆) δ 10.10 (s, 1H), 8.52 (d, *J* = 8.7 Hz, 2H), 7.96 (s, 1H), 7.85 (d, *J* = 8.4 Hz, 2H), 7.74 (d, *J* = 12.9 Hz, 1H), 7.61 (d, *J* = 8.7 Hz, 1H), 7.53 (d, *J* = 8.4 Hz, 2H), 7.48-7.41 (m, 2H), 7.26 (d, *J* = 2.0 Hz, 1H), 7.18 (t, *J =* 7.9 Hz, 2H), 7.08 (dd, *J =* 8.6, 1.9 Hz, 1H), 6.91 (t, *J =* 7.2 Hz, 1H), 4.04 (dd, *J =* 12.1, 3.5 Hz, 1H), 3.84 (d, *J =* 13.5 Hz, 1H), 3.41 (s, 1H), 3.10 (dd, *J =* 12.7, 8.8 Hz, 1H), 3.02-2.92 (m, 1H), 2.89 (s, 2H), 2.73 (s, 2H), 2.69 (s, 1H), 2.11-1.97 (m, 1H), 1.85-1.67 (m, 1H), 1.51 (q, *J* = 12.7, 10.7 Hz, 2H), 1.32 (s, 9H); LC-MS (ESI, m/z) = 514.4 (M+H⁺).

### Example B-13: Synthesis of methyl 4-(4-(tert-butyl)benzamido)-2-((1-((3-fluorobenzyl)carbamoyl)piperidin-3-yl)amino)benzoate

The compound prepared in Preparation Example 4 (300 mg, 0.59 mmol) and 1-fluoro-3-(isocyanatomethyl)benzene (107 mg, 0.71 mmol) were reacted in the same manner as in Example B-1 to obtain the target compound (221 mg).

¹H NMR (300 MHz, DMSO-d₆) δ 10.25 (s, 1H), 7.87 (d, 2H), 7.79 (d, *J* = 8.8 Hz, 1H), 7.59-7.49 (m, 2H), 7.41 (d, *J =* 1.9 Hz, 1H), 7.33 (d, 1H), 7.11-7.06 (m, 4H), 4.27 (d, *J =* 4.1 Hz, 3H), 3.55 (d, *J* = 15.0 Hz, 2H), 3.24-3.15 (m, 2H), 3.09 (q, *J* = 7.4 Hz, 4H), 1.99 (s, 1H), 1.76-1.46 (m, 3H), 1.33 (s, 9H); LC-MS (ESI, m/z) = 561.3 (M+H⁺).

### Example B-14: Synthesis of 4-(4-(tert-butyl)benzamido)-2-((1-((3-fluorobenzyl)carbamoyl)piperidin-3-yl)amino)benzoic acid

The compound obtained in Example B-13 (200 mg, 0.36 mmol) was reacted in the same manner as in Example B-2 to obtain the target compound (188 mg).

¹H NMR (400 MHz, DMSO-d₆) δ 10.09 (s, 1H), 8.57 (s, 1H), 7.91-7.83 (m, 2H), 7.76 (d, *J* = 8.6 Hz, 1H), 7.58-7.49 (m, 2H), 7.41-7.21 (m, 4H), 7.18-6.93 (m, 11H), 6.81 (t, *J =* 6.2 Hz, 2H), 4.25 (d, *J* = 5.9 Hz, 6H), 4.05-3.88 (m, 1H), 3.76 (d, *J* = 13.2 Hz, 2H), 3.06-2.76 (m, 3H), 2.14-1.95 (m, 1H), 1.69 (s, 1H), 1.52-1.37 (m, 2H), 1.33 (s, 9H); LC-MS (ESI, m/z) = 547.3 (M+H⁺).

### Example B-15: Synthesis of 3-((5-(4-(tert-butyl)benzamido)-2-carbamoylphenyl)amino)-N-(3-fluorobenzyl)piperidine-1-carboxamide

The compound obtained in Example B-14 (100 mg, 0.18 mmol) was reacted in the same manner as in Example B-3 to obtain the target compound (56 mg).

¹H NMR (300 MHz, DMSO-d₆) δ 10.11 (s, 1H), 8.49 (d, *J =* 7.8 Hz, 1H), 7.95-7.79 (m, 2H), 7.72 (s, 1H), 7.61 (d, *J =* 8.7 Hz, 1H), 7.57-7.43 (m, 2H), 7.40-7.20 (m, 2H), 7.20-6.85 (m, 6H), 4.26 (d, *J* = 5.7 Hz, 2H), 3.95 (d, *J* = 10.9 Hz, 1H), 3.78-3.63 (m, 1H), 2.97 (t, *J* = 10.4 Hz, 1H), 2.86 (dd, *J =* 12.8, 8.8 Hz, 1H), 2.14-1.94 (m, 1H), 1.69 (s, 1H), 1.45 (q, *J* = 9.7 Hz, 2H), 1.33 (s, 9H); LC-MS (ESI, m/z) = 546.3 (M+H⁺). .

### Example B-16: Synthesis of methyl 4-(4-(tert-butyl)benzamido)-2-((1-((2,6-diethylphenyl)carbamoyl)piperidin-3-yl)amino)benzoate

The compound prepared in Preparation Example 4 (300 mg, 0.59 mmol) and 2,6-diethylphenyl isocyanate (124 mg, 0.71 mmol) were reacted in the same manner as in Example B-1 to obtain the target compound (127 mg).

¹H NMR (300 MHz, DMSO-d₆) δ 10.23 (s, 1H), 7.93 (d, *J* = 7.7 Hz, 1H), 7.85 (d, *J* = 1.9 Hz, 1H), 7.83-7.79 (m, 2H), 7.77 (s, 1H), 7.58-7.51 (m, 2H), 7.45 (d, *J* = 1.9 Hz, 1H), 7.07 (d, *J* = 1.7 Hz, 1H), 7.05 (s, 1H), 7.03 (t, *J =* 2.2 Hz, 1H), 3.81 (s, 1H), 3.58 (s, 2H), 3.40 (dd, *J =* 12.7, 6.9 Hz, 2H), 2.05 (s, 1H), 1.63 (m, *J* = 31.4 Hz, 3H), 1.32 (s, 9H), 1.09 (t, *J =* 7.5 Hz, 6H); LC-MS (ESI, m/z) = 585.4 (M+H⁺).

### Example B-17: Synthesis of 4-(4-(tert-butyl)benzamido)-2-((1-((2,6-diethylphenyl)carbamoyl)piperidin-3-yl)amino)benzoic acid

The compound obtained in Example B-16 (100 mg, 0.17 mmol) was reacted in the same manner as in Example B-2 to obtain the target compound (70 mg).

¹H NMR (300 MHz, DMSO-d₆) δ 12.22 (s, 1H), 10.20 (s, 1H), 8.04 (d, *J* = 7.6 Hz, 1H), 7.82 (dd, *J* = 8.8, 2.2 Hz, 3H), 7.76 (d, *J* = 8.6 Hz, 1H), 7.53 (dd, *J* = 8.8, 2.3 Hz, 2H), 7.42 (d, *J* = 1.9 Hz, 1H), 7.12 (dd, *J* = 8.6, 6.3 Hz, 1H), 7.06-6.98 (m, 3H), 3.93 (d, *J* = 12.8 Hz, 1H), 3.73 (d, *J* = 13.3 Hz, 1H), 3.52 (s, 1H), 3.21 (dd, *J* = 12.8, 8.0 Hz, 2H), 2.11 (s, 1H), 1.75 (s, 1H), 1.67-1.52 (m, 2H), 1.33 (d, *J* = 2.3 Hz, 9H), 1.09 (t, *J* = 7.5 Hz, 6H); LC-MS (ESI, m/z) = 571.3 (M+H⁺).

### Example B-18: Synthesis of 3-((5-(4-(tert-butyl)benzamido)-2-carbamoylphenyl)amino)-N-(2,6-diethylphenyl)piperidine-1-carboxamide

The compound obtained in Example B-17 (100 mg, 0.18 mmol) was reacted in the same manner as in Example B-3 to obtain the target compound (36 mg).

¹H NMR (300 MHz, DMSO-d₆) δ 10.09 (d, *J =* 6.6 Hz, 1H), 8.61-8.39 (m, 2H), 7.90-7.79 (m, 2H), 7.61 (d, *J =* 8.7 Hz, 1H), 7.53 (d, *J* = 8.3 Hz, 2H), 7.26 (d, *J* = 1.9 Hz, 1H), 7.21-7.12 (m, 1H), 7.12-6.96 (m, 3H), 6.49 (m, *J* = 6.7, 2.5 Hz, 1H), 4.03 (d, *J* = 12.5 Hz, 1H), 3.83 (d, *J =* 13.1 Hz, 1H), 3.68 (s, 3H), 3.18-2.93 (m, 2H), 2.89 (s, 1H), 2.71 (d, *J* = 12.8 Hz, 2H), 2.09 (s, 2H), 1.74 (s, 1H), 1.50 (d, *J* = 8.7 Hz, 2H), 1.32 (s, 9H); LC-MS (ESI, m/z) = 570.4 (M+H⁺).

### Example B-19: Synthesis of methyl 4-(4-(tert-butyl)benzamido)-2-((1-((2-ethoxyphenyl)carbamoyl)piperidin-3-yl)amino)benzoate

The compound prepared in Preparation Example 4 (300 mg, 0.59 mmol) and 2-ethoxyphenyl isocyanate (0.11 mL, 0.71 mmol) were reacted in the same manner as in Example B-1 to obtain the target compound (250 mg).

¹H NMR (300 MHz, DMSO-d₆) δ 10.23 (s, 1H), 7.91-7.78 (m, 3H), 7.81-7.71 (m, 2H), 7.58-7.48 (m, 3H), 7.43 (d, *J =* 1.9 Hz, 1H), 7.14 (dd, *J* = 8.8, 1.8 Hz, 1H), 7.00-6.79 (m, 3H), 4.10-3.88 (m, 2H), 3.82 (d, *J* = 13.3 Hz, 1H), 3.75 (s, 3H), 3.59 (s, 2H), 3.26 (dd, *J* = 13.1, 7.4 Hz, 1H), 2.02 (d, *J =* 13.3 Hz, 1H), 1.76 (d, *J* = 9.9 Hz, 1H), 1.62 (d, *J* = 8.1 Hz, 2H), 1.32 (s, 8H), 1.20 (dt, *J* = 13.4, 7.0 Hz, 3H); LC-MS (ESI, m/z) = 573.3 (M+H⁺).

### Example B-20: Synthesis of 4-(4-(tert-butyl)benzamido)-2-((1-((2-ethoxyphenyl)carbamoyl)piperidin-3-yl)amino)benzoic acid

The compound obtained in Example B-19 (185 mg, 0.33 mmol) was reacted in the same manner as in Example B-2 to obtain the target compound (60 mg).

¹H NMR (300 MHz, DMSO-d₆) δ 10.19 (s, 1H), 8.12-8.00 (m, 1H), 7.94-7.71 (m, 4H), 7.60-7.47 (m, 3H), 7.39 (d, *J =* 1.9 Hz, 1H), 7.11 (dd, *J* = 8.8, 1.8 Hz, 1H), 7.06-6.79 (m, 3H), 4.11 (s, 1H), 4.02-3.85 (m, 2H), 3.68 (d, *J* = 16.3 Hz, 1H), 3.53 (d, *J* = 9.6 Hz, 1H), 3.25-3.09 (m, 2H), 2.06 (s, 1H), 1.59 (s, 2H), 1.33 (s, 1H), 1.32 (s, 6H), 1.28 (d, *J* = 16.5 Hz, 1H), 1.21 (d, *J* = 6.9 Hz, 2H); LC-MS (ESI, m/z) = 559.3 (M+H⁺).

### Example B-21: Synthesis of 3-((5-(4-(tert-butyl)benzamido)-2-carbamoylphenyl)amino)-N-(2-ethoxyphenyl)piperidine-1-carboxamide

The compound obtained in Example B-20 (100 mg, 0.18 mmol) was reacted in the same manner as in Example B-3 to obtain the target compound (30 mg).

¹H NMR (300 MHz, DMSO-d₆) δ 10.09 (s, 1H), 8.55 (d, *J* = 7.8 Hz, 1H), 7.82 (d, *J* = 8.3 Hz, 2H), 7.77 (d, *J* = 1.6 Hz, 1H), 7.63 (s, 1H), 7.59 (d, *J* = 6.5 Hz, 1H), 7.51 (d, *J* = 8.4 Hz, 2H), 7.29 (d, *J* = 2.0 Hz, 1H), 7.10 (dd, *J* = 8.6, 1.8 Hz, 1H), 6.99-6.79 (m, 3H), 4.01-3.87 (m, 3H), 3.76 (d, *J* = 13.3 Hz, 1H), 3.45 (s, 1H), 3.16 (d, *J =* 10.5 Hz, 1H), 3.01 (dd, *J* = 13.0, 8.4 Hz, 1H), 2.06 (d, *J* = 7.0 Hz, 1H), 1.99 (s, 1H), 1.75 (s, 1H), 1.54 (d, *J* = 8.6 Hz, 2H), 1.32 (s, 9H), 1.27-1.12 (m, 4H); LC-MS (ESI, m/z) = 558.4 (M+H⁺).

### Example B-22: Synthesis of methyl 4-(4-(tert-butyl)benzamido)-2-((1-((4-iodophenyl)carbamoyl)piperidin-3-yl)amino)benzoate

The compound prepared in Preparation Example 4 (300 mg, 0.59 mmol) and 4-Iodophenyl isocyanate (173 mg, 0.71 mmol) were reacted in the same manner as in Example B-1 to obtain the target compound (270 mg). ¹H NMR (300 MHz, DMSO-d₆) δ 10.23 (s, 1H), 8.64 (s, 1H), 7.83 (d, *J* = 8.4 Hz, 3H), 7.77 (d, *J* = 8.8 Hz, 1H), 7.61-7.46 (m, 4H), 7.42 (d, *J* = 1.9 Hz, 1H), 7.38-7.26 (m, 2H), 7.11 (dd, *J* = 8.8, 1.8 Hz, 1H), 3.94 (d, *J* = 12.0 Hz, 1H), 3.76 (s, 3H), 3.70 (s, 1H), 3.53 (s, 1H), 3.28-3.14 (m, 2H), 2.13-1.94 (m, 1H), 1.85-1.66 (m, 1H), 1.66-1.51 (m, 2H), 1.33 (s, 9H); LC-MS (ESI) m/z calcd for C₃₁H₃₅IN₄O₄ (M⁺): 654.17, found: 655.2 (M+H⁺).

### Example B-23: Synthesis of 4-(4-(tert-butyl)benzamido)-2-((1-((4-iodophenyl)carbamoyl)piperidin-3-yl)amino)benzoic acid

The compound obtained in Example B-22 (250 mg, 0.38 mmol) was reacted in the same manner as in Example B-2 to obtain the target compound (180 mg).

¹H NMR (300 MHz, DMSO-d₆) δ 12.33 (s, 1H), 10.19 (s, 1H), 8.63 (s, 1H), 8.01 (d, *J* = 7.8 Hz, 1H), 7.89-7.79 (m, 2H), 7.75 (d, *J* = 8.7 Hz, 1H), 7.62-7.42 (m, 4H), 7.38 (d, J = 1.9 Hz, 1H), 7.35-7.26 (m, 2H), 7.08 (dd, *J* = 8.8, 1.8 Hz, 1H), 4.00 (d, *J* = 13.4 Hz, 1H), 3.77 (d, *J* = 13.0 Hz, 1H), 3.51 (s, 1H), 3.24-3.05 (m, 2H), 2.04 (t, *J* = 13.6 Hz, 1H), 1.75 (s, 1H), 1.56 (d, *J* = 7.7 Hz, 2H), 1.33 (s, 9H); LC-MS (ESI) m/z calcd for C₃₀H₃₃IN₄O₄ (M⁺): 640.15, found: 641.1 (M+H⁺).

### Example B-24: Synthesis of 3-((5-(4-(tert-butyl)benzamido)-2-carbamoylphenyl)amino)-N-(4-iodophenyl)piperidine-1-carboxamide

The compound obtained in Example B-23 (100 mg, 0.16 mmol) was reacted in the same manner as in Example B-3 to obtain the target compound (56 mg).

¹H NMR (400 MHz, DMSO-d₆) δ 10.09 (s, 1H), 8.62 (s, 1H), 8.52 (d, *J* = 7.9 Hz, 1H), 7.87-7.78 (m, 2H), 7.76-7.66 (m, 1H), 7.61 (d, *J* = 8.7 Hz, 1H), 7.56-7.45 (m, 4H), 7.38-7.29 (m, 2H), 7.28 (d, *J* = 2.0 Hz, 1H), 7.06 (dd, *J* = 8.6, 1.9 Hz, 1H), 4.12-3.94 (m, 1H), 3.83 (d, *J* = 13.3 Hz, 1H), 3.10 (d, *J =* 10.3 Hz, 1H), 2.97 (dd, *J =* 13.0, 8.6 Hz, 1H), 2.15-2.01 (m, 1H), 1.74 (s, 1H), 1.51 (d, *J* = 8.6 Hz, 2H), 1.33 (s, 9H); LC-MS (ESI) m/z calcd for C₃₀H₃₄IN₅O₃ (M⁺): 639.17, found: 640.2 (M+H⁺).

### Example B-25: Synthesis of methyl 2-((1-(adamantan-1-ylcarbamoyl)piperidin-3-yl)amino)-4-(4-(tert-butyl)benzamido)benzoate

The compound prepared in Preparation Example 4 (300 mg, 0.59 mmol) and 1-isocyanatoadamantane (125 mg, 0.71 mmol) were reacted in the same manner as in Example B-1 to obtain the target compound (265 mg).

¹H NMR (300 MHz, DMSO-d₆) δ 10.23 (s, 1H), 7.95-7.84 (m, 2H), 7.77 (dd, *J* = 8.4, 5.5 Hz, 2H), 7.61-7.47 (m, 2H), 7.40 (d, J = 1.9 Hz, 1H), 7.08 (dd, J = 8.9, 1.8 Hz, 1H), 3.59-3.37 (m, 2H), 3.09-2.85 (m, 2H), 2.09 (s, 2H), 2.07-1.97 (m, 1H), 1.92 (s, 3H), 1.86 (d, J = 2.9 Hz, 5H), 1.67 (s, 1H), 1.51 (d, J = 13.0 Hz, 8H), 1.33 (s, 9H); LC-MS (ESI) m/z calcd for C₃₅H₄₆N₄O₄ (M⁺): 586.35, found: 587.3 (M+H⁺).

### Example B-26: Synthesis of 2-((1-(adamantan-1-ylcarbamoyl)piperidin-3-yl)amino)-4-(4-(tert-butyl)benzamido)benzoic acid

The compound obtained in Example B-25 (230 mg, 0.39 mmol) was reacted in the same manner as in Example B-2 to obtain the target compound (217 mg).

¹H NMR (400 MHz, DMSO-d₆) δ 12.40 (s, 1H), 10.24 (s, 1H), 7.92 (dd, *J =* 13.1, 8.2 Hz, 2H), 7.76 (d, *J =* 8.8 Hz, 1H), 7.55 (dd, *J =* 8.2, 4.9 Hz, 2H), 7.38 (d, *J =* 2.0 Hz, 1H), 7.06 (dd, *J* = 8.8, 1.8 Hz, 1H), 3.86-3.72 (m, 1H), 3.58 (d, *J* = 13.0 Hz, 1H), 2.91 (m, *J* = 30.1, 12.5, 8.2 Hz, 2H), 2.07-1.94 (m, 1H), 1.91 (d, *J* = 4.5 Hz, 3H), 1.84 (d, *J =* 5.8 Hz, 3H), 1.65 (s, 1H), 1.60-1.41 (m, 7H), 1.32 (d, *J =* 2.4 Hz, 9H); LC-MS (ESI) m/z calcd for C₃₄H₄₄N₄O₄ (M⁺): 572.34, found: 573.4 (M+H⁺).

### Example B-27: Synthesis of N-(adamantan-1-yl)-3-((5-(4-(tert-butyl)benzamido)-2-carbamoylphenyl)amino)piperidine-1-carboxamide

The compound obtained in Example B-26 (100 mg, 0.18 mmol) was reacted in the same manner as in Example B-3 to obtain the target compound (50 mg).

¹H NMR (400 MHz, DMSO-d₆) δ 10.09 (s, 1H), 8.43 (d, *J* = 7.7 Hz, 1H), 7.88 (d, *J* = 8.4 Hz, 2H), 7.70 (s, 1H), 7.61 (d, *J* = 8.7 Hz, 1H), 7.54 (d, *J* = 8.2 Hz, 2H), 7.26 (d, *J* = 2.1 Hz, 1H), 7.02 (dd, *J* = 8.6, 1.9 Hz, 2H), 5.46 (s, 1H), 3.85 (d, *J* = 13.2 Hz, 1H), 3.66 (d, *J* = 13.0 Hz, 1H), 2.84 (t, *J =* 10.8 Hz, 1H), 2.71 (dd, *J* = 13.1, 8.9 Hz, 1H), 2.03 (d, *J =* 11.5 Hz, 1H), 1.94-1.80 (m, 9H), 1.64 (s, 1H), 1.53 (s, 6H), 1.46-1.36 (m, 2H), 1.33 (s, 9H); LC-MS (ESI) m/z calcd for C₃₄H₄₅N₅O₃ (M⁺): 571.35, found: 572.3 (M+H⁺).

### Example B-28: Synthesis of methyl 4-(4-(tert-butyl)benzamido)-2-((1-(tert-butylcarbamoyl)piperidin-3-yl)amino)benzoate

The compound prepared in Preparation Example 4 (300 mg, 0.59 mmol) and tert-butyl isocyanate (70 mg, 0.71 mmol) were reacted in the same manner as in Example B-1 to obtain the target compound (237 mg).

¹H NMR (300 MHz, DMSO-d₆) δ 10.23 (s, 1H), 7.96-7.83 (m, 2H), 7.79 (dd, *J* = 8.4, 2.6 Hz, 2H), 7.61-7.49 (m, 2H), 7.41 (d, *J* = 1.9 Hz, 1H), 7.09 (dd, *J* = 8.9, 1.8 Hz, 1H), 5.66 (s, 1H), 3.77 (s, 3H), 3.70 (d, *J* = 2.8 Hz, 1H), 3.51 (s, 2H), 3.13-2.94 (m, 2H), 2.12-1.84 (m, 1H), 1.66 (d, *J* = 6.3 Hz, 1H), 1.49 (q, *J* = 9.9, 8.8 Hz, 2H), 1.33 (s, 9H), 1.21 (s, 9H); LC-MS (ESI) m/z calcd for C₂₉H₄₀N₄O₄ (M⁺): 508.30, found: 509.4 (M+H⁺).

### Example B-29: Synthesis of 4-(4-(tert-butyl)benzamido)-2-((1-(tert-butylcarbamoyl)piperidin-3-yl)amino)benzoic acid

The compound obtained in Example B-28 (200 mg, 0.39 mmol) was reacted in the same manner as in Example B-2 to obtain the target compound (91 mg).

¹H NMR (400 MHz, DMSO-d₆) δ 10.19 (s, 1H), 8.00 (s, 1H), 7.88 (d, *J* = 8.1 Hz, 2H), 7.76 (d, *J* = 8.7 Hz, 1H), 7.55 (d, *J* = 8.2 Hz, 2H), 7.36 (d, J = 2.0 Hz, 1H), 7.04 (dd, *J =* 8.7, 1.9 Hz, 1H), 5.64 (s, 1H), 3.89-3.68 (m, 1H), 3.56 (d, *J* = 13.0 Hz, 2H), 3.06-2.84 (m, 2H), 2.01 (d, *J* = 11.1 Hz, 1H), 1.66 (s, 1H), 1.46 (t, *J* = 8.8 Hz, 2H), 1.33 (s, 9H), 1.21 (s, 9H); LC-MS (ESI) m/z calcd for C₂₈H₃₈N₄O₄ (M⁺): 494.29, found: 495.4 (M+H⁺).

### Example B-30: Synthesis of N-(tert-butyl)-3-((5-(4-(tert-butyl)benzamido)-2-carbamoylphenyl)amino)piperidine-1-carboxamide

The compound obtained in Example B-29 (90 mg, 0.14 mmol) was reacted in the same manner as in Example B-3 to obtain the target compound (43 mg).

¹H NMR (400 MHz, DMSO-d₆) δ 10.09 (s, 1H), 8.45 (d, J = 7.8 Hz, 1H), 7.88 (d, *J* = 8.4 Hz, 2H), 7.70 (s, 1H), 7.61 (d, *J* = 8.7 Hz, 1H), 7.55 (d, *J* = 8.3 Hz, 2H), 7.27 (d, *J* = 2.0 Hz, 1H), 7.12-6.90 (m, 2H), 5.63 (s, 1H), 3.92-3.75 (m, 1H), 3.63 (d, *J* = 13.2 Hz, 1H), 2.91 (d, *J* = 11.3 Hz, 1H), 2.83-2.66 (m, 1H), 2.13-1.93 (m, 1H), 1.66 (d, *J* = 11.5 Hz, 1H), 1.52-1.39 (m, 1H), 1.33 (s, 8H), 1.21 (s, 9H); LC-MS (ESI) m/z calcd for C₂₈H₃₉N₅O₃ (M⁺): 493.31, found: 494.4 (M+H⁺). .

### Example B-31: Synthesis of methyl 4-(4-(tert-butyl)benzamido)-2-((1-((2-methoxy-4-nitrophenyl)carbamoyl)piperidin-3-yl)amino)benzoate

The compound prepared in Preparation Example 4 (300 mg, 0.59 mmol) and 1-isocyanato-2-methoxy-4-nitrobenzene (137 mg, 0.71 mmol) were reacted in the same manner as in Example B-1 to obtain the target compound (223 mg).

¹H NMR (300 MHz, DMSO-d₆) δ 10.22 (s, 1H), 8.10 (d, *J =* 9.0 Hz, 1H), 8.01 (s, 1H), 7.90-7.77 (m, 4H), 7.66 (d, *J =* 8.8 Hz, 1H), 7.60 (d, *J* = 2.5 Hz, 1H), 7.55-7.48 (m, 2H), 7.46 (d, *J* = 1.9 Hz, 1H), 7.06 (dd, *J* = 8.9, 1.8 Hz, 1H), 3.79 (s, 3H), 3.76 (d, *J* = 2.6 Hz, 1H), 3.73 (s, 3H), 3.68-3.38 (m, 4H), 2.06 (s, 1H), 1.88-1.51 (m, 3H), 1.32 (s, 9H); LC-MS (ESI) m/z calcd for C₃₂H₃₇N₅O₇ (M⁺): 603.27, found: 604.3 (M+H⁺).

### Example B-32: Synthesis of 4-(4-(tert-butyl)benzamido)-2-((1-((2-methoxy-4-nitrophenyl)carbamoyl)piperidin-3-yl)amino)benzoic acid

The compound obtained in Example B-31 (190 mg, 0.32 mmol) was reacted in the same manner as in Example B-2 to obtain the target compound (120 mg).

¹H NMR (300 MHz, DMSO-d₆) δ 12.38 (s, 1H), 10.18 (s, 1H), 8.11 (d, *J* = 9.0 Hz, 1H), 8.06 (d, *J* = 7.8 Hz, 1H), 8.01 (s, 1H), 7.83-7.76 (m, 2H), 7.68 (d, *J* = 8.7 Hz, 1H), 7.61 (d, *J =* 2.5 Hz, 1H), 7.50 (d, *J =* 8.2 Hz, 2H), 7.44 (d, *J* = 2.0 Hz, 1H), 7.04 (dd, *J* = 8.9, 1.8 Hz, 1H), 3.87 (d, J = 6.1 Hz, 1H), 3.80 (s, 3H), 3.58 (s, 2H), 3.30 (s, 1H), 2.05 (s, 1H), 1.70 (d, *J* = 40.7 Hz, 4H), 1.32 (d, *J* = 3.9 Hz, 9H); LC-MS (ESI) m/z calcd for C₃₁H₃₅N₅O₇ (M⁺): 589.25, found: 590.2 (M+H⁺).

### Example B-33: Synthesis of 3-((5-(4-(tert-butyl)benzamido)-2-carbamoylphenyl)amino)-N-(2-methoxy-4-nitrophenyl)piperidine-1-carboxamide

The compound obtained in Example B-32 (90 mg, 0.15 mmol) was reacted in the same manner as in Example B-3 to obtain the target compound (56 mg).

¹H NMR (400 MHz, DMSO-d₆) δ 10.07 (s, 1H), 8.61 (d, *J* = 7.9 Hz, 1H), 8.13 (d, *J* = 9.1 Hz, 1H), 8.01 (d, *J =* 15.2 Hz, 1H), 7.86 (tt, *J* = 9.0, 4.1 Hz, 1H), 7.81-7.76 (m, 2H), 7.63 (dd, *J* = 20.4, 2.5 Hz, 1H), 7.57 (d, *J* = 8.6 Hz, 1H), 7.50 (dd, *J* = 7.2, 5.1 Hz, 2H), 7.34 (d, *J* = 2.2 Hz, 1H), 7.07-6.97 (m, 1H), 3.80 (s, 3H), 3.68 (d, *J* = 14.4 Hz, 1H), 3.48 (s, 1H), 3.21 (m, *J =* 13.4, 7.8 Hz, 1H), 2.07 (d, *J* = 20.4 Hz, 1H), 1.79 (s, 1H), 1.59 (s, 2H), 1.32 (s, 9H); LC-MS (ESI) m/z calcd for C₃₁H₃₆N₆O₆ (M⁺): 588.27, found: 589.3 (M+H⁺).

### Example B-34: Synthesis of methyl 4-(4-(tert-butyl)benzamido)-2-((1-((4-(trifluoromethyl)phenyl)carbamoyl)piperidin-3-yl)amino)benzoate

The compound prepared in Preparation Example 4 (300 mg, 0.59 mmol) and (4-Trifluoromethyl)phenyl isocyanate (132 mg, 0.71 mmol) were reacted in the same manner as in Example B-1 to obtain the target compound (158 mg).

¹H NMR (300 MHz, DMSO-d₆) δ 10.23 (s, 1H), 8.93 (s, 1H), 7.84 (dd, *J* = 8.3, 6.4 Hz, 3H), 7.76 (d, *J* = 8.8 Hz, 1H), 7.67 (d, *J* = 8.6 Hz, 2H), 7.52 (dd, *J =* 8.6, 2.1 Hz, 4H), 7.45 (d, *J* = 1.9 Hz, 1H), 7.10 (dd, *J =* 8.9, 1.8 Hz, 1H), 3.96 (d, *J =* 12.2 Hz, 1H), 3.75 (s, 3H), 3.71 (s, 1H), 3.55 (s, 1H), 3.26 (dd, *J* = 12.8, 7.9 Hz, 2H), 2.04 (t, *J =* 13.7 Hz, 1H), 1.77 (d, *J =* 7.0 Hz, 1H), 1.71-1.44 (m, 2H), 1.32 (s, 9H); LC-MS (ESI) m/z calcd for C₃₂H₃₅F₃N₄O₄ (M⁺): 596.26, found: 597.3 (M+H⁺).

### Example B-35: Synthesis of 4-(4-(tert-butyl)benzamido)-2-((1-((4-(trifluoromethyl)phenyl)carbamoyl)piperidin-3-yl)amino)benzoic acid

The compound obtained in Example B-34 (110 mg, 0.18 mmol) was reacted in the same manner as in Example B-2 to obtain the target compound (98 mg).

¹H NMR (400 MHz, DMSO-d₆) δ 10.14 (s, 1H), 8.98 (s, 1H), 8.46 (s, 1H), 7.84 (d, *J =* 8.2 Hz, 2H), 7.76 (d, *J =* 8.6 Hz, 1H), 7.69 (d, *J* = 8.5 Hz, 2H), 7.51 (dd, *J* = 8.6, 2.4 Hz, 4H), 7.32 (s, 1H), 7.11-6.98 (m, 1H), 4.10 (d, *J* = 12.1 Hz, 1H), 3.87 (d, *J* = 13.0 Hz, 1H), 3.19-2.95 (m, 3H), 2.08 (d, *J* = 5.9 Hz, 1H), 1.76 (s, 1H), 1.55 (s, 2H), 1.32 (s, 9H); LC-MS (ESI) m/z calcd for C₃₁H₃₃F₃N₄O₄ (M⁺): 582.25, found: 583.2 (M+H⁺).

### Example B-36: Synthesis of 3-((5-(4-(tert-butyl)benzamido)-2-carbamoylphenyl)amino)-N-(4-(trifluoromethyl)phenyl)piperidine-1-carboxamide

The compound obtained in Example B-35 (70 mg, 0.12 mmol) was reacted in the same manner as in Example B-3 to obtain the target compound (38 mg).

¹H NMR (400 MHz, DMSO-d₆) δ 10.09 (s, 1H), 8.91 (s, 1H), 8.54 (d, *J* = 7.9 Hz, 1H), 7.89-7.76 (m, 2H), 7.69 (d, *J* = 8.5 Hz, 2H), 7.61 (d, *J* = 8.7 Hz, 1H), 7.52 (d, *J* = 8.2 Hz, 4H), 7.30 (d, *J* = 2.0 Hz, 1H), 7.11-6.93 (m, 2H), 4.13-4.02 (m, 1H), 3.90-3.79 (m, 1H), 3.42 (s, 1H), 3.15 (d, *J* = 10.2 Hz, 1H), 3.07-2.97 (m, 1H), 2.07 (s, 1H), 1.76 (s, 1H), 1.53 (d, *J* = 7.2 Hz, 2H), 1.32 (s, 8H); LC-MS (ESI) m/z calcd for C₃₁H₃₄F₃N₅O₃ (M⁺): 581.26, found: 582.3 (M+H⁺).

### Example B-37: Synthesis of methyl 4-(4-(tert-butyl)benzamido)-2-((1-(octylcarbamoyl)piperidin-3-yl)amino)benzoate

The compound prepared in Preparation Example 4 (300 mg, 0.59 mmol) and octyl isocyanate (0.12 mL, 0.70 mmol) were reacted in the same manner as in Example B-1 to obtain the target compound (232 mg).

¹H NMR (300 MHz, DMSO-d₆) δ 10.24 (s, 1H), 7.93-7.84 (m, 2H), 7.77 (dd, *J* = 8.3, 3.4 Hz, 2H), 7.61-7.50 (m, 2H), 7.40 (d, *J =* 1.9 Hz, 1H), 7.08 (dd, *J* = 8.9, 1.8 Hz, 1H), 6.42 (t, *J =* 5.4 Hz, 1H), 3.82 (s, 1H), 3.77 (s, 3H), 3.58 (d, *J* = 13.4 Hz, 1H), 3.12-2.85 (m, 4H), 2.04 (d, *J* = 31.1 Hz, 1H), 1.66 (d, *J* = 8.4 Hz, 1H), 1.59-1.44 (m, 2H), 1.38 (d, *J* = 8.2 Hz, 1H), 1.33 (s, 9H), 1.28-1.09 (m, 11H), 0.91-0.77 (m, 3H); LC-MS (ESI, m/z) = 565.4 (M+H⁺).

### Example B-38: Synthesis of 4-(4-(tert-butyl)benzamido)-2-((1-(octylcarbamoyl)piperidin-3-yl)amino)benzoic acid

The compound obtained in Example B-37 (200 mg, 0.35 mmol) was reacted in the same manner as in Example B-2 to obtain the target compound (197 mg).

¹H NMR (300 MHz, DMSO-d₆) δ 10.21 (s, 1H), 7.89 (d, *J* = 8.4 Hz, 1H), 7.55 (d, *J* = 8.3 Hz, 1H), 2.09 (s, 9H), 1.33 (s, 3H), 1.22 (d, *J* = 12.3 Hz, 3H), 0.90-0.69 (m, 1H); LC-MS (ESI, m/z) = 551.4 (M+H⁺).

### Example B-39: Synthesis of 3-((5-(4-(tert-butyl)benzamido)-2-carbamoylphenyl)amino)-N-octylpiperidine-1-carboxamide

The compound obtained in Example B-37 (100 mg, 0.19 mmol) was reacted in the same manner as in Example B-3 to obtain the target compound (60 mg).

¹H NMR (300 MHz, DMSO-d₆) δ 10.11 (s, 1H), 8.43 (d, *J =* 7.8 Hz, 1H), 7.92-7.83 (m, 2H), 7.76 (s, 1H), 7.58 (dd, *J =* 17.5, 8.4 Hz, 3H), 7.27 (s, 1H), 7.01 (d, *J =* 8.5 Hz, 1H), 6.39 (s, 1H), 3.94 (d, *J* = 12.8 Hz, 1H), 3.74 (d, *J* = 12.8 Hz, 1H), 2.99 (d, *J =* 6.7 Hz, 3H), 2.82 (t, *J =* 11.3 Hz, 1H), 2.76-2.64 (m, 1H), 2.02 (d, J = 12.7 Hz, 1H), 1.65 (s, 1H), 1.33 (s, 15H), 1.22 (d, *J =* 10.6 Hz, 12H), 1.09 (t, *J* = 7.1 Hz, 1H), 0.83 (t, *J* = 6.6 Hz, 4H); LC-MS (ESI, m/z) = 550.4 (M+H⁺).

### Example B-40: Synthesis of methyl 4-(4-(tert-butyl)benzamido)-2-((1-((3-methoxyphenyl)carbamoyl)piperidin-3-yl)amino)benzoate

The compound prepared in Preparation Example 4 (300 mg, 0.59 mmol) and 3-methoxy phenyl isocyanate (105 mg, 0.71 mmol) were reacted in the same manner as in Example B-1 to obtain the target compound (250 mg).

¹H NMR (400 MHz, DMSO-d₆) δ 10.23 (s, 1H), 8.51 (s, 1H), 7.89-7.84 (m, 2H), 7.83 (s, 1H), 7.77 (d, *J* = 8.8 Hz, 1H), 7.60-7.48 (m, 2H), 7.41 (d, *J* = 2.0 Hz, 1H), 7.21-6.99 (m, 4H), 6.49 (dd, *J* = 7.8, 2.5, 1.3 Hz, 1H), 3.91 (dd, *J =* 12.9, 3.4 Hz, 1H), 3.71 (s, 1H), 3.68 (s, 3H), 3.55 (t, *J* = 7.4 Hz, 1H), 3.25 (m, *J* = 20.4, 12.8, 8.1 Hz, 2H), 2.06 (d, *J* = 22.4 Hz, 1H), 1.75 (d, *J* = 6.8 Hz, 1H), 1.64-1.45 (m, 2H), 1.32 (s, 9H); LC-MS (ESI, m/z) = 559.3 (M+H⁺).

### Example B-41: Synthesis of 4-(4-(tert-butyl)benzamido)-2-((1-((3-methoxyphenyl)carbamoyl)piperidin-3-yl)amino)benzoic acid

The compound obtained in Example B-40 (200 mg, 0.36 mmol) was reacted in the same manner as in Example B-2 to obtain the target compound (150 mg).

¹H NMR (300 MHz, DMSO-d₆) δ 10.19 (s, 1H), 8.50 (s, 1H), 8.03 (d, *J* = 7.9 Hz, 1H), 7.90-7.82 (m, 2H), 7.76 (d, *J* = 8.7 Hz, 1H), 7.53 (d, *J =* 8.3 Hz, 2H), 7.37 (d, *J* = 2.0 Hz, 1H), 7.21-6.99 (m, 3H), 6.49 (m, *J* = 7.1, 2.4 Hz, 1H), 3.98 (d, *J =* 12.6 Hz, 1H), 3.82-3.70 (m, 1H), 3.68 (s, 3H), 3.50 (s, 1H), 3.25-3.04 (m, 2H), 2.17-1.94 (m, 1H), 1.75 (s, 1H), 1.54 (d, J = 7.9 Hz, 2H), 1.33 (d, *J* = 1.7 Hz, 9H); LC-MS (ESI, m/z) = 545.3 (M+H⁺).

### Example B-42: Synthesis of 3-((5-(4-(tert-butyl)benzamido)-2-carbamoylphenyl)amino)-N-(3-methoxyphenyl)piperidine-1-carboxamide

The compound obtained in Example B-41 (100 mg, 0.18 mmol) was reacted in the same manner as in Example B-3 to obtain the target compound (36 mg).

¹H NMR (300 MHz, DMSO-d₆) δ 10.09 (d, *J* = 6.6 Hz, 1H), 8.61-8.39 (m, 2H), 7.90-7.79 (m, 2H), 7.61 (d, *J* = 8.7 Hz, 1H), 7.53 (d, *J* = 8.3 Hz, 2H), 7.26 (d, *J* = 1.9 Hz, 1H), 7.21-7.12 (m, 1H), 7.12-6.96 (m, 3H), 6.49 (m, *J* = 6.7, 2.5 Hz, 1H), 4.03 (d, *J* = 12.5 Hz, 1H), 3.83 (d, *J* = 13.1 Hz, 1H), 3.68 (s, 3H), 3.18-2.93 (m, 2H), 2.89 (s, 1H), 2.71 (d, *J* = 12.8 Hz, 2H), 2.09 (s, 2H), 1.74 (s, 1H), 1.50 (d, *J* = 8.7 Hz, 2H), 1.32 (s, 9H); LC-MS (ESI, m/z) = 544.3 (M+H⁺).

### Example C-1: Synthesis of methyl 4-(4-(tert-butyl)benzamido)-2-((1-propionylpiperidin-3-yl)amino)benzoate

The compound prepared in Preparation Example 4 (300 mg, 0.59 mmol) was dissolved in DCM (6 mL), and then DIPEA (179 mg, 1.77 mmol) and propionyl chloride (65 mg, 0.71 mmol) were sequentially added and stirred at ambient temperature for 1 hour. The reaction product was confirmed by TLC, diluted with EtOAc, and washed with an aqueous NaHCO₃ solution. The organic layer was dried over anhydrous Na₂SO₄, concentrated under reduced pressure, and separated by column chromatography to obtain the target compound (130 mg).

¹H NMR (300 MHz, DMSO-d₆) δ 10.25 (d, *J* = 3.5 Hz, 1H), 7.88 (d, *J =* 8.3 Hz, 2H), 7.83-7.73 (m, 2H), 7.56 (d, *J* = 7.6 Hz, 2H), 7.03 (d, *J* = 8.9 Hz, 1H), 3.99-3.80 (m, 1H), 3.78 (s, 3H), 3.60 (d, *J =* 44.7 Hz, 3H), 3.21 (s, 2H), 2.46-2.09 (m, 2H), 1.67 (s, 2H), 1.59-1.42 (m, 1H), 1.33 (s, 9H), 1.06-0.86 (m, 3H); LC-MS (ESI, m/z) = 466.4 (M+H⁺).

### Example C-2: Synthesis of 4-(4-(tert-butyl)benzamido)-2-((1-propionylpiperidin-3-yl)amino)benzoic acid

The compound obtained in Example C-1 (124 mg, 0.27 mmol) was dissolved in 5 mL of a THF/MeOH/H₂O mixture (mixing ratio = 3 : 1 : 1), and LiOH•H₂O (30 mg, 0.70 mmol) was added and stirred at ambient temperature for 24 hours. The reaction product was confirmed by TLC, and 1M HCl aqueous solution was added dropwise so that the pH of the reaction solution was 2 to 3. The resulting white solid was filtered to obtain the target compound (55 mg).

¹H NMR (300 MHz, DMSO-d₆) δ 10.21 (s, 1H), 8.07 (d, J = 17.8 Hz, 1H), 7.88 (d, *J =* 8.2 Hz, 2H), 7.76 (dd, *J* = 8.8, 5.1 Hz, 1H), 7.64-7.47 (m, 3H), 7.02 (dd, *J* = 20.7, 8.8 Hz, 1H), 3.83 (dd, *J* = 33.3, 12.8 Hz, 1H), 3.64 (d, *J =* 12.8 Hz, 1H), 3.51 (s, 2H), 3.25 (dd, *J =* 13.1, 7.3 Hz, 4H), 2.32 (m, *J =* 30.9, 16.3, 8.1 Hz, 2H), 1.99 (s, 1H), 1.74-1.43 (m, 3H), 1.33 (s, 9H), 0.97 (m, *J* = 14.7, 7.3 Hz, 3H); LC-MS (ESI, m/z) = 452.4 (M+H⁺).

### Example C-3: Synthesis of 4-(tert-butyl)-N-(4-carbamoyl-3-((1-propionylpiperidin-3-yl)amino)phenyl)benzamide

The compound obtained in Example C-2 (40 mg, 0.09 mmol) and NH₄Cl (12 mg, 0.24 mmol) were dissolved in DMF (4 mL), and DIPEA (30 mg, 0.24 mmol) and HBTU (45 mg, 0.12 mmol) were added and stirred at ambient temperature for 5 hours. The reaction product was confirmed by TLC, diluted with EtOAc, and washed with an aqueous NaHCO₃ solution. The organic layer was dried over anhydrous Na₂SO₄, concentrated under reduced pressure, and separated by column chromatography to obtain the target compound (12 mg).

¹H NMR (300 MHz, DMSO-d₆) δ 10.12 (s, 1H), 8.61 (d, *J* = 7.7 Hz, 1H), 7.87 (d, *J* = 8.4 Hz, 2H), 7.72 (s, 1H), 7.62 (d, *J* = 8.3 Hz, 1H), 7.56 (d, *J* = 8.3 Hz, 2H), 7.45 (d, *J* = 9.3 Hz, 1H), 7.29 (d, *J* = 7.7 Hz, 1H), 7.24 (d, *J* = 10.4 Hz, 1H), 7.04 (d, *J* = 8.8 Hz, 1H), 6.97 (t, J = 7.1 Hz, 1H), 3.99 (d, *J =* 12.4 Hz, 1H), 3.73 (m, *J* = 24.8, 13.2 Hz, 2H), 3.54 (s, 1H), 2.45-2.05 (m, 2H), 1.98 (s, 1H), 1.64 (s, 2H), 1.50 (s, 2H), 1.33 (s, 9H), 0.97 (m, *J* = 14.2, 7.3 Hz, 3H); LC-MS (ESI, m/z) = 451.4 (M+H⁺).

### Example C-4: Synthesis of methyl 2-((1-benzoylpiperidin-3-yl)amino)-4-(4-(tert-butyl)benzamido)benzoate

The compound prepared in Preparation Example 4 (100 mg, 0.20 mmol) and benzoyl chloride (36 mg, 0.26 mmol) were reacted in the same manner as in Example C-1 to obtain the target compound (91 mg).

¹H NMR (300 MHz, DMSO-d₆) δ 10.16 (s, 1H), 7.89 (d, *J* = 8.1 Hz, 3H), 7.78 (d, *J* = 8.8 Hz, 1H), 7.57 (d, *J* = 8.1 Hz, 2H), 7.50-7.16 (m, 6H), 7.07 (s, 1H), 3.80 (d, *J* = 1.2 Hz, 3H), 2.09 (d, *J* = 1.2 Hz, 11H), 1.33 (d, *J* = 1.3 Hz, 9H); LC-MS (ESI, m/z) = 514.5 (M+H⁺).

### Example C-5: Synthesis of 2-((1-benzoylpiperidin-3-yl)amino)-4-(4-(tert-butyl)benzamido)benzoic acid

The compound obtained in Example C-4 (90 mg, 0.18 mmol) was reacted in the same manner as in Example C-2 to obtain the target compound (50 mg).

¹H NMR (300 MHz, DMSO-d₆) δ 10.16 (s, 1H), 7.89 (d, J = 8.1 Hz, 3H), 7.78 (d, J = 8.8 Hz, 1H), 7.57 (d, J = 8.1 Hz, 2H), 7.50-7.16 (m, 6H), 7.07 (s, 1H), 3.80 (d, J = 1.2 Hz, 3H), 2.09 (d, J = 1.2 Hz, 11H), 1.33 (d, J = 1.3 Hz, 9H); LC-MS (ESI, m/z) = 500.5 (M+H⁺).

### Example C-6: Synthesis of N-(3-((1-benzoylpiperidin-3-yl)amino)-4-carbamoylphenyl)-4-(tert-butyl)benzamide

The compound obtained in Example C-5 (50 mg, 0.10 mmol) was reacted in the same manner as in Example C-3 to obtain the target compound (40 mg).

¹H NMR (300 MHz, DMSO-d₆) δ 10.06 (d, J = 33.7 Hz, 1H), 8.61 (s, 1H), 8.29-8.05 (m, 1H), 7.96 (s, 1H), 7.88 (d, J = 8.4 Hz, 2H), 7.58 (t, J = 10.0 Hz, 4H), 7.50-6.95 (m, 8H), 2.90 (s, 3H), 2.74 (d, J = 0.6 Hz, 3H), 2.69 (s, 2H), 1.33 (s, 9H); LC-MS (ESI, m/z) = 499.5 (M+H⁺).

### Example D-1: Synthesis of methyl 4-(4-(tert-butyl)benzamido)-2-((1-ethylpiperidin-3-yl)amino)benzoate

The compound prepared in Preparation Example 4 (300 mg, 0.59 mmol) and ethyl bromide (84 mg, 1.18 mmol) were dissolved in anhydrous DCM (6 mL), then DIPEA (229 mg, 1.77 mmol) was added, and the mixture was stirred at ambient temperature for 1 hour. The reaction product was confirmed by TLC, diluted with EtOAc, and washed with an aqueous NaHCO₃ solution. The organic layer was dried over anhydrous Na₂SO₄, concentrated under reduced pressure, and separated by column chromatography to obtain the target compound (88 mg).

¹H NMR (300 MHz, DMSO-d₆) δ 10.22 (s, 1H), 7.95 (d, *J* = 7.8 Hz, 1H), 7.91-7.83 (m, 2H), 7.76 (d, J = 8.8 Hz, 1H), 7.61-7.49 (m, 2H), 7.37 (d, J = 1.9 Hz, 1H), 7.08 (dd, J = 8.8, 1.8 Hz, 1H), 3.77 (s, 3H), 3.58 (s, 1H), 2.71 (d, J = 11.1 Hz, 1H), 2.37 (m, *J* = 12.1, 7.1, 5.1 Hz, 5H), 1.80-1.61 (m, 2H), 1.58-1.42 (m, 2H), 1.33 (s, 9H), 1.02 (t, *J* = 7.1 Hz, 3H); LC-MS (ESI, m/z) = 438.4 (M+H⁺).

### Example D-2: Synthesis of 4-(4-(tert-butyl)benzamido)-2-((1-ethylpiperidin-3-yl)amino)benzoic acid

The compound obtained in Example D-1 (80 mg, 0.18 mmol) was dissolved in 5 mL of a THF/MeOH/H₂O mixture (mixing ratio = 3 : 1 : 1), and then lithium hydroxide monohydrate (30 mg, 0.70 mmol) was added and stirred at ambient temperature for 24 hours. The reaction product was confirmed by TLC, and 1M HCl aqueous solution was added dropwise so that the pH of the reaction solution was 2 to 3. The resulting white solid was filtered to obtain the target compound (33 mg).

¹H NMR (400 MHz, DMSO-d₆) δ 12.51 (s, 1H), 10.33 (s, 1H), 8.01-7.83 (m, 3H), 7.79 (d, *J* = 8.7 Hz, 1H), 7.56 (dd, J = 8.9, 2.3 Hz, 3H), 7.05 (d, *J* = 8.8 Hz, 1H), 3.86 (s, 1H), 3.61 (s, 1H), 3.14 (s, 3H), 2.84 (s, 2H), 2.16 (s, 1H), 1.92 (d, *J =* 7.0 Hz, 2H), 1.33 (s, 9H), 1.25 (d, *J =* 7.3 Hz, 4H); LC-MS (ESI, m/z) = 424.5 (M+H⁺).

### Example D-3: Synthesis of 4-(tert-butyl)-N-(4-carbamoyl-3-((1-ethylpiperidin-3-yl)amino)phenyl)benzamide

The compound obtained in Example D-2 (30 mg, 0.07 mmol) and NH₄Cl (12 mg, 0.24 mmol) were dissolved in DMF (4 mL), and then DIPEA (30 mg, 0.24 mmol) and HBTU (45 mg, 0.12 mmol) were added and stirred at ambient temperature for 5 hours. The reaction product was confirmed by TLC, diluted with EtOAc, and washed with an aqueous NaHCO₃ solution. The organic layer was dried over anhydrous Na₂SO₄, concentrated under reduced pressure, and separated by column chromatography to obtain the target compound (16 mg).

¹H NMR (300 MHz, DMSO-d₆) δ 10.28 (s, 1H), 8.50 (d, *J =* 7.8 Hz, 1H), 7.97-7.84 (m, 2H), 7.78 (dd, *J =* 8.4, 1.1 Hz, 2H), 7.67-7.49 (m, 4H), 7.46-7.35 (m, 2H), 7.30 (m, *J =* 8.1, 6.8, 1.2 Hz, 1H), 7.05 (dd, *J =* 8.6, 1.9 Hz, 2H), 3.63 (s, 2H), 3.25 (d, J = 11.2 Hz, 2H), 3.02 (s, 2H), 2.84-2.71 (m, 2H), 1.84 (d, *J* = 13.2 Hz, 1H), 1.69 (d, *J* = 11.3 Hz, 1H), 1.33 (s, 9H), 1.25 (t, *J* = 3.6 Hz, 1H), 1.19-1.05 (m, 3H); LC-MS (ESI, m/z) = 423.4 (M+H⁺).

### Example D-4: Synthesis of methyl 4-(4-(tert-butyl)benzamido)-2-((1-butylpiperidin-3-yl)amino)benzoate

The compound prepared in Preparation Example 4 (200 mg, 0.376 mmol), butyraldehyde (0.04 mL, 0.45 mmol), and AcOH (0.02 mL, 0.41 mmol) were dissolved in DCM (5 mL), and then NaBH(OAc)₃ (120 mg, 0.563 mmol) was added and stirred at ambient temperature for 4 hours. The reaction product was confirmed by TLC, diluted with EtOAc, and washed with an aqueous NaHCO₃ solution. The organic layer was dried over anhydrous Na₂SO₄, concentrated under reduced pressure, and separated by column chromatography to obtain the target compound (123 mg).

¹H NMR (500 MHz, DMSO-d₆) δ 10.23 (s, 1H), 8.02-7.94 (m, 1H), 7.87 (d, J = 8.0 Hz, 2H), 7.75 (d, J = 9.0 Hz, 1H), 7.54 (d, J = 8.0 Hz, 2H), 7.38 (s, 1H), 7.07 (d, J = 9.0 Hz, 1H), 3.76 (s, 3H), 3.58 (s, 1H), 2.64 (s, 1H), 2.43-2.18 (m, 5H), 1.75-1.62 (m, 2H), 1.49 (s, 2H), 1.40 (s, 3H), 1.31 (s, 10H), 0.87 (d, J = 7.5 Hz, 3H); LC-MS (ESI, m/z) = 466.5 (M+H⁺).

### Example D-5: Synthesis of 4-(4-(tert-butyl)benzamido)-2-((1-butylpiperidin-3-yl)amino)benzoic acid

The compound obtained in Example D-4 (158 mg, 0.34 mmol) was reacted in the same manner as in Example D-2 to obtain the target compound (121 mg).

¹H NMR (500 MHz, DMSO-d₆) δ 12.52 (s, 1H), 10.35 (s, 1H), 7.97-7.87 (m, 3H), 7.78 (d, *J* = 8.8 Hz, 1H), 7.57-7.47 (m, 3H), 7.12 (d, *J* = 8.8 Hz, 1H), 3.89 (s, 1H), 3.59 (s, 1H), 3.38 (s, 1H), 3.04 (s, 2H), 2.84 (s, 2H), 2.13 (s, 1H), 1.91 (s, 2H), 1.69 (s, 2H), 1.32 (s, 12H), 0.90 (t, *J =* 7.4 Hz, 3H); LC-MS (ESI, m/z) = 452.4 (M+H⁺).

### Example D-6: Synthesis of 4-(tert-butyl)-N-(3-((1-butylpiperidin-3-yl)amino)-4-carbamoylphenyl)benzamide

The compound obtained in Example D-5 (90 mg, 0.2 mmol) was reacted in the same manner as in Example D-3 to obtain the target compound (45 mg).

¹H NMR (500 MHz, DMSO-d₆) δ 10.16 (s, 1H), 9.32 (s, 1H), 8.54 (s, 1H), 7.88 (d, *J =* 8.0 Hz, 2H), 7.78 (s, 1H), 7.63 (d, *J =* 8.6 Hz, 1H), 7.56 (d, *J =* 8.0 Hz, 2H), 7.12 (s, 1H), 6.81 (s, 1H), 3.80-3.41 (m, 2H), 3.07-2.81 (m, 3H), 2.10 (d, *J* = 15.0 Hz, 1H), 1.96 (d, *J* = 15.0 Hz, 1H), 1.77 (s, 1H), 1.65 (s, 1H), 1.33 (s, 11H), 0.90 (s, 3H); LC-MS (ESI, m/z) = 451.5 (M+H⁺).

### Example D-7: Synthesis of methyl 4-(4-(tert-butyl)benzamido)-2-((1-methylpiperidin-3-yl)amino)benzoate

The compound prepared in Preparation Example 4 (200 mg, 0.376 mmol) was dissolved in ACN (5.4 mL, 0.07 M), and then formaldehyde solution (0.36 mL, 1.31 mmol) and NaBH(OAc)₃ (320 mg, 1.5 mmol) were sequentially added dropwise, followed by stirring at ambient temperature for 24 hours. The reaction product was confirmed by TLC, diluted with EtOAc, and washed with an aqueous NaHCO₃ solution. The organic layer was dried over anhydrous Na₂SO₄, concentrated under reduced pressure, and separated by column chromatography to obtain the target compound (142 mg).

¹H NMR (500 MHz, CDCl₃) δ 9.24 (s, 1H), 8.06 (d, *J* = 7.4 Hz, 2H), 7.92 (d, J = 8.9 Hz, 1H), 7.81 (s, 1H), 7.65 (s, 1H), 7.44-7.38 (m, 2H), 7.34 (s, 1H), 3.95 (s, 2H), 3.83 (s, 3H), 3.40 (s, 1H), 2.70 (s, 3H), 2.60 (d, J = 12.0 Hz, 1H), 2.22 (d, J = 12.0 Hz, 2H), 2.16 (s, 1H), 2.04-1.97 (m, 2H), 1.54 (t, J = 13.0 Hz, 1H), 1.30 (s, 10H); LC-MS (ESI, m/z) = 424.4 (M+H⁺).

### Example D-8: Synthesis of 4-(4-(tert-butyl)benzamido)-2-((1-methylpiperidin-3-yl)amino)benzoic acid

The compound obtained in Example D-7 (144 mg, 0.34 mmol) was reacted in the same manner as in Example D-2 to obtain the target compound (108 mg).

¹H NMR (500 MHz, DMSO-d₆) δ 10.52 (s, 1H), 10.34 (s, 1H), 7.94 (d, *J* = 8.3 Hz, 2H), 7.88 (d, *J* = 7.9 Hz, 1H), 7.78 (d, *J =* 8.6 Hz, 1H), 7.54 (d, *J =* 8.6 Hz, 2H), 7.48 (s, 1H), 7.12 (d, *J =* 8.6 Hz, 1H), 3.82 (s, 1H), 3.63 (d, J = 12.0 Hz, 1H), 3.40 (d, *J =* 12.0 Hz, 1H), 2.88 (t, *J =* 10.6 Hz, 2H), 2.79 (s, 3H), 2.16 (d, *J* = 12.0 Hz, 1H), 1.90 (q, J = 15.0, 13.6 Hz, 3H), 1.32 (s, 12H); LC-MS (ESI, m/z) = 410.4 (M+H⁺).

### Example D-9: Synthesis of 4-(tert-butyl)-N-(4-carbamoyl-3-((1-methylpiperidin-3-yl)amino)phenyl)benzamide

The compound obtained in Example D-8 (70 mg, 0.171 mmol) was reacted in the same manner as in Example D-3 to obtain the target compound (27 mg).

¹H NMR (500 MHz, DMSO-d₆) δ 10.10 (s, 1H), 8.44 (s, 1H), 7.88 (s, 2H), 7.69 (s, 1H), 7.59-7.53 (m, 2H), 7.24 (s, 1H), 7.05 (s, 1H), 7.01-6.89 (m, 1H), 3.49 (s, 1H), 2.84 (s, 1H), 2.23 (s, 4H), 2.02 (s, 1H), 1.83 (s, 1H), 1.70 (s, 1H), 1.55 (s, 1H), 1.32 (s, 10H); LC-MS (ESI, m/z) = 409.4 (M+H⁺).

### Example D-10: Synthesis of 4-(tert-butyl)-N-(4-carbamoyl-3-(piperidin-3-ylamino)phenyl)benzamide trifluoroacetate

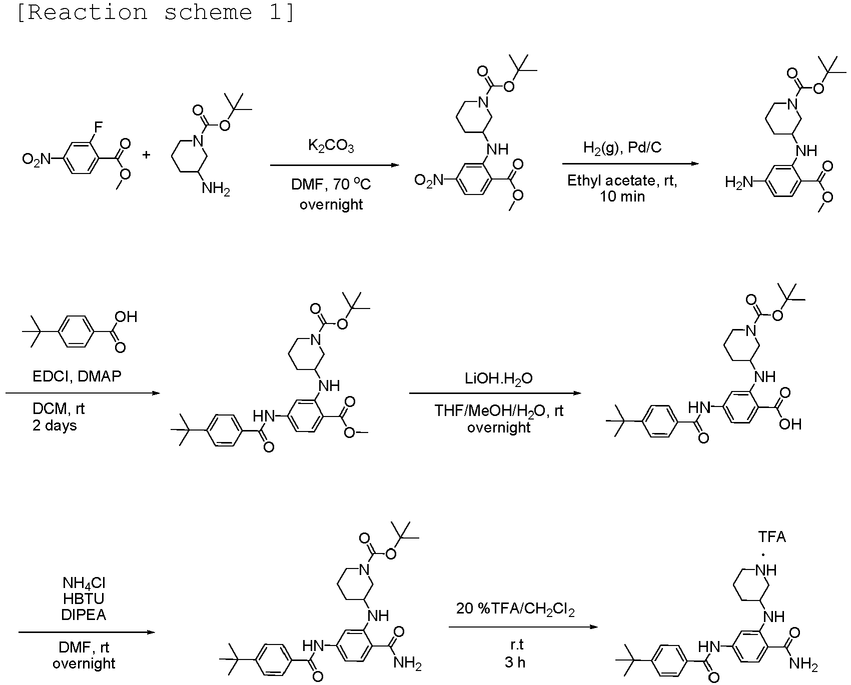

### Step 1: Synthesis of tert-butyl 3-((2- (methoxycarbonyl)-5-nitrophenyl)amino)piperidine-1-

### carboxylate

Methyl 2-fluoro-4-nitrobenzoate (5.00 g, 25.11 mmol) was added to DMF (250 ml), and tert-butyl 3-aminopiperidine-1-carboxylate (5.53 g, 27.62 mmol), potassium carbonate (6.94 g, 50.22 mmol) were added and stirred at 70°C overnight. The product was confirmed by TLC and extracted with ethyl acetate/water and ammonium chloride. The resulting product was dried over anhydrous sodium sulfate, concentrated, and separated by column chromatography to obtain the target compound (6.01 g, 63 %).

¹H NMR (400 MHz, DMSO-d₆) δ 8.06 (t, *J* = 9.0 Hz, 2H), 7.58-7.49 (m, 1H), 7.36 (dd, *J =* 8.7, 2.2 Hz, 1H), 3.85 (s, 3H), 3.76 (m, *J* = 7.2, 3.8 Hz, 1H), 3.50 (s, 4H), 1.94 (m, *J* = 12.8, 8.3, 3.9 Hz, 1H), 1.81-1.49 (m, 4H), 1.31 (d, *J* = 70.9 Hz, 9H).

### Step 2: Synthesis of tert-butyl 3-((5-amino-2-(methoxycarbonyl)phenyl)amino)piperidine-1-carboxylate

The compound obtained in Step 1 (200 mg, 0.53 mmol) and Pd/C (2.80 mg, 0.03 mmol) were added, and stirred at room temperature under hydrogen gas for 10 minutes. The reaction product was confirmed by TLC, filtered through a Celite filter using methanol, and concentrated. The concentrate was extracted with ethyl acetate/water and brine. The reaction product was dried over anhydrous sodium sulfate and concentrated to obtain the target compound (168 mg, 91%).

¹H NMR (300 MHz, Chloroform-d) δ 8.15 (d, *J* = 7.5 Hz, 1H), 8.07 (d, *J* = 8.7 Hz, 1H), 7.57 (d, *J* = 2.2 Hz, 1H), 7.38 (d, *J* = 8.8, 2.2 Hz, 1H), 3.91 (s, 3H), 3.87-3.82 (m, 1H), 3.62 (s, 2H), 3.39-3.16 (m, 2H), 2.08 (d, *J* = 12.7 Hz, 1H), 1.90-1.61 (m, 3H), 1.44 (s, 9H).

### Step 3: Synthesis of tert-butyl 3-((5-(4-(tert-butyl)benzamido)-2-(methoxycarbonyl)phenyl)amino)piperidine-1-carboxylate

The compound obtained in Step 2 (800 mg, 2.29 mmol), EDCI (526 mg, 2.75 mmol), and DMAP (27 mg, 0.23 mmol) were added to DCM (23 ml) and stirred for 20 minutes. Then, 4-(tert-butyl)benzoic acid (619 mg, 2.75 mmol) was added and stirred at room temperature for 2 days. The reaction product was extracted under dichloromethane/water conditions. The organic solvent layer was dried over anhydrous sodium sulfate, concentrated, and separated by column chromatography to obtain the target compound (1.06 g, 90 %).

¹H NMR (300 MHz, DMSO-d₆) δ 10.23 (s, 1H), 7.88 (d, *J* = 8.4 Hz, 3H), 7.78 (d, *J* = 8.8 Hz, 1H), 7.56 (d, *J* = 8.4 Hz, 2H), 7.05 (s, 1H), 3.76 (s, 3H), 1.95 (s, 1H), 1.68 (s, 2H), 1.54 (s, 2H), 1.33 (s, 9H), 1.24 (s, 9H).

### Step 4: Synthesis of 2-((1-(tert-butoxycarbonyl)piperidin-3-yl)amino)-4-(4-(tert-butyl)benzamido)benzoic acid

The compound obtained in Step 3 (100 mg, 0.20 mmol) was dissolved in THF/MeOH/H₂O (mixing ratio = 3 : 1 : 1, 5 mL), and lithium hydroxide monohydrate (24 mg, 0.59 mmol) was added, followed by stirring overnight at room temperature. The product was confirmed by TLC, then 1M HCl aqueous solution was added to adjust the pH to 2-3, and the resulting solid product was filtered to obtain the target compound (86 mg, 88%).

¹H NMR (300 MHz, DMSO-d₆) δ 10.03 (s, 1H), 8.96 (s, 1H), 7.88 (d, 2H), 7.76 (d, J = 8.5 Hz, 1H), 7.54 (d, 2H), 7.29 (s, 1H), 6.87 (s, 1H), 3.78 (d, J = 12.5 Hz, 2H), 3.34 (s, 2H), 3.16-2.97 (m, 2H), 1.97 (d, *J* = 16.8 Hz, 1H), 1.71 (s, 1H), 1.51 (d, *J* = 17.3 Hz, 3H), 1.33 (s, 9H), 1.29 (s, 9H).

### Step 5: Synthesis of tert-butyl 3-((5-(4-(tert-butyl)benzamido)-2-carbamoylphenyl)amino)piperidine-1-carboxylate

The compound obtained in Step 4 (80 mg, 0.16 mmol) was added to DMF (2 ml), then DIPEA (0.09 ml, 0.48 mmol) and HBTU (91.82 g, 0.24 mmol) were added, and the reaction mixture was stirred at room temperature for 30 minutes. NH₄Cl (25.90 mg, 0.48 mmol) was added, and the resulting mixture was stirred at room temperature overnight. The product was confirmed by TLC and extracted with ethyl acetate/water and ammonium chloride. The resulting product was dried over anhydrous sodium sulfate, concentrated, and separated by column chromatography to obtain the target compound (62 mg, 78 %).

¹H NMR (300 MHz, DMSO-d₆) δ 10.10 (s, 1H), 8.59 (s, 1H), 7.88 (d, *J* = 8.3 Hz, 2H), 7.70 (s, 1H), 7.61 (d, *J =* 8.7 Hz, 1H), 7.55 (d, *J =* 8.4 Hz, 2H), 7.36 (s, 1H), 6.97 (s, 2H), 3.64 (d, *J* = 12.3 Hz, 1H), 3.39 (s, 2H), 3.26 (s, 1H), 1.96 (t, *J =* 9.6 Hz, 1H), 1.68 (s, 1H), 1.51 (d, *J =* 15.7 Hz, 2H), 1.33 (s, 9H), 1.27 (s, 9H). LC MS (ESI) m/z calcd for C₂₈H₃₈N₄O₄ (M⁺) 494.29; found 395.5(M+H⁺). .

### Step 6: Synthesis of 4-(tert-butyl)-N-(4-carbamoyl-3-(piperidin-3-ylamino)phenyl)benzamide trifluoroacetate

The compound obtained in Step 5 (50 mg, 0.10 mmol) was added to 20% TFA in DCM and reacted at room temperature for 3 hours. The product was confirmed by TLC and the solid compound was washed several times with diethyl ether to obtain the target compound as a white solid (25 mg, 63%).

¹H NMR (300 MHz, DMSO-d₆) δ 10.16 (s, 1H), 8.74 (s, 2HI), 8.53 (d, *J* = 7.3 Hz, 1H), 7.97-7.84 (m, 2H), 7.80 (d, *J* = 8.4 Hz, 1H), 7.64 (d, *J* = 8.7 Hz, 1H), 7.60-7.50 (m, 2H), 7.41 (d, *J* = 1.9 Hz, 1H), 7.18-7.03 (m, 1H), 6.93 (dd, *J =* 8.7, 1.9 Hz, 1H), 3.66 (s, 3H), 3.20 (s, 2H), 2.88 (s, 3H), 2.10 (d, *J =* 11.8 Hz, 1H), 1.96-1.83 (m, 1H), 1.76 (d, *J* = 11.0 Hz, 1H), 1.52 (d, *J* = 10.1 Hz, 2H), 1.33 (s, 10H).

¹³C NMR (101 MHz, DMSO-d₆) δ 171.66, 166.21, 155.10, 149.55, 143.76, 132.54, 130.46, 128.01, 125.68, 110.03, 107.51, 102.56, 46.78, 46.22, 43.42, 35.17, 31.38, 29.23, 21.23.

LC MS (ESI) m/z calcd for C₂₃H₃₀N₄O₂ (M⁺) 394.5; found 395.5 (M+H⁺). .

### Example D-11: Synthesis of 4-(tert-butyl)-N-(4-carbamoyl-3-(piperidin-4-ylamino)phenyl)benzamide trifluoroacetate

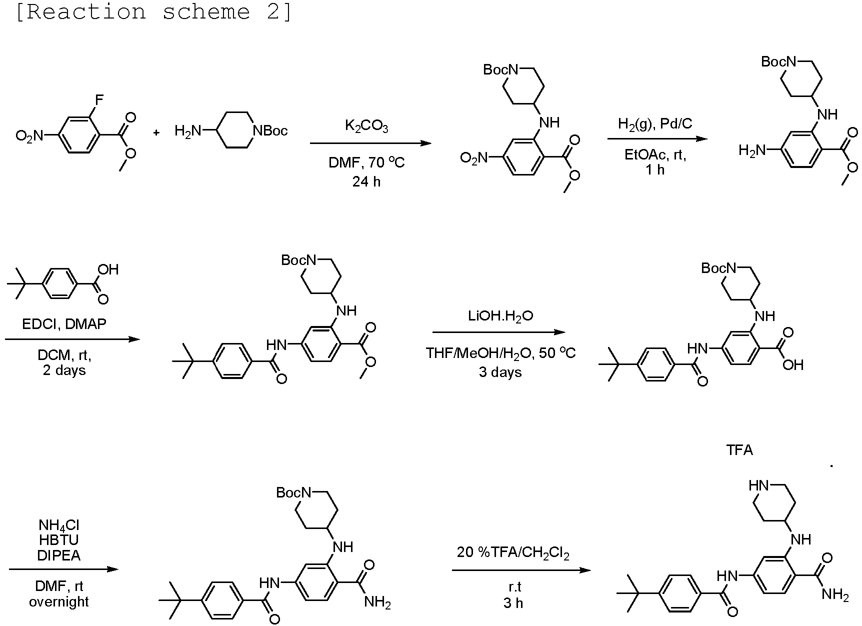

### Step 1: Synthesis of tert-butyl 4-((2-(methoxycarbonyl)-5-nitrophenyl)amino)piperidine-1-carboxylate

Methyl 2-fluoro-4-nitrobenzoate (1.00 g, 5.02 mmol) was added to DMF (50 mL), and tert-butyl 4-aminopiperidine-1-carboxylate (1.11 g, 5.53 mmol) and potassium carbonate (2.08 g, 15.07 mmol) were added and stirred at 70°C for 24 hours. The resulting compound was extracted with ethyl acetate/water and saturated aqueous NH₄Cl solution. The resulting product was dried over anhydrous sodium sulfate, concentrated, and separated by column chromatography to obtain the target compound (1.20 g, 63 %).

¹H NMR (300 MHz, Chloroform-d) δ 8.07 (d, *J* = 8.8 Hz, 2H), 7.51 (d, *J* = 2.2 Hz, 1H), 7.36 (dd, *J* = 8.8, 2.2 Hz, 1H), 4.06-3.94 (m, 2H), 3.92 (s, 3H), 3.67 (d, *J* = 7.3 Hz, 1H), 3.14 (m, *J* = 13.6, 10.4, 3.1 Hz, 2H), 2.07 (m, *J* = 13.4, 3.9 Hz, 2H), 1.62-1.52 (m, 2H), 1.49 (s, 9H).

### Step 2: Synthesis of tert-butyl 4-((5-amino-2-(methoxycarbonyl)phenyl)amino)piperidine-1-carboxylate

The compound obtained in Step 1 (100 mg, 0.26 mmol) was dissolved in ethyl acetate (5 mL), then Pd/C (2.80 mg, 0.03 mmol) was added, and the mixture was stirred at room temperature for 1 hour under hydrogen gas. The reaction product was confirmed by TLC and filtered through a Celite filter using methanol. The filtrate was concentrated and extracted with ethyl acetate/water and brine. The reaction product was dried over anhydrous sodium sulfate and concentrated to obtain the target compound (86 mg, 93 %).

¹H NMR (300 MHz, Chloroform-d) δ 7.82 (d, *J* = 8.7 Hz, 1H), 6.65 (s, 1H), 6.39 (dd, *J =* 8.7, 2.1 Hz, 1H), 3.98 (d, *J =* 14.6 Hz, 2H), 3.84 (d, *J* = 2.2 Hz, 3H), 3.50 (t, *J =* 5.0 Hz, 1H), 2.95 (t, *J =* 12.3 Hz, 2H), 2.03 (d, *J* = 8.6 Hz, 3H), 1.64-1.50 (m, 2H), 1.46 (d, *J* = 4.1 Hz, 9H).

### Step 3: Synthesis of tert-butyl 4-((5-(4-(tert-butyl)benzamido)-2-(methoxycarbonyl)phenyl)amino)piperidine-1-carboxylate

The compound obtained in Step 2 (1.20 g, 3.43 mmol), EDCI (790 mg, 4.12 mmol), DMAP (41 mg, 0.34 mmol) were added and stirred at ambient temperature for 20 minutes, and then 4-(tert-butyl)benzoic acid (734 mg, 4.12 mmol) was added. The product was confirmed after stirring at room temperature for 2 days. The product was extracted with dichloromethane/water and brine. The resulting product was dried over anhydrous sodium sulfate, concentrated, and separated by column chromatography to obtain the target compound (1.56 g, 89 %).

¹H NMR (300 MHz, Chloroform-d) δ 8.11 (s, 1H), 7.92 (d, *J* = 8.7 Hz, 1H), 7.89-7.79 (m, 2H), 7.71-7.63 (m, 1H), 7.56-7.48 (m, 2H), 6.83 (d, *J* = 8.6 Hz, 1H), 3.98 (t, *J* = 13.6 Hz, 2H), 3.87 (s, 3H), 3.68 (t, *J* = 8.7 Hz, 1H), 3.08 (t, J = 11.6 Hz, 2H), 2.09 (d, *J* = 14.3 Hz, 3H), 1.64-1.52 (m, 2H), 1.49 (s, 9H), 1.38 (s, 9H).

### Step 4: Synthesis of 2-((1-(tert-butoxycarbonyl)piperidin-4-yl)amino)-4-(4-(tert-butyl)benzamido)benzoic acid

The compound obtained in Step 3 (1.56 g, 3.06 mmol) was dissolved in THF/MeOH/H₂O (mixing ratio = 3 : 1 : 1, 30 mL), and lithium hydroxide monohydrate (513 mg, 12 mmol) was added, followed by stirring at 50°C for 3 days. The product was confirmed by TLC, concentrated, and adjusted to pH 2-3 by adding 1M HCl aqueous solution. The orange solid compound produced was obtained by filtration under reduced pressure and washed with diethyl ether to obtain the target compound (1.25 g, 82%).

¹H NMR (300 MHz, Chloroform-d) δ 8.05 (d, *J* = 6.0 Hz, 1H), 7.98 (d, *J* = 8.7 Hz, 1H), 7.89-7.78 (m, 2H), 7.69 (s, 1H), 7.59-7.47 (m, 2H), 6.67 (d, *J* = 8.8 Hz, 1H), 4.14-3.87 (m, 2H), 3.69 (s, 1H), 3.09 (t, *J* = 12.0 Hz, 2H), 2.24-1.96 (m, 3H), 1.81-1.54 (m, 2H), 1.37 (s, 9H), 1.32-1.23 (m, 2H).

### Step 5: Synthesis of tert-butyl 4-((5-(4-(tert-butyl)benzamido)-2-carbamoylphenyl)amino)piperidine-1-carboxylate

The compound obtained in Step 4 (1.25 g, 2.52 mmol) was added to DMF (25 ml), then DIPEA (1.32 ml, 7.57 mmol) and HBTU (1.44 g, 3.78 mmol) were added, and the resulting mixture was stirred at room temperature for 30 minutes. NH₄Cl (404.72 mg, 7.57 mmol) was added, and the resulting mixture was stirred at room temperature overnight. The product was confirmed by TLC and extracted with ethyl acetate/water and saturated ammonium chloride. The resulting product was dried over anhydrous sodium sulfate, concentrated, and separated by column chromatography to obtain the target compound (1.13 g, 91 %).

¹H NMR (400 MHz, DMSO-d₆) δ 10.10 (s, 1H), 8.49 (d, *J* = 7.3 Hz, 1H), 7.91-7.81 (m, 2H), 7.61 (d, *J* = 8.7 Hz, 1H), 7.58-7.52 (m, 2H), 7.24 (d, *J* = 2.0 Hz, 1H), 7.12-6.96 (m, 2H), 3.80 (d, *J* = 13.8, 4.2 Hz, 2H), 3.47 (s, 1H), 3.00 (d, *J* = 39.1 Hz, 2H), 2.69 (s, 2H), 1.98 (d, *J* = 12.4 Hz, 2H), 1.41 (s, 9H), 1.33 (s, 9H).

### Step 6: Synthesis of 4-(tert-butyl)-N-(4-carbamoyl-3-(piperidin-4-ylamino)phenyl)benzamide trifluoroacetate

The compound obtained in Step 5 (1.31 g, 2.65 mmol) was added to 20% TFA in DCM (26 mL) and reacted at room temperature for 3 hours. The product was confirmed by TLC and concentrated, and then the resulting solid product was washed several times with diethyl ether to obtain the target compound (1.21 g, 90%).

¹H-NMR (300 MHz, DMSO-d₆) δ 10.13 (s, 1H), 8.59 (s, 1H), 8.39 (s, 1H), 7.91-7.83 (m, 2H), 7.63 (d, *J* = 8.7 Hz, 1H), 7.59-7.49 (m, 2H), 7.34 (d, *J =* 2.0 Hz, 1H), 6.98 (dd, *J =* 8.7, 1.9 Hz, 1H), 3.57 (d, *J =* 10.2 Hz, 1H), 3.39 (d, *J* = 7.0 Hz, 2H), 3.18-3.00 (m, 2H), 2.22-2.09 (m, 2H), 1.58 (d, *J* = 11.1 Hz, 2H), 1.33 (s, 9H).

¹³C NMR (101 MHz, DMSO-d₆) δ 171.66, 166.21, 155.10, 149.55, 143.76, 132.54, 130.46, 128.01, 125.68, 110.03, 107.51, 102.56, 46.78, 46.22, 43.42, 35.17, 31.38, 29.23, 21.23.

MS (ESI) m/z calcd for C₂₃H₃₀N₄O₂ (M⁺) 394.24; found 395.4 (M+H⁺).

### Example E-1: Synthesis of methyl 2-((1-benzylpiperidin-3-yl)amino)-4-(4-(tert-butyl)benzamido)benzoate

The compound prepared in Preparation Example 4 (300 mg, 0.59 mmol) and chloromethyl(benzene) (149 mg, 1.18 mmol) were dissolved in anhydrous DCM (6 mL), then DIPEA (229 mg, 1.77 mmol) was added, and the mixture was stirred at ambient temperature for 1 hour. The reaction product was confirmed by TLC, diluted with EtOAc, and washed with an aqueous NaHCO₃ solution. The organic layer was dried over anhydrous Na₂SO₄, concentrated under reduced pressure, and separated by column chromatography to obtain the target compound (245 mg).

¹H NMR (300 MHz, DMSO-d₆) δ 10.19 (s, 1H), 8.10 (d, *J* = 7.9 Hz, 1H), 7.94-7.82 (m, 2H), 7.77 (d, *J* = 8.8 Hz, 1H), 7.61-7.51 (m, 2H), 7.41 (m, *J* = 7.4, 1.9 Hz, 2H), 7.36 (d, *J =* 1.9 Hz, 1H), 7.34-7.26 (m, 2H), 7.25-7.14 (m, 1H), 7.05 (dd, *J* = 8.9, 1.8 Hz, 1H), 3.81 (s, 3H), 3.62 (d, *J* = 9.9 Hz, 1H), 2.62 (d, *J* = 10.8 Hz, 1H), 2.45 (s, 1H), 2.36 (s, 2H), 1.99 (s, 1H), 1.81-1.44 (m, 4H), 1.33 (s, 9H); LC-MS (ESI, m/z) = 500.5 (M+H⁺).

### Example E-2: Synthesis of 2-((1-benzylpiperidin-3-yl)amino)-4-(4-(tert-butyl)benzamido)benzoic acid

The compound obtained in Example E-1 (245 mg, 0.50 mmol) was dissolved in 5 mL of a THF/MeOH/H₂O mixture (mixing ratio = 3 : 1 : 1), and LiOH•H₂O (63 mg, 1.50 mmol) was added and stirred at ambient temperature for 24 hours. The reaction product was confirmed by TLC, and 1M HCl aqueous solution was added dropwise so that the pH of the reaction solution was 2 to 3. The resulting white solid was filtered to obtain the target compound (146 mg).

¹H NMR (400 MHz, DMSO-d₆) δ 12.51 (s, 1H), 10.63 (s, 1H), 10.35 (s, 1H), 7.96 (d, *J* = 8.2 Hz, 2H), 7.87 (d, *J =* 8.0 Hz, 1H), 7.78 (d, *J* = 8.7 Hz, 1H), 7.62 (d, *J* = 5.7 Hz, 2H), 7.58-7.54 (m, 2H), 7.53 (s, 1H), 7.48-7.39 (m, 3H), 7.09 (d, *J* = 8.8 Hz, 1H), 4.46 (d, *J* = 14.0 Hz, 1H), 4.29 (s, 1H), 3.93 (s, 1H), 3.65 (d, *J* = 10.3 Hz, 1H), 2.86 (s, 2H), 2.14 (d, *J* = 11.9 Hz, 1H), 1.92 (d, *J* = 4.3 Hz, 2H), 1.45 (d, *J* = 25.2 Hz, 1H), 1.33 (s, 9H); LC-MS (ESI, m/z) = 486.4 (M+H⁺).

### Example E-3: Synthesis of N-(3-((1-benzylpiperidin-3-yl)amino)-4-carbamoylphenyl)-4-(tert-butyl)benzamide

The compound obtained in Example E-2 (45 mg, 0.09 mmol) and NH₄Cl (12 mg, 0.24 mmol) were dissolved in DMF (4 mL), and then DIPEA (30 mg, 0.24 mmol) and HBTU (45 mg, 0.12 mmol) were added and stirred at ambient temperature for 5 hours. The reaction product was confirmed by TLC, diluted with EtOAc, and washed with an aqueous NaHCO₃ solution. The organic layer was dried over anhydrous Na₂SO₄, concentrated under reduced pressure, and separated by column chromatography to obtain the target compound (13 mg).

¹H NMR (300 MHz, DMSO-d₆) δ 10.08 (s, 1H), 8.59 (d, *J* = 8.1 Hz, 1H), 7.96 (s, 1H), 7.88 (d, *J* = 8.3 Hz, 2H), 7.69 (s, 1H), 7.57 (dd, *J* = 11.3, 8.5 Hz, 3H), 7.40 (d, J = 7.4 Hz, 2H), 7.35-7.18 (m, 4H), 6.99 (d, *J* = 8.7 Hz, 2H), 3.36 (s, 2H), 2.66 (s, 3H), 1.75 (s, 2H), 1.51 (d, *J* = 12.9 Hz, 2H), 1.33 (s, 9H); LC-MS (ESI, m/z) = 485.4 (M+H⁺).

The chemical structures of the compounds according to Examples are summarized in Table 1 below.

**[Table 1]**

| Example | Structure | Example | Structure |
|---|---|---|---|
| A-1 | 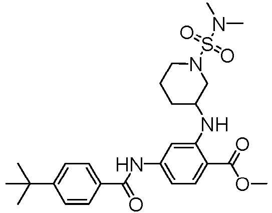 | A-2 | 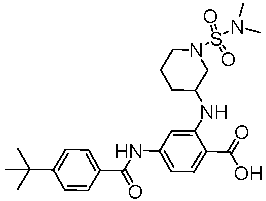 |
| A-3 | 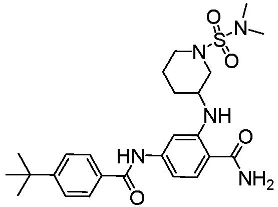 | A-4 | 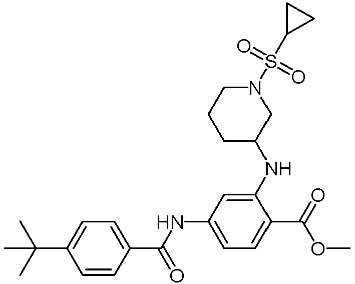 |
| A-5 | 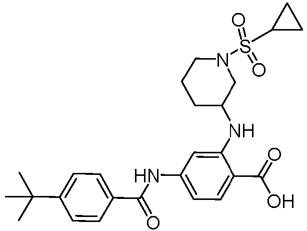 | A-6 | 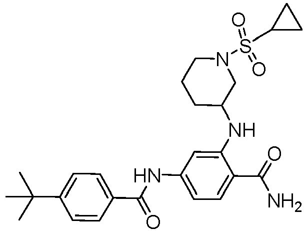 |
| A-7 | 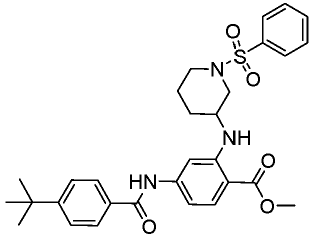 | A-8 | 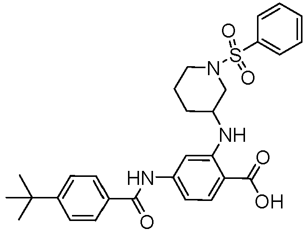 |
| A-9 | 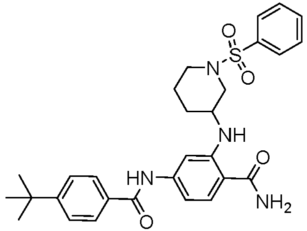 | A-10 | 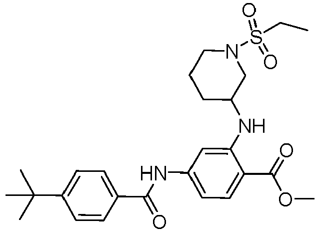 |
| A-11 | 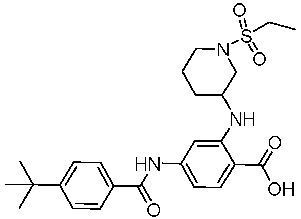 | A-12 | 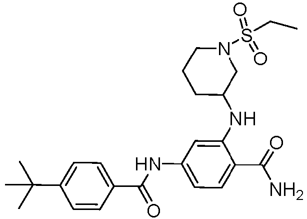 |
| A-13 | 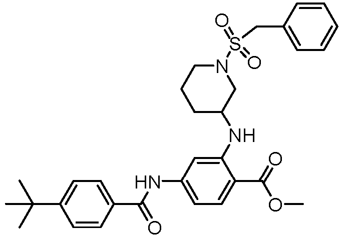 | A-14 | 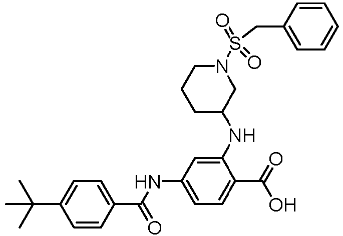 |
| A-15 | 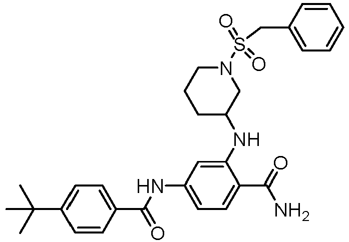 | A-16 | 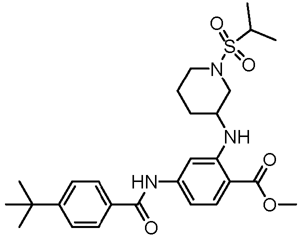 |
| A-17 | 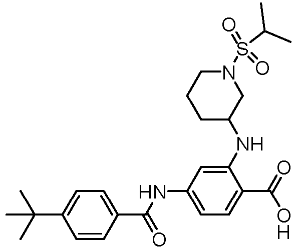 | A-18 | 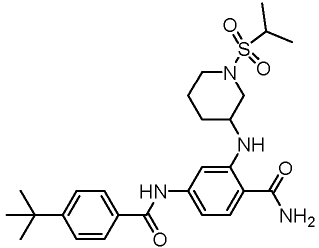 |
| A-19 | 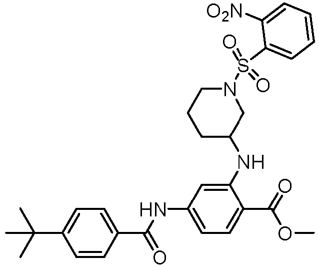 | A-20 | 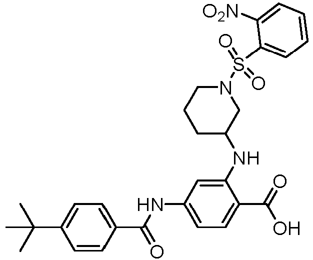 |
| A-21 | 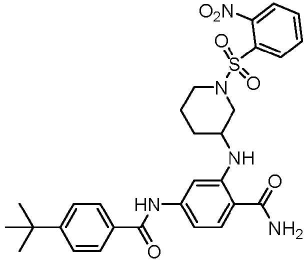 | A-22 | 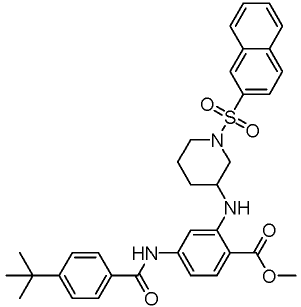 |
| A-23 | 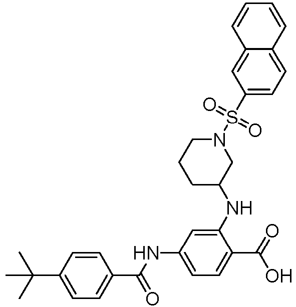 | A-24 | 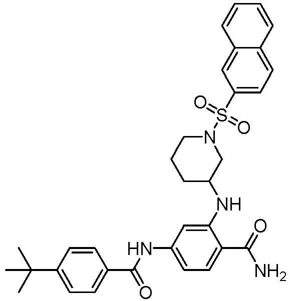 |
| A-25 | 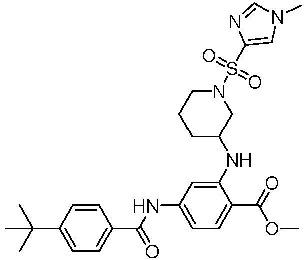 | A-26 | 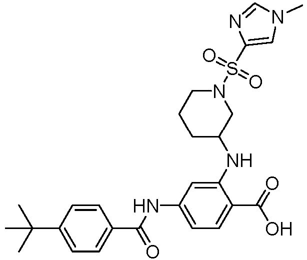 |
| A-27 | 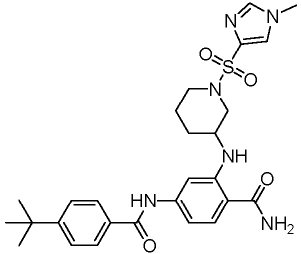 | A-28 | 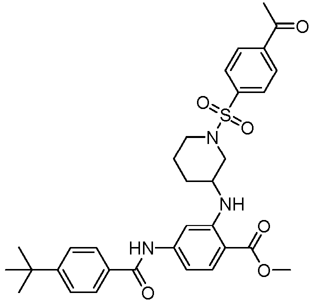 |
| A-29 | 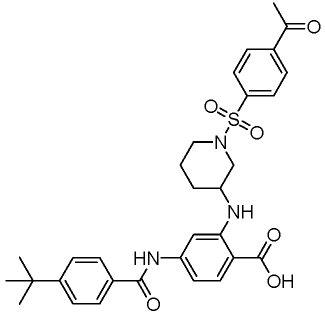 | A-30 | 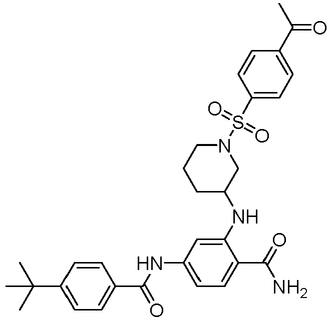 |
| A-31 | 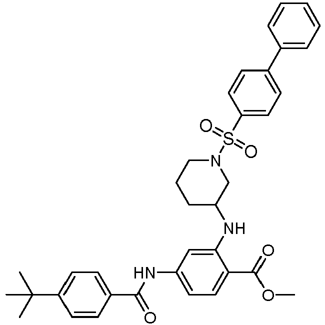 | A-32 | 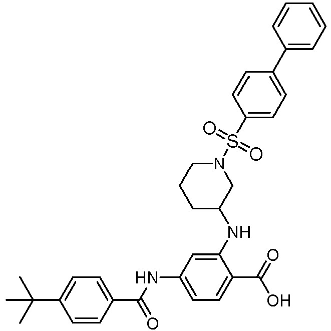 |
| A-33 | 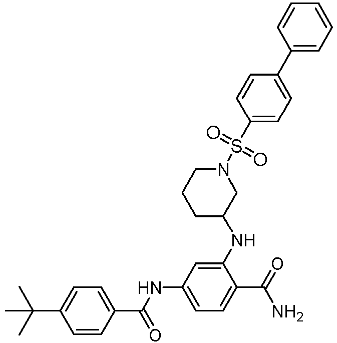 | | |
| B-1 | 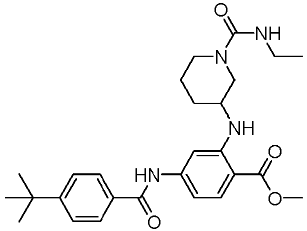 | B-2 | 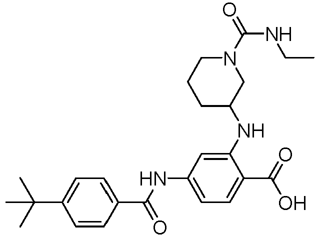 |
| B-3 | 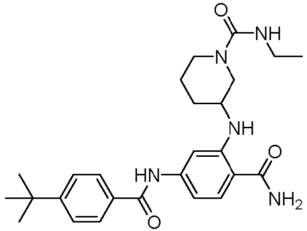 | B-4 | 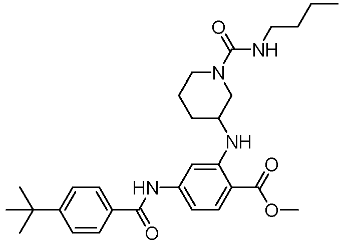 |
| B-5 | 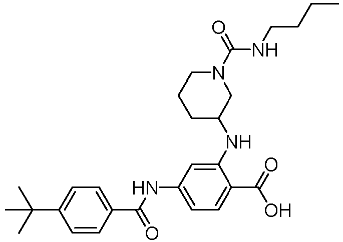 | B-6 | 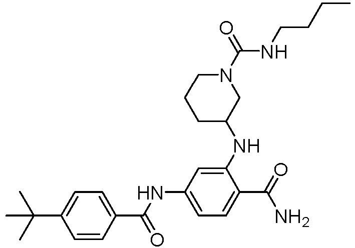 |
| B-7 | 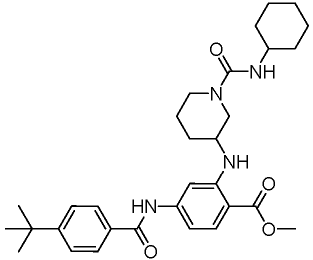 | B-8 | 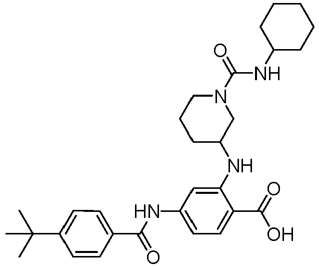 |
| B-9 | 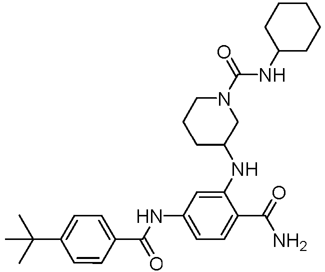 | B-10 | 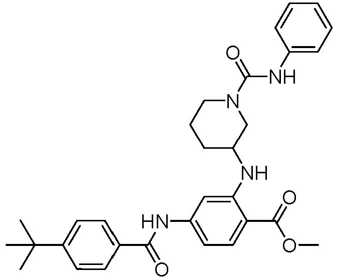 |
| B-11 | 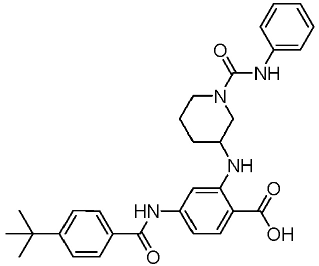 | B-12 | 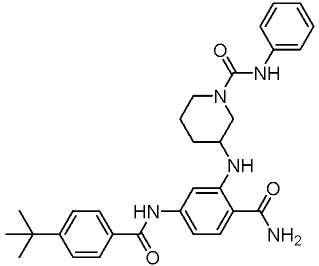 |
| B-13 | 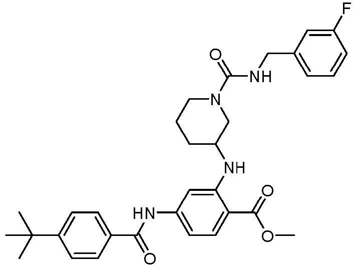 | B-14 | 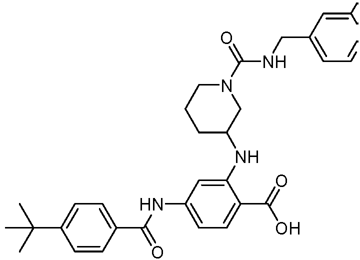 |
| B-15 | 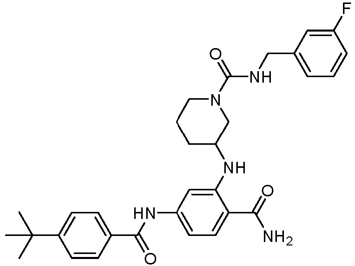 | B-16 | 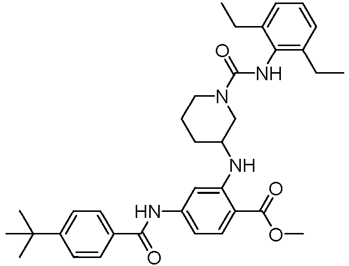 |
| B-17 | 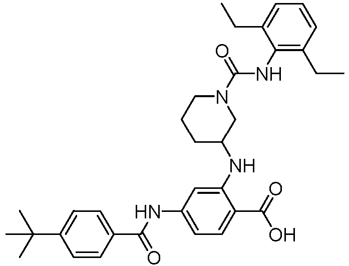 | B-18 | 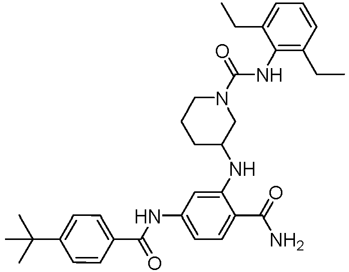 |
| B-19 | 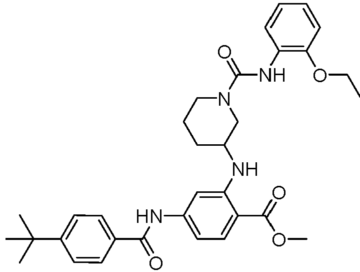 | B-20 | 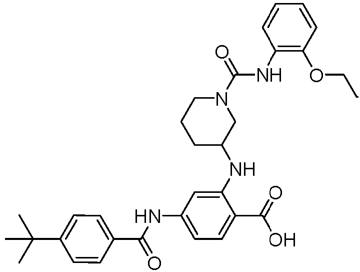 |
| B-21 | 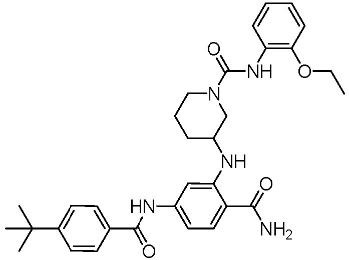 | B-22 | 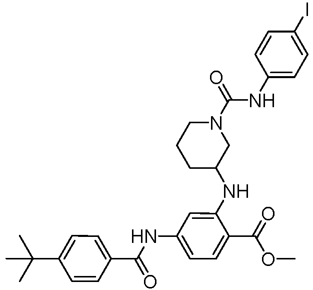 |
| B-23 | 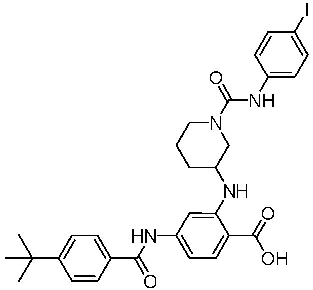 | B-24 | 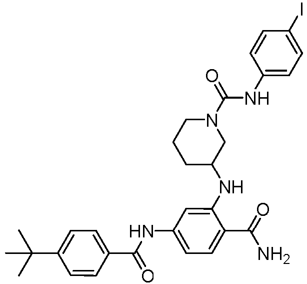 |
| B-25 | 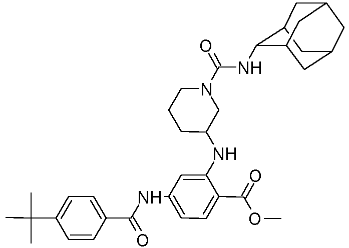 | B-26 | 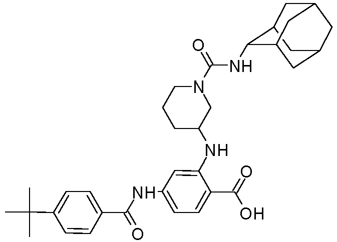 |
| B-27 | 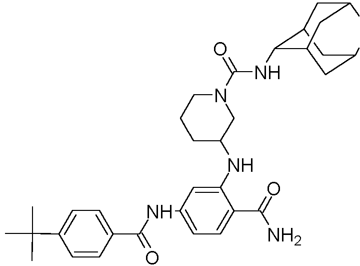 | B-28 | 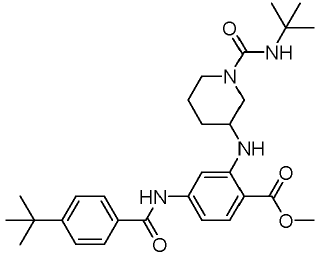 |
| B-29 | 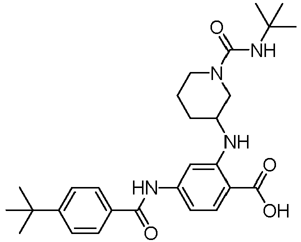 | B-30 | 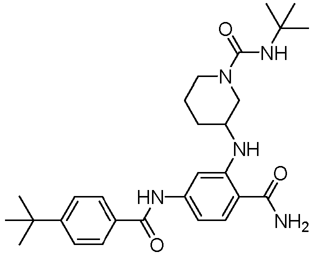 |
| B-31 | 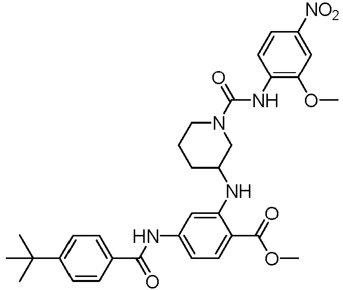 | B-32 | 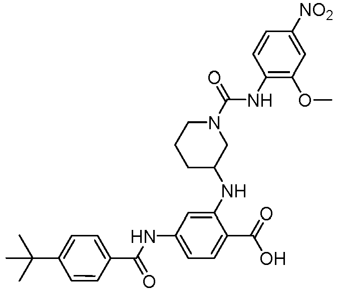 |
| B-33 | 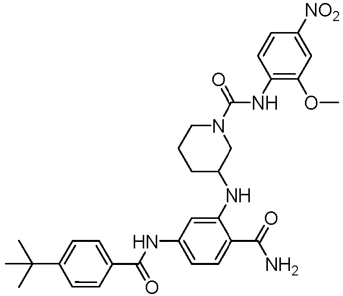 | B-34 | 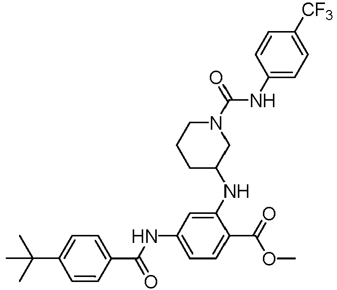 |
| B-35 | 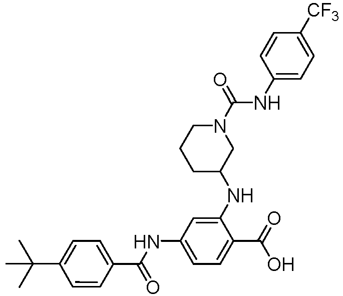 | B-36 | 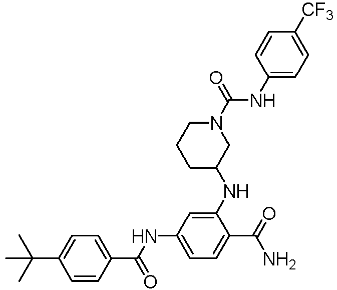 |
| B-37 | 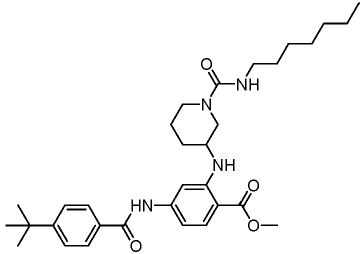 | B-38 | 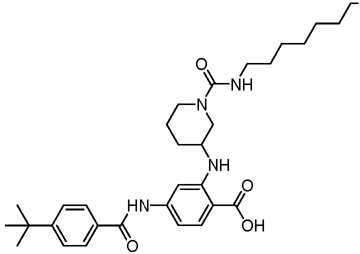 |
| B-39 | 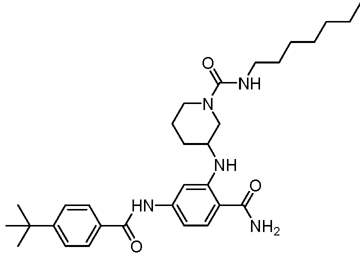 | B-40 | 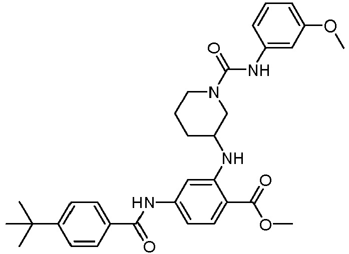 |
| B-41 | 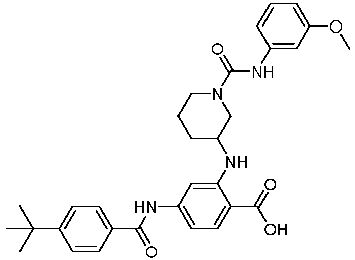 | B-42 | 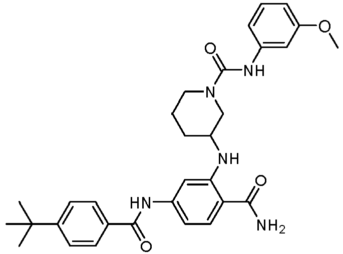 |
| C-1 | 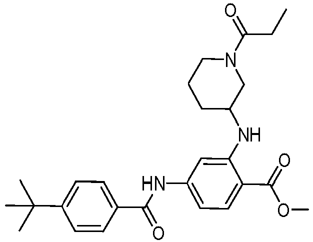 | C-2 | 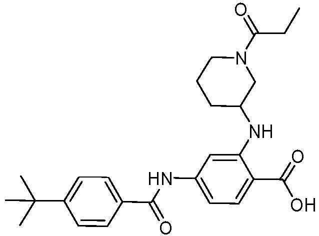 |
| C-3 | 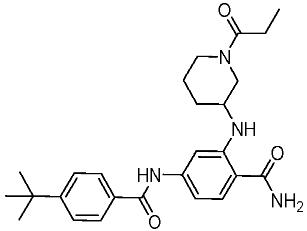 | C-4 | 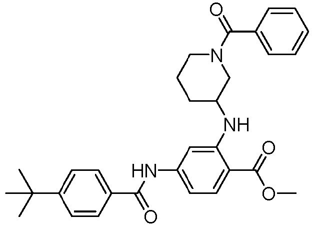 |
| C-5 | 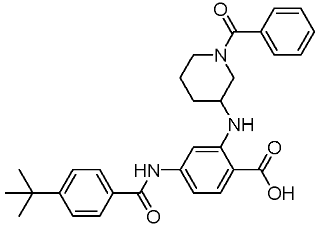 | C-6 | 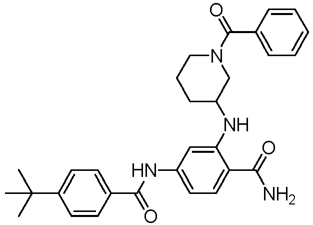 |
| D-1 | 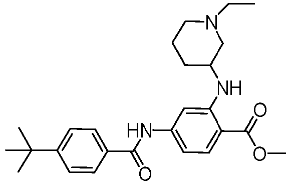 | D-2 | 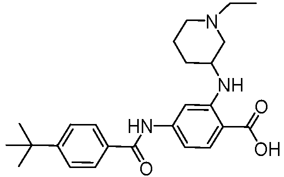 |
| D-3 | 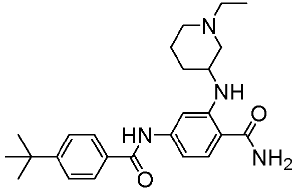 | D-4 | 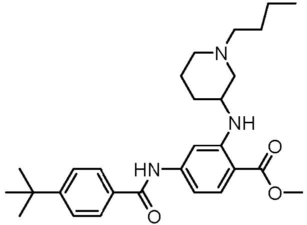 |
| D-5 | 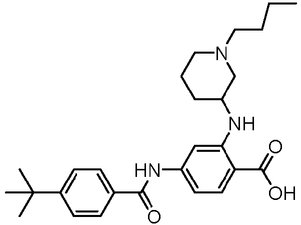 | D-6 | 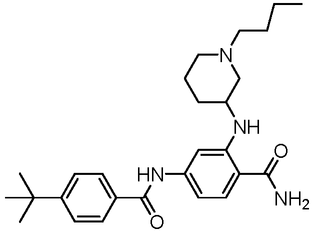 |
| D-7 | 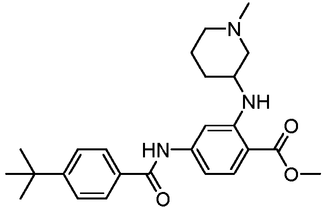 | D-8 | 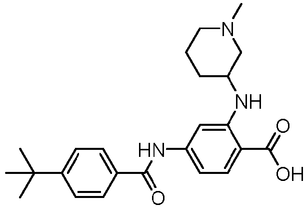 |
| D-9 | 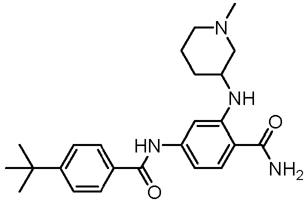 | D-10 | 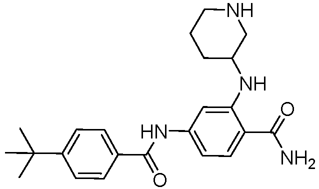 |
| D-11 | 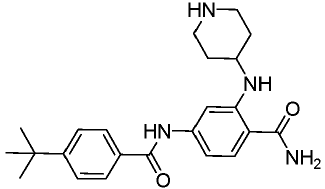 | | |
| E-1 | 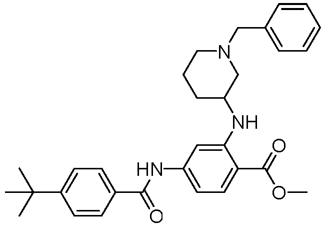 | E-2 | 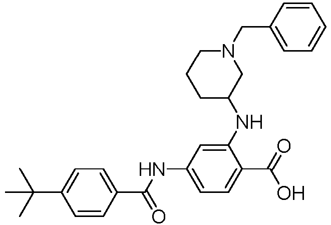 |
| E-3 | 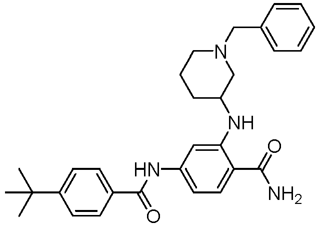 | | |

### Experimental Example 1. Evaluation of FLT3 inhibitory activity of the compound according to the present invention

In order to confirm the inhibition of cancer cell proliferation of the compound, the WST-8 assay (Cyto X cell viability assay kit, LPS solution, Daejeon, Korea) was performed according to the manufacturer's protocol. MV4-11, an acute myeloid leukemia cell line, was inoculated into a 96-well plate at 3 X 10³ per well. The cells were cultured for 24 hours, and treated with the compound at a concentration of 10 µM. After 72 hours, the culture medium was discarded and a chromatographic solution was added. Following a 1-hour incubation at 37°C, color development was observed, and the absorbance was measured at 480 nm using a Versamax microplate reader (Molecular Devices, CA, USA). In addition, in order to confirm the IC₅₀ value of the compound in MV4-11, the cells were treated with the compound with the final concentration of 0.02 to 100 µM in the same manner as the experiment above.

The results are summarized in Table 2 below.

**[Table 2]**

| Example | % inhibition at 10 µM | IC₅₀ (µM) |
|---|---|---|
| A-3 | 87.62 | 6.74 |
| A-15 | 95.88 | 4.1 |
| A-18 | 90.35 | 9.6 |
| B-4 | 84.61 | 15.3 |
| B-9 | 94.58 | 2.4 |
| B-12 | 96.18 | 3.8 |
| B-15 | 100 | 4.75 |
| B-18 | 93.72 | 5.32 |
| B-24 | 94.03 | 4.68 |
| B-27 | 100 | 3.37 |
| B-33 | 80.46 | - |
| B-36 | 97.23 | 7.81 |
| B-39 | 100 | 3.26 |
| C-6 | 97.1 | 4.48 |
| D-1 | 100 | 3.42 |
| D-4 | 97.8 | 2.2 |
| D-6 | 95.46 | 4.5 |
| D-7 | 100 | 4.59 |
| D-9 | 100 | 4.3 |
| D-10 | 70 | 2.0 |
| D-11 | 70 | 1.7 |
| E-1 | 70.10 | 2.2 |
| E-2 | 71.07 | 9.6 |

Table 2 above shows that the compounds according to Examples of the present invention exhibits high inhibitory activity against FLT3.

As described above, the present invention has been described in detail through preferred Examples and Experimental Examples, but the scope of the present invention is not limited to the specific compounds according to Examples of the present invention and should be interpreted by the claims. Further, those skilled in the art should understand that many modifications and variations can be made without departing from the scope of the present invention.

## Claims

1. A compound represented by the following Chemical Formula 1, a stereoisomer thereof, or a pharmaceutically acceptable salt thereof: 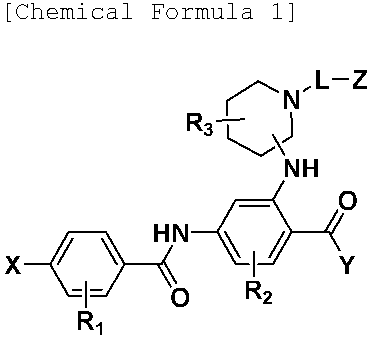 in Chemical Formula 1 above,
X is -C₁₋₆ alkyl or -C₁₋₆ haloalkyl;
Y is -C₁₋₆ alkyl, -OH, -O-C₁₋₆ alkyl, -NH₂, -NH-C₁₋₆ alkyl, or -N(C₁₋₆ alkyl) (C₁₋₆ alkyl);
L is -C(=O)-, -C(=O)NH-, -S(=O)₂-, or null;
Z is -H, -C₁₋₁₀ alkyl, -OH, -O-C₁₋₆ alkyl, -NH₂, -NH-C₁₋₆ alkyl, -N (C₁₋₆ alkyl) (C₁₋₆ alkyl), - (CH₂)n-cycloalkyl, - (CH₂)n-heterocycloalkyl, -(CH₂)n-aryl, or -(CH₂)n-heteroaryl, wherein at least one H of the - (CH₂)n-cycloalkyl, - (CH₂)n-heterocycloalkyl, -(CH₂)n-aryl, or -(CH₂)n-heteroaryl ring may be substituted with -C₁₋₆ alkyl, -C₁₋₆ aminoalkyl, -C₁₋₆ hydroxyalkyl, -C₁₋₆ haloalkyl, -CN, -C (=O) -C₁₋₆ alkyl, -NH₂, - NH-C₁₋₆ alkyl, -N(C₁₋₆ alkyl) (C₁₋₆ alkyl), -NO₂, -OH, -O-C₁₋₆ alkyl, -halo, aryl, or heteroaryl;
n is 0, 1, 2, 3, or 4; and
R₁ to R₃ are each independently -H, -C₁₋₆ alkyl, -C₁₋₆ aminoalkyl, -C₁₋₆ hydroxyalkyl, -C₁₋₆ haloalkyl, -CN, -NH₂, - NH-C₁₋₆ alkyl, -N(C₁₋₆ alkyl) (C₁₋₆ alkyl), -NO₂, -OH, -O-C₁₋₆ alkyl, or -halo.

2. The compound represented by Chemical Formula 1, the stereoisomer thereof, or the pharmaceutically acceptable salt thereof according to claim 1, wherein
X is -C₁₋₆ alkyl;
Y is -OH, -O-C₁₋₆ alkyl, -NH₂, -NH-C₁₋₆ alkyl, or -N(C₁₋₆ alkyl) (C₁₋₆ alkyl);
L is -C(=O)-, -C(=O)NH-, -S(=O)₂-, or null;
Z is -H, -C₁₋₁₀ alkyl, -NH₂, -NH-C₁₋₆ alkyl, -N(C₁₋₆ alkyl) (C₁₋₆ alkyl), -(CH₂)n-cycloalkyl, -(CH₂)n-aryl, or - (CH₂)n-heteroaryl, wherein at least one H of the -(CH₂)n-cycloalkyl, -(CH₂)n-aryl, or -(CH₂)n-heteroaryl ring may be substituted with -C₁₋₆ alkyl, -C₁₋₆ haloalkyl, -C(=O)-C₁₋₆ alkyl, -NO₂, -O-C₁₋₆ alkyl, -halo, or aryl;
n is 0, 1, or 2; and
R₁ to R₃ are each independently -H.

3. A compound represented by the following Chemical Formula 2, a stereoisomer thereof, or a pharmaceutically acceptable salt thereof: 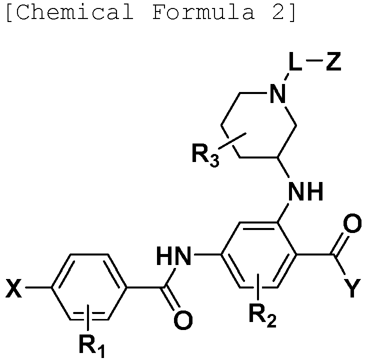 in Chemical Formula 2 above,
X is -C₁₋₆ alkyl or -C₁₋₆ haloalkyl;
Y is -C₁₋₆ alkyl, -OH, -O-C₁₋₆ alkyl, -NH₂, -NH-C₁₋₆ alkyl, or -N(C₁₋₆ alkyl) (C₁₋₆ alkyl);
L is -C(=O)-, -C(=O)NH-, -S(=O)₂-, or null;
Z is -H, -C₁₋₁₀ alkyl, -OH, -O-C₁₋₆ alkyl, -NH₂, -NH-C₁₋₆ alkyl, -N (C₁₋₆ alkyl) (C₁₋₆ alkyl), - (CH₂)n-cycloalkyl, - (CH₂)n-heterocycloalkyl, -(CH₂)n-aryl, or -(CH₂)n-heteroaryl, wherein at least one H of the - (CH₂)n-cycloalkyl, - (CH₂)n-heterocycloalkyl, -(CH₂)n-aryl, or -(CH₂)n-heteroaryl ring may be substituted with -C₁₋₆ alkyl, -C₁₋₆ aminoalkyl, -C₁₋₆ hydroxyalkyl, -C₁₋₆ haloalkyl, -CN, -C (=O) -C₁₋₆ alkyl, -NH₂, - NH-C₁₋₆ alkyl, -N(C₁₋₆ alkyl) (C₁₋₆ alkyl), -NO₂, -OH, -O-C₁₋₆ alkyl, -halo, aryl, or heteroaryl;
n is 0, 1, 2, 3, or 4; and
R₁ to R₃ are each independently -H, -C₁₋₆ alkyl, -C₁₋₆ aminoalkyl, -C₁₋₆ hydroxyalkyl, -C₁₋₆ haloalkyl, -CN, -NH₂, - NH-C₁₋₆ alkyl, -N(C₁₋₆ alkyl) (C₁₋₆ alkyl), -NO₂, -OH, -O-C₁₋₆ alkyl, or -halo.

4. A compound represented by the following Chemical Formula 3, a stereoisomer thereof, or a pharmaceutically acceptable salt thereof: 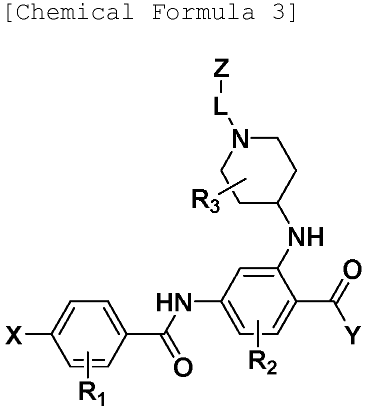 in Chemical Formula 3 above,
X is -C₁₋₆ alkyl or -C₁₋₆ haloalkyl;
Y is -C₁₋₆ alkyl, -OH, -O-C₁₋₆ alkyl, -NH₂, -NH-C₁₋₆ alkyl, or -N(C₁₋₆ alkyl) (C₁₋₆ alkyl);
L is -C(=O)-, -C(=O)NH-, -S(=O)₂-, or null;
Z is -H, -C₁₋₁₀ alkyl, -OH, -O-C₁₋₆ alkyl, -NH₂, -NH-C₁₋₆ alkyl, -N(C₁₋₆ alkyl) (C₁₋₆ alkyl), -(CH₂)n-cycloalkyl, - (CH₂)n-heterocycloalkyl, -(CH₂)n-aryl, or -(CH₂)n-heteroaryl, wherein at least one H of the -(CH₂)n-cycloalkyl, -(CH₂)n-heterocycloalkyl, -(CH₂)n-aryl, or -(CH₂)n-heteroaryl ring may be substituted with -C₁₋₆ alkyl, -C₁₋₆ aminoalkyl, -C₁₋₆ hydroxyalkyl, -C₁₋₆ haloalkyl, -CN, -C(=O)-C₁₋₆ alkyl, -NH₂, - NH-C₁₋₆ alkyl, -N(C₁₋₆ alkyl) (C₁₋₆ alkyl), -NO₂, -OH, -O-C₁₋₆ alkyl, -halo, aryl, or heteroaryl;
n is 0, 1, 2, 3, or 4; and
R₁ to R₃ are each independently -H, -C₁₋₆ alkyl, -C₁₋₆ aminoalkyl, -C₁₋₆ hydroxyalkyl, -C₁₋₆ haloalkyl, -CN, -NH₂, - NH-C₁₋₆ alkyl, -N(C₁₋₆ alkyl) (C₁₋₆ alkyl), -NO₂, -OH, -O-C₁₋₆ alkyl, or -halo.

5. A compound is selected from the group consisting of the following compounds, a stereoisomer thereof, or a pharmaceutically acceptable salt thereof:
(1) methyl 4-(4-(tert-butyl)benzamido)-2-((1-(N,N-dimethylsulfamoyl)piperidin-3-yl)amino)benzoate;
(2) 4-(4-(tert-butyl)benzamido)-2-((1-(N,N-dimethylsulfamoyl)piperidin-3-yl)amino)benzoic acid;
(3) 4-(tert-butyl)-N-(4-carbamoyl-3-((1-(N,N-dimethylsulfamoyl)piperidin-3-yl)amino)phenyl)benzamide;
(4) methyl 4-(4-(tert-butyl)benzamide)-2-((1-(cyclopropylsulfonyl)piperidin-3-yl)amino)benzoate;
(5) 4-(4-(tert-butyl)benzamido)-2-((1-(cyclopropylsulfonyl)piperidin-3-yl)amino)benzoic acid;
(6) 4-(tert-butyl)-N-(4-carbamoyl-3-((1-(cyclopropylsulfonyl)piperidin-3-yl)amino)phenyl)benzamide;
(7) methyl 4-(4-(tert-butyl)benzamido)-2-((1-(phenylsulfonyl)piperidin-3-yl)amino)benzoate;
(8) 4-(4-(tert-butyl)benzamido)-2-((1-(phenylsulfonyl)piperidin-3-yl)amino)benzoic acid;
(9) 4-(tert-butyl)-N-(4-carbamoyl-3-((1-(phenylsulfonyl)piperidin-3-yl)amino)phenyl)benzamide;
(10) methyl 4-(4-(tert-butyl)benzamido)-2-((1-(ethylsulfonyl)piperidin-3-yl)amino)benzoate;
(11) 4-(4-(tert-butyl)benzamido)-2-((1-(ethylsulfonyl)piperidin-3-yl)amino)benzoic acid;
(12) 4-(tert-butyl)-N-(4-carbamoyl-3-((1-(ethylsulfonyl)piperidin-3-yl)amino)phenyl)benzamide;
(13) methyl 2-((1-(benzylsulfonyl)piperidin-3-yl)amino)-4-(4-(tert-butyl)benzamido)benzoate;
(14) 2-((1-(benzylsulfonyl)piperidin-3-yl)amino)-4-(4-(tert-butyl)benzamido)benzoic acid;
(15) N-(3-((1-(benzylsulfonyl)piperidin-3-yl)amino)-4-carbamoylphenyl)-4-(tert-butyl)benzamide;
(16) methyl 4-(4-(tert-butyl)benzamido)-2-((1-(isopropylsulfonyl)piperidin-3-yl)amino)benzoate;
(17) 4-(4-(tert-butyl)benzamido)-2-((1-(isopropylsulfonyl)piperidin-3-yl)amino)benzoic acid;
(18) 4-(tert-butyl)-N-(4-carbamoyl-3-((1-(isopropylsulfonyl)piperidin-3-yl)amino)phenyl)benzamide;
(19) methyl 4-(4-(tert-butyl)benzamido)-2-((1-((2-nitrophenyl)sulfonyl)piperidin-3-yl)amino)benzoate;
(20) 4-(4-(tert-butyl)benzamido)-2-((1-((2-nitrophenyl)sulfonyl)piperidin-3-yl)amino)benzoic acid;
(21) 4-(tert-butyl)-N-(4-carbamoyl-3-((1-((2-nitrophenyl)sulfonyl)piperidin-3-yl)amino)phenyl)benzamide;
(22) methyl 4-(4-(tert-butyl)benzamido)-2-((1-(naphthalen-2-ylsulfonyl)piperidin-3-yl)amino)benzoate;
(23) 4-(4-(tert-butyl)benzamido)-2-((1-(naphthalen-2-ylsulfonyl)piperidin-3-yl)amino)benzoic acid;
(24) 4-(tert-butyl)-N-(4-carbamoyl-3-((1-(naphthalen-2-ylsulfonyl)piperidin-3-yl)amino)phenyl)benzamide;
(25) methyl 4-(4-(tert-butyl)benzamido)-2-((1-((1-methyl-1H-imidazol-4-yl)sulfonyl)piperidin-3-yl)amino)benzoate;
(26) 4-(4-(tert-butyl)benzamido)-2-((1-((1-methyl-1H-imidazol-4-yl)sulfonyl)piperidin-3-yl)amino)benzoic acid;
(27) 4-(tert-butyl)-N-(4-carbamoyl-3-((1-((1-methyl-1H-imidazol-4-yl)sulfonyl)piperidin-3-yl)amino)phenyl)benzamide;
(28) methyl 2-((1-((4-acetylphenyl)sulfonyl)piperidin-3-yl)amino)-4-(4-(tert-butyl)benzamido)benzoate;
(29) 2-((1-((4-acetylphenyl)sulfonyl)piperidin-3-yl)amino)-4-(4-(tert-butyl)benzamido)benzoic acid;
(30) N-(3-((1-((4-acetylphenyl)sulfonyl)piperidin-3-yl)amino)-4-carbamoylphenyl)-4-(tert-butyl)benzamide;
(31) methyl 2-((1-([1,1'-biphenyl]-4-ylsulfonyl)piperidin-3-yl)amino)-4-(4-(tert-butyl)benzamido)benzoate;
(32) 2-((1-([1,1'-biphenyl]-4-ylsulfonyl)piperidin-3-yl)amino)-4-(4-(tert-butyl)benzamido)benzoic acid;
(33) N-(3-((1-([1,1'-biphenyl]-4-ylsulfonyl)piperidin-3-yl)amino)-4-carbamoylphenyl)-4-(tert-butyl)benzamide;
(34) methyl 4-(4-(tert-butyl)benzamido)-2-((1-(ethylcarbamoyl)piperidin-3-yl)amino)benzoate;
(35) 4-(4-(tert-butyl)benzamido)-2-((1-(ethylcarbamoyl)piperidin-3-yl)amino)benzoic acid;
(36) 3-((5-(4-(tert-butyl)benzamido)-2-carbamoylphenyl)amino)-N-ethylpiperidine-1-carboxamide;
(37) methyl 4-(4-(tert-butyl)benzamido)-2-((1-(butylcarbamoyl)piperidin-3-yl)amino)benzoate;
(38) 4-(4-(tert-butyl)benzamido)-2-((1-(butylcarbamoyl)piperidin-3-yl)amino)benzoic acid;
(39) N-butyl-3-((5-(4-(tert-butyl)benzamido)-2-carbamoylphenyl)amino)piperidine-1-carboxamide;
(40) methyl 4-(4-(tert-butyl)benzamido)-2-((1-(cyclohexylcarbamoyl)piperidin-3-yl)amino)benzoate;
(41) 4-(4-(tert-butyl)benzamido)-2-((1-(cyclohexylcarbamoyl)piperidin-3-yl)amino)benzoic acid;
(42) 3-((5-(4-(tert-butyl)benzamido)-2-carbamoylphenyl)amino)-N-cyclohexylpiperidine-1-carboxamide;
(43) methyl 4-(4-(tert-butyl)benzamido)-2-((1-(phenylcarbamoyl)piperidin-3-yl)amino)benzoate;
(44) 4-(4-(tert-butyl)benzamido)-2-((1-(phenylcarbamoyl)piperidin-3-yl)amino)benzoic acid;
(45) 3-((5-(4-(tert-butyl)benzamido)-2-carbamoylphenyl)amino)-N-phenylpiperidine-1-carboxamide;
(46) methyl 4-(4-(tert-butyl)benzamido)-2-((1-((3-fluorobenzyl)carbamoyl)piperidin-3-yl)amino)benzoate;
(47) 4-(4-(tert-butyl)benzamido)-2-((1-((3-fluorobenzyl)carbamoyl)piperidin-3-yl)amino)benzoic acid;
(48) 3-((5-(4-(tert-butyl)benzamido)-2-carbamoylphenyl)amino)-N-(3-fluorobenzyl)piperidine-1-carboxamide;
(49) methyl 4-(4-(tert-butyl)benzamido)-2-((1-((2,6-diethylphenyl)carbamoyl)piperidin-3-yl)amino)benzoate;
(50) 4-(4-(tert-butyl)benzamido)-2-((1-((2,6-diethylphenyl)carbamoyl)piperidin-3-yl)amino)benzoic acid;
(51) 3-((5-(4-(tert-butyl)benzamido)-2-carbamoylphenyl)amino)-N-(2,6-diethylphenyl)piperidine-1-carboxamide;
(52) methyl 4-(4-(tert-butyl)benzamido)-2-((1-((2-ethoxyphenyl)carbamoyl)piperidin-3-yl)amino)benzoate;
(53) 4-(4-(tert-butyl)benzamido)-2-((1-((2-ethoxyphenyl)carbamoyl)piperidin-3-yl)amino)benzoic acid;
(54) 3-((5-(4-(tert-butyl)benzamido)-2-carbamoylphenyl)amino)-N-(2-ethoxyphenyl)piperidine-1-carboxamide;
(55) methyl 4-(4-(tert-butyl)benzamido)-2-((1-((4-iodophenyl)carbamoyl)piperidin-3-yl)amino)benzoate;
(56) 4-(4-(tert-butyl)benzamido)-2-((1-((4-iodophenyl)carbamoyl)piperidin-3-yl)amino)benzoic acid;
(57) 3-((5-(4-(tert-butyl)benzamido)-2-carbamoylphenyl)amino)-N-(4-iodophenyl)piperidine-1-carboxamide;
(58) methyl 2-((1-(adamantan-1-ylcarbamoyl)piperidin-3-yl)amino)-4-(4-(tert-butyl)benzamido)benzoate;
(59) 2-((1-(adamantan-1-ylcarbamoyl)piperidin-3-yl)amino)-4-(4-(tert-butyl)benzamido)benzoic acid;
(60) N-(adamantan-1-yl)-3-((5-(4-(tert-butyl)benzamido)-2-carbamoylphenyl)amino)piperidine-1-carboxamide;
(61) methyl 4-(4-(tert-butyl)benzamido)-2-((1-(tert-butylcarbamoyl)piperidin-3-yl)amino)benzoate;
(62) 4-(4-(tert-butyl)benzamido)-2-((1-(tert-butylcarbamoyl)piperidin-3-yl)amino)benzoic acid;
(63) N-(tert-butyl)-3-((5-(4-(tert-butyl)benzamido)-2-carbamoylphenyl)amino)piperidine-1-carboxamide;
(64) methyl 4-(4-(tert-butyl)benzamido)-2-((1-((2-methoxy-4-nitrophenyl)carbamoyl)piperidin-3-yl)amino)benzoate;
(65) 4-(4-(tert-butyl)benzamido)-2-((1-((2-methoxy-4-nitrophenyl)carbamoyl)piperidin-3-yl)amino)benzoic acid;
(66) 3-((5-(4-(tert-butyl)benzamido)-2-carbamoylphenyl)amino)-N-(2-methoxy-4-nitrophenyl)piperidine-1-carboxamide;
(67) methyl 4-(4-(tert-butyl)benzamido)-2-((1-((4-(trifluoromethyl)phenyl)carbamoyl)piperidin-3-yl)amino)benzoate;
(68) 4-(4-(tert-butyl)benzamido)-2-((1-((4-(trifluoromethyl)phenyl)carbamoyl)piperidin-3-yl)amino)benzoic acid;
(69) 3-((5-(4-(tert-butyl)benzamido)-2-carbamoylphenyl)amino)-N-(4-(trifluoromethyl)phenyl)piperidine-1-carboxamide
(70) methyl 4-(4-(tert-butyl)benzamido)-2-((1-(octylcarbamoyl)piperidin-3-yl)amino)benzoate;
(71) 4-(4-(tert-butyl)benzamido)-2-((1-(octylcarbamoyl)piperidin-3-yl)amino)benzoic acid;
(72) 3-((5-(4-(tert-butyl)benzamido)-2-carbamoylphenyl)amino)-N-octylpiperidine-1-carboxamide;
(73) methyl 4-(4-(tert-butyl)benzamido)-2-((1-((3-methoxyphenyl)carbamoyl)piperidin-3-yl)amino)benzoate;
(74) 4-(4-(tert-butyl)benzamido)-2-((1-((3-methoxyphenyl)carbamoyl)piperidin-3-yl)amino)benzoic acid;
(75) 3-((5-(4-(tert-butyl)benzamido)-2-carbamoylphenyl)amino)-N-(3-methoxyphenyl)piperidine-1-carboxamide;
(76) methyl 4-(4-(tert-butyl)benzamido)-2-((1-propionylpiperidin-3-yl)amino)benzoate;
(77) 4-(4-(tert-butyl)benzamido)-2-((1-propionylpiperidin-3-yl)amino)benzoic acid;
(78) 4-(tert-butyl)-N-(4-carbamoyl-3-((1-propionylpiperidin-3-yl)amino)phenyl)benzamide;
(79) methyl 2-((1-benzoylpiperidin-3-yl)amino)-4-(4-(tert-butyl)benzamido)benzoate;
(80) 2-((1-benzoylpiperidin-3-yl)amino)-4-(4-(tert-butyl)benzamido)benzoic acid;
(81) N-(3-((1-benzoylpiperidin-3-yl)amino)-4-carbamoylphenyl)-4-(tert-butyl)benzamide;
(82) methyl 4-(4-(tert-butyl)benzamido)-2-((1-ethylpiperidin-3-yl)amino)benzoate;
(83) 4-(4-(tert-butyl)benzamido)-2-((1-ethylpiperidin-3-yl)amino)benzoic acid;
(84) 4-(tert-butyl)-N-(4-carbamoyl-3-((1-ethylpiperidin-3-yl)amino)phenyl)benzamide;
(85) methyl 4-(4-(tert-butyl)benzamido)-2-((1-butylpiperidin-3-yl)amino)benzoate;
(86) 4-(4-(tert-butyl)benzamido)-2-((1-butylpiperidin-3-yl)amino)benzoic acid;
(87) 4-(tert-butyl)-N-(3-((1-butylpiperidin-3-yl)amino)-4-carbamoylphenyl)benzamide;
(88) methyl 4-(4-(tert-butyl)benzamido)-2-((1-methylpiperidin-3-yl)amino)benzoate;
(89) 4-(4-(tert-butyl)benzamido)-2-((1-methylpiperidin-3-yl)amino)benzoic acid;
(90) 4-(tert-butyl)-N-(4-carbamoyl-3-((1-methylpiperidin-3-yl)amino)phenyl)benzamide;
(91) 4-(tert-butyl)-N-(4-carbamoyl-3-(piperidin-3-ylamino)phenyl)benzamide;
(92) 4-(tert-butyl)-N-(4-carbamoyl-3-(piperidin-4-ylamino)phenyl)benzamide;
(93) methyl 2-((1-benzylpiperidin-3-yl)amino)-4-(4-(tert-butyl)benzamido)benzoate;
(94) 2-((1-benzylpiperidin-3-yl)amino)-4-(4-(tert-butyl)benzamido)benzoic acid; and
(95) N-(3-((1-benzylpiperidin-3-yl)amino)-4-carbamoylphenyl)-4-(tert-butyl)benzamide.

6. A pharmaceutical composition for preventing or treating cancer, comprising: the compound according to any one of claims 1 to 5, a stereoisomer thereof, or a pharmaceutically acceptable salt thereof, as an active ingredient.

7. The pharmaceutical composition of claim 6, wherein the cancer is blood cancer.

8. The pharmaceutical composition of claim 7, wherein the cancer is acute myeloid leukemia (AML).

9. The pharmaceutical composition of claim 6, wherein the cancer is at least one selected from the group consisting of liver cancer, thyroid cancer, colon cancer, myelodysplastic syndrome, glioblastoma, acute myeloid leukemia, acute lymphoblastic leukemia, multiple myeloma, colorectal cancer, chronic myeloid leukemia, chronic lymphocytic leukemia, adrenal cancer, non-small cell lung cancer, small cell lung cancer, pediatric lymphoma, pediatric leukemia, glioma, kidney cancer, malignant bone cancer, malignant lymphoma, malignant melanoma, stomach cancer, breast cancer, prostate cancer, rectal cancer, pancreatic cancer, lung cancer, and squamous cell carcinoma of the lung.

10. Use of the compound according to any one of claims 1 to 5, the stereoisomer thereof, or the pharmaceutically acceptable salt thereof, for use in the manufacture of a medicament for the treatment or prevention of Fms-like tyrosine kinase-3 (FLT3)-related diseases.

11. The use of claim 10, wherein the FLT3-related disease is cancer.

12. A method for treating or preventing Fms-like tyrosine kinase-3 (FLT3)-related diseases, comprising: administering a therapeutically effective amount of the compound according to any one of claims 1 to 5, the stereoisomer thereof, or the pharmaceutically acceptable salt thereof to a subject in need thereof.

13. The method of claim 12, wherein the FLT3-related disease is cancer.
